(19) Europäisches Patentamt
European Patent Office
Office européen des brevets

(11) Publication number : 0 496 548 A1

# (12) EUROPEAN PATENT APPLICATION

(21) Application number : 92300451.9

(22) Date of filing : 20.01.92

(51) Int. Cl.[5] : C07C 59/72, C07C 43/23, A61K 31/557

(30) Priority : 22.01.91 US 644303

(43) Date of publication of application : 29.07.92 Bulletin 92/31

(84) Designated Contracting States : AT BE CH DE DK ES FR GB GR IT LI NL SE

(71) Applicant : PURDUE RESEARCH FOUNDATION
328 Enad Building, Purdue University
West Lafayette, IN 47907 (US)

(72) Inventor : Fuchs, Philip L.
20 Brook Hollow
West Lafayette, Indiana 47906 (US)

(74) Representative : Bannerman, David Gardner et al
Withers & Rogers 4 Dyer's Buildings Holborn
London, EC1N 2JT (GB)

## (54) Carbacyclin analogs.

(57) Carbacyclin analogs that exhibit platelet aggregation inhibition activity and other biological activites common to structurally related prostacyclins are provided.

EP 0 496 548 A1

Background and Summary of the Invention

The present invention relates to novel carbacyclin analogs having pharmacological activity profiles similar to those of prostacyclins.

Prostacyclin, a compound of the formula

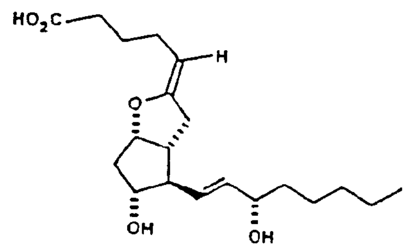

1

is endogenous to mammalian species and has been implicated in potentiation or control of platelet aggregation, hypertension, pregnancy, and cancer metastasis. Therapeutic application of prostacyclin is complicated by its extremely short half-life (generally 2-3 minutes) in vivo. Since this short half-life is believed to be related to the hydrolytic instability of the enol ether moiety of prostacyclin, attempts to synthesize a more stable long-lived analog have concentrated on replacement of the enol ether oxygen atom with a carbon atom to form the prostacyclin analog known as carbacyclin. Since carbacyclin has the chemical structure

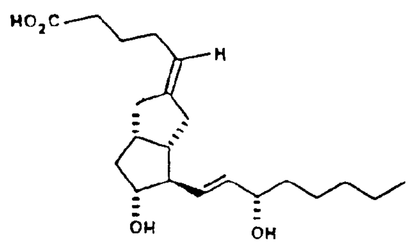

2

it does not suffer from hydrolytic instability. However, the serum half-life of carbacyclin is still comparable to that of prostacyclin in vivo because of enzymatic oxidation. Accordingly, there has been a need to define carbacyclin analogs having reduced susceptibility to enzymatic oxidation while retaining prostacyclin-like activity.

In accordance with the present invention there are provided carbacyclin analogs of the formula

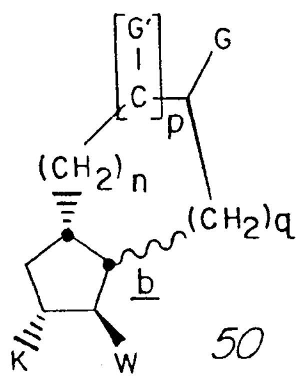

50

wherein K is hydrogen, hydroxymethyl, or hydroxy;
W is a group of the formula

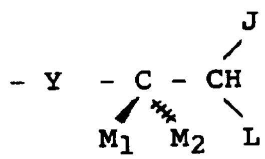

wherein Y is $-CH_2CH2_2-$, cis -CH=CH-, trans -CH=CH-, -C≡C,

$M_1$ and $M_2$ are independently hydrogen or hydroxy, or $M_1$ and $M_2$ taken together form =O; and

L and J are independently hydrogen or $C_1$-$C_6$ alkyl, or L and J taken together with the carbon atom bridging those groups form a $C_3$-$C_7$ cycloalkyl group; covalent bond b is in the alpha (a) or beta (β) configuration;

n is 0 or 1, p is 0 or 1, and q is 1 or 2; provided that

wenn n = 0, covalent bond b is in the alpha configuration;

when p = 0, n = q = 1 and b is in the beta configuration;

when p = 0, n = q = 1 and G is $-CH_2CH_2C-$ $(CH_3)_2CH_2Q$, or $=CHCH_2C(CH_3)_2CH_2Q$, wherein Q = -COOH or $-CH_2OH$;

when p = 1, G and G′ taken together with the carbon atoms to which they are bondedd form a group of the formula

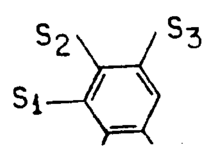

wherein $S_1$, $S_2$, and $S_3$ are hydrogen or a group -AEQ wherein A is $-CH_2-$ or -O-; E is $-(CH_2)_r-$ wherein r is 0-4, and Q is as defined above provided that at least two of $S_1$, $S_2$ and $S_3$ are hydrogen; and

when Q is -COOH, the pharmaceutically acceptable non-toxic salts and $C_1$-$C_{12}$ alkyl esters of the carboxylic acids represented thereby.

Provided also in accordance with the present invention are carbacyclin analogs of the formula

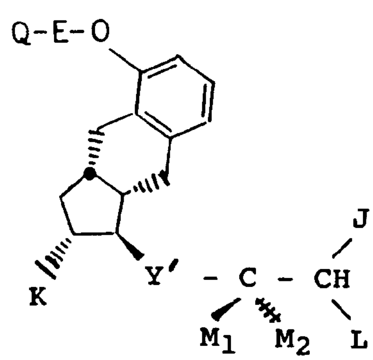

wherein K is hydrogen, hydroxy or hydroxymethyl;

Y′ is cis-CH=CH-, trans -CH=CH-, or -C≡C-;

$M_1$ and $M_2$ are independently hydrogen or hydroxy or $M_1$ and $M_2$ taken together form =O;

L and J are independently hydrogen or $C_1$-$C_7$ alkyl or L and J taken together with the carbon atom bridging those groups form a $C_3$-$C_7$ cycloalkyl group;

E is $-CH_2$ or $-CH_2CH_2-$; and

Q is -COOH or $-CH_2OH$; and when Q is -COOH the pharmaceutically acceptable salts of the carboxylic acids represented thereby.

Preferred compound embodiments of this invention are best represented by formulas 51-55

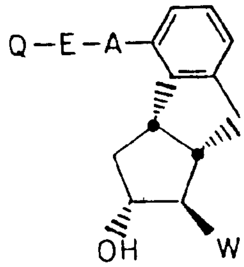

51

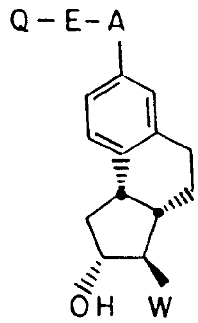

52

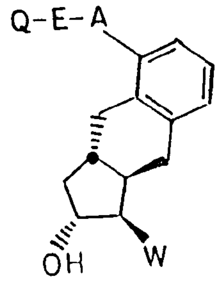

53

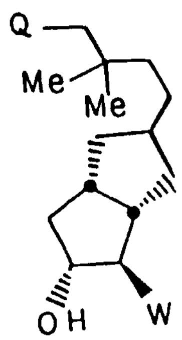

54

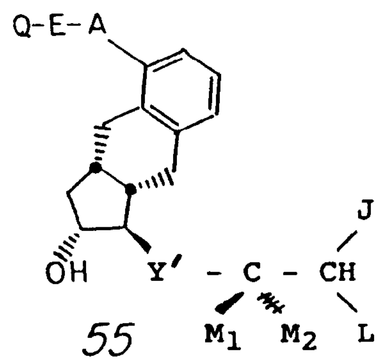

55

wherein Q, E, A, W, Y′, $M_1$, $M_2$, J and L are as defined above.

The relationship between those preferred compounds of this invention and the parameters n, p, q and b as defined above are summarized in Table I below.

TABLE I

| FORMULA | n | p | q | bond b |
|---|---|---|---|---|
| 51 | 0 | 1 | 1 | α |
| 52 | 0 | 1 | 2 | α |
| 53 | 1 | 1 | 1 | β |
| 54 | 1 | 0 | 1 | α |

The compounds of this invention have been found to exhibit prostaglandin-like activity and find use for treatment of patient conditions recognized in the art as responsive to therapy with prostaglandins and analogs thereof.

## Detailed Description of the Invention

The carbacyclin analogs of this invention are prepared generally by utilizing art-recognized synthetic procedures such as those set forth in U.S. Patent 4,420,632 to Aristoff, issued December 13, 1983, and herein incorporated by reference.

Compounds of this invention are illustrated more particularly in Table 2 below with reference to the indicated Formulas and the foregoing summary of the invention.

TABLE 2

| Formula | Q | E | A | W: Y/Y' | W: $M_1/M_2$ | W: -CHJL |
|---|---|---|---|---|---|---|
| 51, 52, 53, 55 | $-CH_2OH$ | $-(CH_2)_2-$ | O | $-C\equiv C-$ | H/OH | cyclohexyl |
| 51, 52, 53, 55 | -COOH | $-CH_2-$ | O | cis-CH=CH- | H/OH | cyclohexyl |
| 51, 52, 53 | -COOH | $-(CH_2)_4-$ | $-CH_2-$ | $-(CH_2)_2-$ | H/H | cyclopentyl |
| 51, 52, 53, 55 | $-CH_2OH$ | $-(CH_2)_2-$ | O | $-C\equiv C-$ | H/H | cyclobutyl |
| 51, 52, 53 | -COOH | $-(CH_2)_3-$ | $-CH_2-$ | $-(CH_2)_2-$ | =O | cycloheptyl |
| 51, 52, 53, 55 | $-CH_2OH$ | $-(CH_2)_2-$ | O | cis-CH=CH- | =O | 4-hexyl |
| 51, 52, 53, 55 | -COOH | $-CH_2-$ | O | $-C\equiv C-$ | H/OH | 3-octyl |
| 51, 52, 53, 55 | -COOH | $-CH_2-$ | O | cis-CH=CH- | OH/H | cyclohexyl |
| 54 | -COOH | - | - | $-C\equiv C-$ | OH/H | isopropyl |
| 54 | $-CH_2OH$ | - | - | trans-CH=CH- | H/OH | methyl |

The following Examples are provided to illustrate preparation of selected preferred compounds of this invention. In the Examples, all reactions were performed under a positive pressure of argon in oven-dried flasks except where noted. Tetrohydrofuran (THF) and diethyl ether were purified by distillation from benzophenone-sodium ketyl under nitrogen in a standing still. Hexane was maintained in a standing still over calcium hydride under nitrogen. Benzene, toluene, methylene chloride, dimethylsulfoxide, trimethylamine, N,N-dimethylformamide (DMF), hexamethylphosphoric triamide (HMPA), diisopropylethylamine, and 2,6-lutidine were distilled from calcium hydride. Triethylamine, diisopropylethylamine, and 2,6-lutidine were stored over sodium hydroxide pellets. Solutions of tetra-n-butyl ammonium fluoride (TBAF) (1.0 M in THF, Lancaster) were stored over 4Å molecular sieves. Brine refers to a saturated aqueous solution of sodium chloride. All other reaction, chromatographic, recrystallization and work-up solvents were spectroscopic grade. Sodium hydride was washed with dry hexanes three times to remove mineral oil and stored under argon. Methyllithium, n-butyllithium (nBuLi), and t-butyllithium were analyzed by titration of a solution of menthol in benzene containing 2,2′-bipyridyl as an indicator at room temperature under argon.

Chromatography was done on silica gel (60-200 mesh) from Sargent, Welch, or Davison chemicals. Deactivated silica gel columns refer to columns that were slurry packed using 1% triethylamine in hexanes as the solvent then flushed with the eluted solvent.

Analytical TLC was performed on silica gel 60 F-254 plates (Merck), and was visualized by dipping in p-panisaldehyde solution followed by heating on a hot plate.

Proton NMR spectra were recorded on a General Electric QE-300 (300 MHz) instrument and proton chemical shifts are reported in parts per million (ppm) downfield from tetramethylsilane as internal reference (0.0 ppm). Carbon NMR spectra were also recorded on the General Electric QE-300 instrumentt (75 MHz). Carbon chemical shifts are reported in ppm relative to internal tetramethylsilane reference (0.0 ppm) and are denoted as "e" (zero or two protons) or "o" (one or three protons) as determined from the APT (attached proton test) pulse sequence. All NMR spectra were recordeed in $CDCl_3$. Infrared spectra were recorded on a Perkin Elmer 1420 spectrophotometer either neat or as $CHCl_3$ solutions and are reported in $cm^{-1}$ and microns. The mass spectra were obtained on a Finnigan 4000 mass spectrometer or a CEC-21-110-B high resolution mass spectrometer using electron impact and chemical ionization with the molecular ion designated as M. Melting points were determined on a Laboratory Devices MelTemp apparatus. Melting points are uncorrected.

A selected list of abbreviations used in the Examples and in the structural formulas is provided as follows:

| | |
|---|---|
| bp | Boiling point |
| DMAP | 4-Dimethylaminopyridine |
| DMF | N, N-Dimethylformamide |
| DMSO | Dimethylsulfoxide |
| EtOAc | Ethyl acetate |
| HMPA | Hexamethylphosphoric triamide |
| hr | Hours |
| min | Minutes |
| MOM | Methoxymethoxy |
| mp | Melting point |
| NCS | N-chlorosuccinimide |
| NBS | N-bromosuccinimide |

| | |
|---|---|
| NMP | N-methylpyrrolidone |
| Ox | Oxazoline |
| TBAF | Tetra-n-butyl ammonium fluoride |
| TBDMS | t-Butyldimethylsilyl |
| TBDPS | t-Butyldiphenylsilyl |
| TFA | Trifluoroacetic Acid |
| THF | Tetrahydrofuran |
| THP | Tetrahydropyranyl |
| TMS | Trimethylsilyl |

The compound numbers in the Examples reference the structural formulas in the reaction schemes following the Examples and form part of this specification. In the formulas depicted in those reaction schemes and in those formulas hereinabove, a series of short parallel lines indicate covalent bonding in the alpha configuration, i.e., substituents bonded to the ring below the plane of the ring, and a wedge shaped bond indicates bonding in the beta configuration, i.e., the substituent positioned above the plane of the ring. Wavy lines represent bonding of a substituent or ring member either in the alpha or beta configuration. Abbreviations for chemical moieties and/or functional groups coupled to the carbon skeleton are consistent with the above listing of abbreviations.

EXAMPLE 1

Synthesis of ammonium salt 16, a synthetic intermediate that could be utilized in convergent synthesis of compounds according to the present invention, is described in Hutchinson, D.K. Ph.D. Thesis, Purdue University, 1987. With reference to the chemical scheme indicated by compounds of the Formula 16a-16, the following modifications to that synthetic procedure are described:

Resolution of sulfide alcohol 16c was greatly facilitated if racemic 16c is as pure as practical. preparation from compound 16b which had been previously isolated did not offer much improvement. It was critical, however, that all the peracid be consumed before the addition of the thiophenol. If thiophenol was introduced while peracid was still present, copious quantities of diphenyl disulfide were formed, and required tedious chromatography to remove. The undissolved solids present in the cyclopentadiene epoxidation contain sodium peracetate, and gave a positive test with starch-iodide paper. Therefore, before testing for the presence of peracid, the stirring was stopped and the mixture allowed to settle for 1-2 min.

When crystallizing the chiral urethane 16f derived from 16c, it was necessary to minimize the solvent evaporation during this process to avoid rapid precipitation of the contents of the mother liquor. Therefore, gravity filtration through a coarse sintered glass funnel was employed.

Cleavage of the urethane was most conveniently conducted in refluxing ether instead of toluene, for ease of solvent removal. Reaction time was about the same. The urethane must be dry (free of methanol or water): this was especially true when urethane from the mother liquors was cleaved.

The reaction of 16g with TBDPSCl and imidazole was performed with vigorous mechanical stirring and slow addition (over several hours) of the silyl chloride in THF; the temperature of -40°C was retained during the entire addition. Yields of up to 98% of chiral 16h (132 mmol scale) were routinely obtained. Slow stirring or overly-rapid addition of the silyl chloride afforded poor yields of 16h; much bis-silylated material was formed in these instances.

Silica gel chromatography of amine 16i has, on occasion, lead to isomerization. Recrystallization of crude 16i could not be affected from a wide variety of solvents, and often gave isomerized product. However, it was found that formation of amine 16i was efficient if the reaction used dimethylamine to displace the mesylate and was only allowed to stir for 20 minutes or less. Amine 16i was routinely purified by column chromatography using 60-200 mesh silica gel eluting with 5-25% (5% increments) of ethyl acetate/hexane. It was essential to get the amine in crystalline form as soon as possible to avoid isomerization. This could be accomplished by "shocking" the purified oil with hexanes and roto-vaping the hexanes off. If amine 16i was stored for any length of time, it was best to put it under high vacuum (ca. 0.5 torr) for 5 to 10 minutes before putting it in solution to avoid the formation of isomerization products via free dimethylamine which may be liberated during storage.

Formation of ammonium salt 16 from amine 16i was found to be extremely sensitive to the pH of the solution used to perform the anion exchange reaction. When the aqueous/THF solution of sodium borontetrafluoride was adjusted to have a pH of approximately 10 with sodium carbonate, the ratio of compound 16 to isomerized products was approximately one to three as judged from NMR analysis of the crude reaction mixture. When the solution had a pH of approximately 7.5, the ratio of compound 16 to its isomerized products was one to one, and when the pH of the solution was adjusted to 1.5 by adding tetrafluoro boronic acid, only 16 could be seen in the NMR of the crude mixture. Apparently, acidic solutions protonate any free amine, which may be in

the solution, inhibiting the isomerization process. By using acidic solutions of boron tetraflouride, quantitative yields of ammonium salt 16 could be obtained from amine 16i. Ammonium salt 16 could be stored for over a year in the freezer without detectable levels of decomposition.

EXAMPLE 2

Synthesis of indynaprost intermediate (1S,4R)-4-(t-Butyldiphenylsilyloxy)-1-[3′-(hydroxymethoxy)-2-phenyl]-2-phenylsulfonylcyclopent-2-ene 17 (Compound 17 is an intermediate used in the synthesis of (1R, 2R, 3aS, 8aS-1, 2, 3, 3a, 8, 8a-Hexahydro-4-carboxymethoxy-1-(1′-cyclohexyl-1′-hydroxypro-2′-yn-3′-yl)-2-hydroxycyclopent[a]indene; Trivial Name: Indynaprost; See Example 3)

To a slurry of commercially available 3-hydroxybenzaldehyde 10 (recrystallized twice from water and dried under high vacuum; 34.8g, 0.328 mol), methoxymethoxy chloride (MOMCl) (29.6 mL, 0.390 mol) in $CH_2Cl_2$ (350 mL) at 0°C was added diisopropyl ethylamine $(iPr)_2NEt$) (68.8 mL, 0.395 mol) dropwise over 12 minutes at a rate sufficient to maintain the reaction temperature below 10°C, followed by removal of the cooling bath. During the addition, the reaction became homogeneous. After stirring 1 hour at ambient temperature, the reaction was washed with 5% HCl (350 mL), saturated $NaHCO_3$ (350 mL), saturated NaCl (350 mL) and dried ($Na_2SO_4$). Evaporation of the solvent and distillation of the residue (bp 90-92°C/1.2 mm) afforded pure 3-(methoxy-methoxy) benzaldehyde 11 as a colorless oil, 44.8 g, 0.270 mol, 82% yield.

To a slurry of $NaBH_4$ (9.8 g, 0.259 mol) in 1:1 THF/EtOH (580 mL) at 0°C was added dropwise a solution of aldehyde 11 (41.1 g, 0.247 mol) in THF (70 mL) over 20 min at a rate sufficient to maintain the reaction temperature below 10°C. Ten minutes after the end of the addition, the mixture was diluted with deionized water (1 L) and extracted with $CH_2Cl_2$ (3x200 mL). The combined organics were washed with saturated NaCl (500 mL) and dried ($Na_2SO_4$). Evaporation of the solvent and distillation of the residue (bp 92-93°C/0.5 mm) afforded pure 3-(Methoxymethoxy)benzyl alcohol 12 as a colorless oil, 38.5 g, 0.229 mol, 93% yield.

To a solution of MOM ether 12 (10.02 g, 59.6 mmol) in 2:1 hexane/toluene (255 mL) with water bath cooling was added dropwise nBuLi (42.0 mL of a 2.95M solution in hexane, 124 mmol) over 22 min at a rate to maintain the reaction temperature below 30°C. A gummy precipitate appeared during this time. Stirring continued for 1 hr at ambient temperature at which time THF (100 mL) was added. After achieving homogeneity (ca. 5 min), the solution was cooled to -78°C and 2-Chloro-1-iodoethane (6.3 mL, 71.1 mmol) was added slowly. After stirring 75 min at this temperature, the cooling bath was removed and the reaction allowed to warm overnight. At room temperature, saturated $NH_4Cl$ (25 mL) was added dropwise, followed by dilution with deionized water (500 mL) and separation of the layers. The aqueous layer was extracted with ether (2x100 mL). The combined organics were washed with saturated NaCl (100 mL), dried ($Na_2SO_4$) and the solvents evaporated to afford 15.3 g of paste. Recrystallization (ether/hexane; 2 crops) gave pure 2-Iodo-3-(methoxymethoxy)benzyl alcohol 13 as white needles, 9.63 g.

To a solution of benzyl alcohol 13 (1.42 g, 4.83 mmol), trimethylsilylchloride (TMSCl) (1.25 mL, 9.85 mmol acid-free[132]), 4-dimethylaminopyridine (DMAP) (6.0 mg, 0.049 mmol) and DMF (10.0 mL) was added $Et_3N$ (1.70 mL, 12.2 mmol) slowly; a thick white precipitate formed immediately. After stirring 10 min, the reaction mixture was poured into iced 10% $Na_2CO_3$ (100 mL) and extracted with hexane (3x50 mL). The combined organics were dried ($K_2CO_3$) and evaporated to afford pure 2-Iodo-3-(methoxymethoxy)-1-[(trimethylsilyloxy)methyl)benzene 14 as a pale yellow oil which solidified on standing, 1.63 g, 4.45 mmol, 92% yield, mp 32.5-35°C.

To a solution of aryl iodide 14 (0.4083 g, 1.11 mmol) in ether (5.6 mL) at -78°C was added nBuLi (0.49 mL of a 2.39 M solution in hexane, 1.17 mmol) slowly. After 15 min stirring, a solution of ammonium salt 16 (0.6083 g, 1.00 mmol) in $CH_2Cl_2$ (5.6 mL) was added slowly (a bright yellow solution resulted) and stirring continued for 2 hr. An HF solution (1.50 mL of a 1.48 M solution in 95:5 $CH_3CN/H_2O$, 2.22 mmol) was added, the mixture warmed to 0°C for 15 min, cautiously quenched with saturated $NH_4Cl$ (10 mL) and the cooling bath removed. When the reaction had warmed to room temperature, it was extracted with $CH_2Cl_2$ (3x20 mL). The combined organics were washed with saturated NaCl (20 mL), dried ($Na_2SO_4$) and evaporated to afford the crude adduct (1S, 4R)-4-(t-Butyldiphenylsilyloxy)-1-[3′-(hydroxymethoxy)-2-phenyl]-2-phenylsulfonylcyclopent-2-ene 17 as a glassy foam, 0.6354 g. This material 17 was generally used directly in the synthesis of indynaprost (See Example 3) or could be purified by chromatography (24 g 60-200 mesh silica gel, 20% EtOAc/hexane). Purified compound 17 was recrystallized from $CCl_4$/heptane to afford small prisms, mp 127.5-129°C. The physical characteristics of compound 17 include: IR: 2.87 (OH), 3.55 ($OCH_3$), 7.66, 9.09 (sulfone). $^1H$ NMR: 7.95 (d of d, 2H, ortho-Phenylsulfonyl), 7.76-7.23 (m, 15H, aryl), 7.07 (t, 1H, vinyl), 6.20 (d of d, 1H, ortho-MOM aryl), 5.43 (m, 1H, silyloxy carbinol), 5.13 (br m, 1H, benzyl methine), 5.00 (d of d, 2H $OCH_2O$), 4.37 (d of d, $ArCH_2OH$), 3.05 (s, 3H, $OCH_3$), 2.77 (m, 1H, cyclopentenyl-$CH_2$-β), 2.57 (m, 1H, cyclopentenyl-$CH_2$-α), 1.35 (s, 9H, tBu). $^{13}C$ NMR: 155.43 (e), 146.18 (e), 145.49 (o), 140.58 (e), 139.56 (e), 135.64 (o), 133.51 (e), 133.37 (o), 132.62 (o), 129.83 (o), 128.27 (o), 127.77 (o), 125.85 (e), 123.28 (o), 114.60 (o), 113.04 (o), 93.28 (e), 78.91 (o), 63.52

(e), 55.58 (o), 42.35 (e), 42.03 (o), 26.77 (o), 19.08 (e). Exact mass: calculated for $C_{36}H_{39}O_5SSi$: 611.2287. Found: 611.2293 (M+H-$H_2O$).

EXAMPLE 3

Synthesis of (1R, 2R, 3aS, 8aS)-1, 2, 3, 3a, 8, 8a-Hexahydro-4-carboxymethoxy-1-(1′-cyclohexyl-1′-hydroxypro-2′-yn-3′-yl)-2-hydroxycyclopent[a]indene (Trivial Name: Indynaprost; n=0, p=1, q=1, covalent bond b is in the alpha configuration, K is hydroxy, Y is -C≡C-, $M_1$ is hydrogen, $M_2$ is hydroxy, L and J taken together with the carbon atom bridging those groups form a $C_6$ cycloalkyl group, $S_1$ is -AEQ, wherein A is -O-, E is -$(CH_2)_r$ wherein r is 1, Q is -COOH, and $S_2$ and $S_3$ are hydrogen).

A solution of crude benzyl alcohol 17 (0.6354 g, ca. 1.0 mmol), (4-dimethylaminophenyl) diphenylphosphine (0.6180 g, 2.02 mmol) and $CCl_4$ (8.0 mL) was heated at reflux for 2 min; the reaction rapidly becomes dark orange-brown in color and a gummy precipitate is deposited. After cooling to room temperature with a water bath, the mixture was diluted with $CH_2Cl_2$ (20 mL) stirred until homogeneous, washed with 5% HCl (2x10 mL) and the solvent evaporated. The residue was triturated with ether (100 mL) and filtered with suction through a plug of 60-200 mesh silica gel (20 g) in a sintered glass funnel. The filtrate was evaporated to afford (1S,4R)-4-(t-Butyldiphenyl-silyloxy)-1-[3′-(chloromethyl)-1′-(methoxymethoxy)-2-phenyl]-2-phenylsulfonylcyclopent-2 -ene 18 as a foam, 0.5519 g, 0.853 mmol, 85% yield (based on ammonium salt 16); the crude product was homogeneous by TLC. Recrystallization from heptane afforded tiny needles, mp 131.5-133°C.

To a solution of (1S)-1-Cyclohexyl-1-(tetrahydropyran-2-yloxy)-2-propyne 19 (2.60 g, 11.7 mmol) prepared, in THF (60 mL] at 0 0G was added nBuLi (4.8 mL of a 2.56 mL solution in hexane, 12.3 mmol) slowly [Compound 19 is prepared by silylation of (1S)-1-cyclohexyl-3-propyn-2-ol which is in turn prepared according the Midland procedure; See Midland, M. M., J. Org. Chem 1975, 40, 2250]. The resultant pale yellow solution was stirred 10 min, at which time HMPA (3.0 mL) and compound 18 (3.78 g, 5.84 mmol) in THF (60 mL) were added sequentially (a dark brown color results immediately) and the cooling bath removed. After stirring 2 hr at ambient temperature the reaction was quenched with saturated $NH_4Cl$ (50 mL), diluted with deionized water (500 mL) and extracted with hexane (3x100 mL). The combined organic layers were washed with saturated NaCl (100 mL), dried ($Na_2SO_4$) and evaporated to afford a dark foam, 6.14 g. Chromatography (240 g 60-200 mesh silica gel, 10% to 30% EtOAc/hexane gradient) afforded recovered acetylene 19 (1.06 g, 4.77 mmol, 41%). Continued elution gave pure (1R,1′S,2R,3aS,8aS)-1,2,3,3a,8,8a-Hexahydro-2-(t-butyldiphenylsilyloxy)-1-(1′-cyclohexyl-1′-tetrahydropyranyloxy-2-propy-3-yl)-4-methoxymethoxy-8a-phenylsulfonylcyclo-pent[a]indene 20 as a foam, 3.96 g, 4.75 mmol, 81% yield.

A mixture of sulfone 20 (6.02 g, 7.23 mmol), Na/Hg (28.38 g, 6% Na, 74.0 mmol I $Na_2HPO_4$ (5.13 g, 36.1 mmol) and EtOH (120 mL) was heated at reflux for 2 hours, at which time an additional aliquot of amalgam (3.23 g, 8.43 mmol) was added. Reflux was continued for another hour, then the mixture cooled to 0°C, saturated $NH_4Cl$ (100 mL) added and the cooling bath removed. Stirring continued until the amalgam had been quenched (generally overnight), at which time the mixture was diluted with deionized water (1 L), acidified with 5% NCl to ca. pH 1 and extracted with $CH_2Cl_2$ (3x200 mL). The combined organics were washed with saturated NaCl (200 mL), dried ($Na_2SO_4$) and evaporated to afford crude (1R,1′S,2R,3aS,8aS)-1,2,3,3a,8,8a-Hexahydro-2-(t-butyldiphenylsilyloxy)-1-(1′-cyclohexyl-1′-tetrahydropyranoxyl-2′-propyn-3′-yl)-4-methoxymethoxycyclopent [ a]indene 22 as a yellow foam, 5.00 g, 7.22 mmol, 99% yield (homogeneous by TLC). Although generally used directly in the next step, this material could be purified by chromatography (200 g 60-200 mesh silica gel, 5% EtOAc/hexane) to afford pure compound 22 in 77% yield.

A solution of crude acetal 22 (3.09 g, ca. 4.2 mmol), p-TsOH-$H_2O$ (82.0 mg, 0.431 mmol) and isopropanol (30 mL) was heated at reflux for 17 hr, then cooled to room temperature and 10% $Na_2CO_3$ (10 mL) and ether (50 mL) were added sequentially. After shaking and separation of the layers, the organics were dried ($Na_2SO_4$) and evaporated. Chromatography of the residue (100 g 60-200 mesh silica gel, 10% EtOAc/hexane) afforded pure (1R,1′S,2R,3aS,8aS)-1,2,3,3a, 8,8a-Hexahydro-2-(t-butyldiphenylsilyloxy)-1-(1′-cyclohexyl-1′-hydroxy-2′-propyn-3′-yl)-4-hydroxycyclopent[a]indene 24 as a yellow foam, 2.92 mmol, 67% yield (based on compound 22).

A mixture of phenol 24 (1.65 g, 2.92 mmol), chloroacetonitrile (12.0 mL, 190 mmol) and $Cs_2CO_3$ (1.91 g, 5.86 mmol) was stirred for 24 hr, then filtered through cellite. The filter cake was washed with $CH_2Cl_2$ (3x20 mL) and the filtrates combined. The $CH_2Cl_2$ was removed on a rotary evaporator. The excess chloroacetonitrile was removed by Kugelrohr distillation (water aspirator pressure, 50°C water bath) to afford 2.45 of dark glass. Chromatography (80 g 60-200 mesh silica gel, 80% to 100% $CH_2Cl_2$/hexane gradient) gave pure (1R,1′S,3aS,8aS)-1,2,3,3a,8,8a-Hexahydro-2-(t-butyldiphenylsilyloxy)-4-cyanomethoxy-1-(1′-cyclohexyl-1′-hydroxy-2′-propyn-3′-yl)-cyclopent[a]indene 26 as a white foam, 1.52 g, 2.52 mmol, 86% yield.

A mixture of nitrile 26 (1.88 g, 3.11 mmol), methanol (71 mL) and Na0H (21 mL of a 25% aqueous solution)

was heated at reflux for 24 hr, then cooled to room temperature. The solution was acidified with 5% HCl (150 mL) and extracted with $CN_2Cl_2$ (3x100 mL). The combined organics were washed with saturated NaCl (100 mL) and the layers quickly separated, as a fluffy precipitate began to form. After standing overnight, filtration afforded pure (1R, 2R, 3aS, 8aS-1, 2, 3, 3a, 8, 8a-Hexahydro-4-carboxymethoxy-1-(1′-cyclohexyl-1′-hydroxypro-2′-yn-3′-yl)-2-hydroxycyclopent[a]indene 100 (indynaprost), 0.41 g. The filtrate was dried ($Na_2SO_4$), evaporated and the residue subjected to chromatography (60 g 60-200 mesh silica gel, eluting sequentially with hexane, 50% EtOAc/hexane, EtOAc, 1% HOAc/EtOAc and finally 5% MeOH/1% HOAc/ EtOAc) to afford 0.69 g of off-white solid. Recrystallization from EtOAc/hexane provided pure 100 as small white needles, mp 179-189°C. The physical characteristics of compound 100 include: $[\alpha]_D$=-177.4° (c 1.04, MeOH). IR (KBr): 2.92, 3.13 (OH), 4.46 (acetylene), 5.74 (C=O). $^1$H NMR ($d_6$-acetone): 7.07 (t, 1H, H6), 6.76 (d, 1H, H7), 6.62 (d, 1H, H5), 4.65 (s, 2H, H2″), 4.00 (d, 1H, J3′4′=6.1 Hz, H3′), 3.94 (d of d, 1H, $J_{12}$=5.5 Hz, $J_{23\alpha}$=10.8, $J_{23\beta}$=3.8 Hz, H2), 3.83 (t of d, 1H, $J_{3a3\alpha}$=small, $J_{3a3\beta}$=3.5 Hz, $J_{3a8a}$=8.8 Hz, H3a), 3.26 (d of d, 1H, J8β8α=16.5 Hz, J8β8a=4.0 Hz, H8β), 3.17 (m,1H, $J_{8al}$=8.1 Hz, $J_{8a3a}$=8.8 Hz, $J_{8a8\alpha}$=9.9 Hz, $J_{8a8\beta}$=4.0 Hz, H8a), 3.06 (d of d, 1H, $J_{8\alpha 8\beta}$=16.5 Hz, $J_{8\alpha 8a}$=9.9 Hz, H8α), 2.84 (d of d, 1H, $J_{12}$=5.5 Hz, $J_{18a}$=8.1 Hz, H1), 2.19 (d of d, 1H, $J_{23\beta}$=3.8 Hz, $J_{3\alpha 3\beta}$=13.0 Hz, $J_{3\beta 3a}$=3.5 Hz, H3β), 2.01 (m, 1H, $J_{3\alpha 2}$=10.8 Hz, $J_{3\alpha 3\beta}$=13.0 Hz, $J_{3\alpha 3a}$=small, H3α), 1.83-1.62 (m, 4H, cyclohexyl $CH_2$), 1.40 (m, 1H, H4′), 1.28-1.00 (m, 4H, cyclohexyl $CH_2$). $^{13}$C NMR ($D_6$-DMSO): 170.33 (e), 154.04 (e), 144.72 (e), 133.94 (e), 127.98 (o), 116.85 (o), 108.77 (o), 84.50 (e), 84.40 (e), 76.22 (o), 65.46 (o), 64.37 (e), 44.47 (o), 44.14 (o), 43.38 (o), 41.82 (o), 40.58 (e), 35.87 (e), 28.45 (e), 27.70 (e), 26.19 (e), 25.62 (e). Elemental analysis: calculated for $C_{23}H_{28}O_5$: C 71.84, H 7.34. Found: C 72.14, H 7.69. Exact mass: calculated for $C_{23}H_{28}O_5$: 384.1937. Found: 384.1940. HPLC analysis: 6 cm ODS column, 30:70 water/MeOH, flow 1.00 ml/min, detected by UV at 210 nm; retention time 0.69 min, single peak after recrystallization.

EXAMPLE 4

Synthesis of (1R,2R,3aS,8aS)-1,2,3,3a,8,8a-Hexahydro-4-carboxy-1-(1-cyclohexyl-1-hydroxyprop-2-en-3-yl)-2-hydroxycyclopent(a) indene 110 (Trivial Name: Z-Indenaprost; n=0, p=1, q=1, covalent bond b is in the alpha configuration, K is hydroxy, Y is cis -CH=CH-, $M_1$ is hydrogen, $M_2$ is hydroxy, L and J taken together with the carbon atom bridging those groups form a $C_6$ cycloalkyl group, $S_1$ is -AEQ, wherein A is -O-, E is $-(CH_2)_r$ wherein r is 1, Q is -COOH, and $S_2$ and $S_3$ are hydrogen).

A suspension solution of compound 24 (prepared as described in Example 3) (67 mg, 0.119 mmol) and palladium catalyst supported on calcium carbonate and poisoned with lead (25 mg, 0.0119 mmol) in 7 ml of ethanol was stirred at room temperature under 1 atm of hydrogen in a balloon for 2 hr. The mixture was filtered through a pad of celite, washing the pad with ethanol. The filtrate was concentrated in vacuo affording a crude oil. The oil was purified by flash chromatography on 10 g of silica gel (60-200 mesh) eluted with 20% ethyl acetate/hexanes providing 66 mg (98% yield) of pure (1S(1S),2R,3aS,8aS)-1,2,3,3a,8,8a-Hexahydro-2-(t-butyldiphenylsilyloxy)-1-(cis-1-cyclohexyl-1-hydroxylprop-2-en-3-yl)-4-hydroxycyclopent(a)indene 101 as a white wax.

A solution of silyl ether 101 (66 mg, 0.116 mmol) in 4 ml of THF was formed. The solution was stirred at room temperature with 30 mg of powdered 4Å molecular sieves to which TBAF (1.2 ml of a 1.0 M solution in THF, 1.2 mmol), was added, followed by stirring for 24 hr. The mixture was diluted with 10 ml 5% HCl and extracted with 4 x 10 ml of ethyl acetate. The combined ethyl acetate layers were dried over sodium sulfate and concentrated in vacuo affording a crude oil. The oil was purified by chromatography on 5 g of silica gel (60-200 mesh) eluted with 10%, 30% ethyl acetate/hexanes to provide 28 mg (73% yield) of (1S(1S),2R,3aS,8aS)-1,2,3,3a,8,8a-Hexahydro-2-hydroxyl--(cis-1-cyclohexyl-1-hydroxylprop-2-en-3-yl)-4-hydroxycyclopent(a)indene 102 as a white wax.

A mixture the phenol 102 (28 mg, 0.085 mmol), chloroacetonitrile (2 ml, 31 mmol) and $Cs_2CO_3$ (83 mg, 0.25 mmol) was then stirred at room temperature for 5 hr. The excess chloroacetonitrile was removed by vacuo to afford a crude oil. The oil was purified by chromatography on 5 g of silica gel (60-200 mesh) eluted with 10%, 50% ethyl acetate/hexanes) providing 28 mg (90% yield) of (1R,2R,3aS,8aS)-1,2,3,3a,8,8a-Hexahydro-4-cyanomethoxy-1-(cis-1-cyclohexyl-1-hydroxylprop-2-en-3-yl)-2- hydroxycyclopent(a)indene 103 as a white foam.

A mixture of nitrile 103 (35 mg, 0.095 mmol), methanol (8 ml) and sodium hydroxide (3 ml of 25% aqueous solution) was then heated at reflux for 2.5 hr, then cooled to room temperature. The solution was diluted with 5 ml of ethyl acetate, washed with 10 ml of 5% HCl and extracted with 4x8 ml of ethyl acetate. The combined ethyl acetate layers were dried over sodium sulfate and concentrated in vacuo affording 35 mg (95% yield) of pure 110 as a white foam: $R_f$=0.04 (2:1 EtOAc/hexanes); $[\alpha]_D$=-192°, c=0.225 (MeOH); $^1$H-NMR ($CDCl_3$) 7.09 (t, 1H, $H_6$), 6.80 (d, 1H, $H_7$), 6.55 (d, 1H, $H_5$), 5.60 (dd, 1H, J=12, J=7, $H_{2'}$), 5.52 (dd, 1H, J=12, J=9, $H_{3'}$), 4.67 (s, 2H, $ArCH_2COOH$), 4.20 (t, 1H, J=7, $H_{1'}$), 3.90 (m, 2H, $H_2$ and $H_{3a}$), 3.12 (m, 1H, $H_{8a}$), 3.06 (m, 1H, $H_1$), 2.93 (m, 2H, $H_{8\alpha}$ and $H_{8\beta}$); $^{13}$C-NMR ($CDCl_3$) 172.5 (e), 153.9 (e), 145.4 (e), 134.9 (o), 133.7 (o), 131.5 (o), 128.0

(o), 117.7 (o), 108.5 (o), 72.8 (o), 64.6 (e), 48.9 (o), 44.4 (o), 44.3 (o), 44.0 (o), 43.8 (o), 39.0 (e), 34.3 (e), 28.6 (e), 28.5 (e), 26.4 (e), 26.0 (e), 25.9 (e); exact mass (Cl) calculated for (M-OH) $C_{23}H_{29}O_4$ 369.1666, found 369.2023.

## EXAMPLE 5

Synthesis of -1R,2R,3aS,8aS)-1,2,3,3a,8,8a-1-Hexahydro-4-carboxy-1-(1-cyclohexyl-1-hydroxyprop-3-yl)-2-hydroxycyclopent(a)indene 120 (Trivial Name: Indanaprost; n=0, p=1, q=1, covalent bond b is in the alpha configuration, K is hydroxy, Y is $-CH_2CH_2-$, $M_1$ is hydrogen, $M_2$ is hydroxy, L and J taken together with the carbon atom bridging those groups form a $C_6$ cycloalkyl group, $S_1$ is -AEQ, wherein A is -O-, E is $-(CH_2)_r$ wherein r is 1, Q is -COOH, and $S_2$ and $S_3$ are hydrogen)

A suspension solution of compound 24 (prepared as described in Example 3) (143 mg, 0.253 mmol) and platinum catalyst supported on activated carbon (49 mg, 0.0126 mmol) in 10 ml of ethanol was stirred at room temperature under 1 atm of hydrogen in a balloon for 5 hr. The mixture was filtered through a pad of celite, washing the pad with ethanol. The filtrate was concentrated in vacuo affording a crude oil. The oil was purified by flash chromatography on 10 g of silica gel (60-200 mesh) eluted with 10% ethyl acetate/hexanes. The procedure afforded 104 mg (82% yield) of (1S(1S),2R,3aS,8aS)-1,2,3,3a,8,8a-Hexahydro-2-(t-butyldiphenylsilyloxy)-1-(cis-1-cyclohexyl-1, -hydroxylprop-3-yl)-4-hydroxycyclopent(a)indene 111 and 12 mg (8% yield) of 121 as white wax.

To a solution of the silyl ether 102 (104 mg, 0.183 mmol) in 7 ml of THF stirred at room temperature with 50 mg of powdered 4Å molecular sieves was added TBAF (2.2 ml of a 1.0 M solution in THF, 2.2 mmol) was also added, followed by stirring for 24 hr. The mixture diluted with 15 ml 5% HCl and extracted with 4 x 15 ml of ethyl acetate. The combined ethyl acetate layers were dried over sodium sulfate and concentrated in vacuo affording a crude oil. The oil was purified by chromatography on 10 g of silica gel (60-200 mesh) eluted with 10%, 30% and 70% ethyl acetate/hexanes providing 48 mg (79% yield) of (1S(1S),2R,3aS,8aS)-1,2,3,3a,8,8a-Hexahydro-2-hydroxyl-(cis-1-cyclohexyl-1-hydroxylprop-3-yl)-4-hydroxycyclopent(a)indene 112 as a white wax.

A mixture of phenol 112 (48 mg, 0.145 mmol), chloroacetonitrile (5 ml, 77mmol) and $Cs_2CO_3$ (142 mg, 0.435 mmol) was stirred at room temperature for 5 hr. The excess chloroacetonitrile was removed by vacuo to afford a crude oil. The oil was purified by chromatography on 5 g of silica gel (60-200 mesh) eluted with 10%, 60% ethyl acetate/hexanes providing 50 mg (93% yield) of (1R,2R,3aS,8aS)-1,2,3,3a,8,8a-1-hexahydro-4-cyanomethoxy-1-(cis-1-cyclohexyl-1-hydroxylprop-3-yl)-2-hydroxycyclopent(a)indene 113 as a white foam.

A mixture of nitrile 113 (21 mg, 0.075 mmol), methanol (7 ml) and sodium hydroxide (2 ml of 25% aqueous solution) was heated at reflux for 2.5 hr, then cooled to room temperature. The solution was diluted with 5 ml of ethyl acetate, washed with 10 ml of 5% HCl and extracted with 4x5 ml of ethyl acetate. The combined ethyl acetate layers were dried over sodium sulfate and concentrated in vacuo affording 19 mg (86% yield) of pure 120 as a white foam. The physical characteristics of compound 120 include: $R_f$=0.02 (2:1 EtOAc/hexanes); $[\alpha]D$=-143°, c=0.225 (MeOH); $^1$H-NMR ($CD_3COCD_3$ and $CD_3COOD$) 7.06 (t, 1H, H6), 6.78 (d, 1H, $H_7$), 6.65 (d, 1H, $H_5$), 4.74 (s, 2H, $ArCH_2COOH$), 3.76 (dd, 1H, J=8, J=9, $H_{3a}$), 3.67 (m, 1H, $H_2$), 3.42 (m, 1H, $H_{1'}$), 3.07 (m, 1H, $H_{8a}$), 2.88 (m, 2H, $H_{8\alpha}$ and $H_{8\beta}$); $^{13}$C-NMR ($CD_3COCD_3$ and $CD_3COOD$) 171.4 (e), 154.0 (e), 145.1 (e), 127.7 (o), 117.1 (o), 108.4 (o), 76.1 (o), 75.8 (o), 64.0 (e), 49.7 (o), 42.9 (o), 42.1 (o), 39.6 (e), 38.2 (e), 33.0 (e), 32.1 (e), 29.3 (e), 29.1 (e), 28.8 (e), 26.2 (e), 25.9 (e); exact mass (Cl) calculated for (M+H-2$H_2O$) $C_{23}H_{29}O_3$ 353.2116, found 353.2110.

## EXAMPLE 6

Synthesis of (1R,2R,3aS,8aS)-1,2,3,3a,8,8a-Hexahydro-4-carboxy-1-(3-cyclohexylprop-1-yl)-2-hydroxycyclopent(a)indene 130 (Trivial Name: Deoxyindanaprost; n=0, p=1, q=1, covalent bond b is in the alpha configuration, K is hydroxy, Y is $-CH_2CH_2-$, $M_1$ is hydrogen, $M_2$ is hydrogen, L and J taken together with the carbon atom bridging those groups form a $C_6$ cycloalkyl group, $S_1$ is -AEQ, wherein A is -O-, E is $-(CH_2)_r$ wherein r is 1, Q is -COOH, and $S_2$ and $S_3$ are hydrogen).

To silyl ether 121 (33 mg, 0.060 mmol, formed as described in Example 5) in 3 ml of THF stirred at room temperature with 20 mg of powdered 4Å molecular sieves was added TBAF (0.6 ml of a 1.0 M solution in THF) followed by stirring for 48 hr. The mixture diluted with 10 ml 5% HCl and extracted with 4 x 10 ml of ethyl acetate. The combined ethyl acetate layers were dried over sodium sulfate and concentrated in vacuo affording a crude oil. The oil was purified by chromatography on 5 g of silica gel (60-200 mesh) eluted with 10%, 30% ethyl acetate/hexanes providing 17 mg (90% yield) of (1S(1S),2R,3aS,8aS)-1,2,3, 3a,8,8a-Hexahydro-2-hydroxyl--(cis-3-cyclohexylprop-1-yl) 4-hydroxycyclopent (a)indene 122 as a white wax.

A mixture of phenol 122 (17 mg, 0.054 mmol), chloroacetonitrile (1.5 ml, 23 mmol) and $Cs_2CO_3$ (53 mg, 0.162 mmol) was stirred at room temperature for 5 hr. The excess chloroacetonitrile was removed by vacuum to afford a crude oil. The oil was purified by chromatography on 5 g of silica gel (60-200 mesh) eluted with 10%, 30% ethyl acetate/ hexanes providing 13 mg (70% yield) of (1R,2R,3aS,8aS)-1,2,3,3a,8,8a-1-hexahydro-4-cyanomethoxy-1-(cis-3-cyclohexylprop-3-yl)-2-hydroxycyclopent(a)indene 123 as a white foam.

A mixture of nitrile 123 (13 mg, 0.038 mmol), methanol (4 ml) and sodium hydroxide (2 ml of 25% aqueous solution) was heated at reflux for 3 hr, then cooled to room temperature. The solution was diluted with 5 ml of ethyl acetate, washed with 10 ml of 5% HCl and extracted with 4x5 ml of ethyl acetate. The combined ethyl acetate layers were dried over sodium sulfate and concentrated in vacuo affording 12 mg (87% yield) of pure 130 as a white foam. The physical characteristics of compound 130 include: $R_f$=0.06 (2:1 EtOAc/hexanes), $[\alpha]_D$=-136°, c=0.5 (MeOH); $^1$H-NMR ($CDCl_3$) 7.10 (t, 1H, $H_6$), 6.83 (d, 1H, $H_7$), 6.56 (d, 1H, $H_5$), 4.69 (s, 2H, $ArCH_2COOH$), 3.81 (dd, 1H, J=8, J=9, $H_{3a}$), 3.70 (m, 1H, $H_2$), 3.07 (m, 1H, $H_{8a}$), 2.92 (dd, 1H, J=10, J=16, $H_{8\beta}$); 2.86 (dd, 1H, J=5, J=16, $H_{8\alpha}$) $^{13}$C-NMR ($CDCl_3$) 172.9 (e), 153.7 (e), 145.7 (e), 135.2 (o), 127.9 (o), 117.9 (o), 108.5 (o), 77.2 (o), 64.5 (e), 50.2 (o), 43.2 (o), 42.2 (o), 40.2 (e), 37.8 (e), 33.5 (e), 33.3 (e), 29.6 (e), 28.9 (e), 26.7 (e), 26.4 (e), 25.5 (e); exact mass (CI) calculated for (M-OH) $C_{23}H_{21}O_3$ 355.2273, found 355.2269.

EXAMPLE 7

Synthesis of 2S(1S),2R,3aS,8aS)-5-Carboxy methoxy-1-(1-cyclohexyl-1-hydroxyprop-2-yn-3-yl)-2-hydroxy-1,2,3,3a,8,8a-hexahydrocyclopent[a]indene 200 (Trivial Name: pseudoindynaprost; n=0, p=1, q=1, covalent bond b is in the alpha configuration, K is hydroxy, Y is $-CH_2CH_2-$, $M_1$ is hydrogen, $M_2$ is hydrogen, L and J taken together with the carbon atom bridging those groups form a $C_6$ cycloalkyl group, $S_2$ is -AEQ, wherein A is $-CH_2-$, E is $-(CH_2)_r$ wherein r is 0, Q is -COOH, and $S_1$ and $S_3$ are hydrogen)

To a solution of p-tolylacetic acid 239 (47.51 g, 316.7 mmol) in 750 ml of carbon tetrachloride heated to reflux under a flowing steam of nitrogen and irradiated with 150 watt white light in a three-necked round bottom flask was added dropwise bromine (17.13 ml, 332.6 mmol) over 60 min. After addition of the bromine was complete, the solution was irradiated and heated for an additional 60 min during which time the product began to precipitate out of solution. Hydrogen bromide, which was generated during the reaction, was bubbled through an aqueous solution of sodium hydroxide. The heterogeneous solution was cooled to 0°C and filtered. The solid was washed with hexanes and dried affording 41.34 g (57% yield) of 4-bromomethylbenzeneacetic acid 238 as a white powder.

To a solution of acid 238 (40.90 g, 178.6 mmol) in 275 ml of THF cooled to 0°C was added $BH_3$/THF complex (223 ml of a 1.0 M solution in THF, 223. mmol) over 40 min. The cooling bath was removed, and the solution as stirred at room temperature for 16 h. The mixture was then quenched by SLOW addition of ca. 350 ml of a saturated aqueous solution of potassium carbonate. The THF of the biphasic mixture was removed in vacuo, and the resulting aqueous phase was extracted with 2 x 300 ml of ether. The combined ether layers were dried over sodium sulfate and concentrated in vacuo affording 38.19 g (99% yield) of 2-(4-Bromomethylphenyl)ethanol 237 as a white solid which was used routinely without further purification.

To a solution of alcohol 237 (38.10 g, 177.2 mmol) in 500 ml of methylene chloride cooled to -10°C was added imidazole (14.48 g, 212.7 mmol) followed by the t-butyldimethylsilyl chloride (29.38 g, 194.9 mmol). The mixture was stirred for 1.5 h at -10°C then diluted with 200 ml of methylene chloride. The methylene chloride layer was washed with 3 x 500 ml of a 5% aqueous solution of hydrochloric acid and 500 ml of a saturated aqueous solution of sodium bicarbonate, dried over sodium sulfate, and concentrated in vacuo providing an oil. The oil was purified by chromatography on ca. 600 g of silica gel (60-200 mesh) eluted with 10% then 20% ethyl acetate/hexanes. The crude oil was applied to the column using a minimal amount of methylene chloride. In this manner, 48.80 g (84% yield) of 4-Bromomethyl-1-(2-t-butyldimethylsilyoxyethyl)benzene 236 was obtained as clear, thin, pale yellow oil.

Through a solution of benzyl bromide 236 (48.80 g,148.3 mmol) of benzyl bromide 236 in 1000 ml of THF cooled to -15°C was bubbled dimethylamine for 20 min. After the first few minutes of introduction of the dimethylamine, a bright yellow-colored solution appeared then slowly faded to a colorless solution over ca. 2 min more time. The mixture was stirred for 22 hours while keeping the temperature below 10°C. The mixture was concentrated in vacuo providing a solid that was dissolved in 25 ml of methylene chloride and washed with 25 ml of a saturated aqueous solution of sodium bicarbonate. The methylene chloride layer was removed, dried over sodium sulfate, and concentrated in vacuo to afford 41.55 g (96% yield) of 1-(2-t-butyldimethylsilyloxyethyl)-4-N,N-dimethylaminomethylbenzene 235 as a yellow oil that partially solidified upon standing.

To a solution of N,N-dimethylbenzylamine 235 (9.20 g, 22.0 mmol) in 16 ml of diethyl ether stirred at ambient temperature was added dropwise n-butyllithium (5.41 ml of a 1.6 M solution in hexane, 8.65 mmol) giving a bright yellow solution. The solution was stirred at ambient temperature for 18 hours during which time it became

a yellow heterogeneous mixture. The reaction was quenched by slow addition of 1-chloro-2-iodoethane (0.76 ml, 8.65 mmol) resulting in ethylene evolution and causing the solvent to reflux gently. The white heterogeneous mixture was diluted with 20 ml water, and the ether layer was removed. The aqueous layer was extracted with 20 ml of diethyl ether. The combined ether layers were washed with a saturated sodium bicarbonate solution, dried over sodium sulfate, and concentrated in vacuo to give a crude yellow oil. This oil was chromatographed eluted with 0%, 2%, 4%, and 8% ethyl acetate/hexanes (v/v) to give 7.98 g of the yellow solid (90% yield) 2-iodo-N,N-dimethylbenzenemethanamine 234 as a thin yellow oil.

To a solution of compound 234 (1.652 g, 6.33 mmol) in 20 ml of methylene chloride cooled to -78°C was added dropwise ethyl chloroformate (33.3 ml, 348 mmol) followed by SLOW dropwise addition of triethylamine (8.82 ml, 63.3 mmol) over 15 min. The mixture was allowed to slowly warm to room temperature over 10 hours with vigorous gas evolution occurring around -15°C. The tan mixture was diluted with 50 ml water and 20 ml methylene chloride. The methylene chloride layer was removed, and the aqueous layer was extracted with 50 ml methylene chloride. The combined methylene chloride layers were washed with 2 x 80 ml 5% HCl and 80 ml of brine, dried over sodium sulfate, and concentrated in vacuo to give a dark thin oil. The oil was chromatographed on 60 g silica gel (60-200 mesh) using hexanes to give 1.281 g (80%) of 233 as a clear colorless oil.

To a 0.1 M solution of 233 (1.8 equivalents) in THF cooled to -100°C (MeOH/$N_2$ bath) was added dropwise a solution of n-butyllithium in hexanes (1.75 equivalents) resulting in pale yellow solution containing compound 232 which was stirred for 10 min. A preformed solution of copper [I] bromide dimethylsulfide complex (1.5 equivalents for 233) containing 0.05 equivalents of copper metal dust and 6 equivalents of lithium bromide, which was 0.1 M in copper [I] bromide dimethylsulfide complex in THF, was added via cannula to the aryl anion formed by the reaction 232. The "bromo copper" solution was formed according to the following procedure. Lithium bromide and copper metal dust were heated under vacuum (ca. 0.5 torr) to remove water. The flask was allowed to cool to room temperature, the copper [I] bromide dimethylsulfide complex was added followed by the THF. The solution, which initially may have some green color that fades over time, was allowed to stir for 10 min at room temperature then cooled to -100°C before being transferred to the aryl anion. Once transferred, the aryl cuprate 231 was allowed to stir for 10 minutes at -100°C before addition of electrophiles (1.0 equivalent).

To a solution of 231 in 100 ml of THF was added via cannula a solution of homochiral ammonium salt 16 (1.88 g, 3.09mmol) in 50 ml methylene chloride precooled to -100°C resulting in a yellow solution that was stirred for 15 minutes and then allowed to warm to -40°C before quenching with 10 ml saturated ammonium chloride solution. The THF layer was removed, and the aqueous layer was extracted with 15 ml ether. The combined organic layers were washed with 2x20 ml of 1:1 ammonia hydroxide /saturated ammonium chloride solution, 20 ml of a saturated ammonium chloride, dried with sodium sulfate, and concentrated in vacuo to give a pale yellow oil. This oil was chromatographed on 60 g silica gel 60-200 mesh, using 200 ml portions of 0%, 3%, 10%, 15%, and 20% ethyl acetate /hexane (v/v) giving 2.04 g of (1S,4R)-1-[1-(2-t-Butyldimethylsiloxyethyl)-4-chloromethyl-3-phenyl]-4-(t-butyldiphenylsilyloxy)-2-phenylsulfonyl-cyclopent-2-ene 230.

To a solution of the acetylene 19 (3.081 g, 13.86 mmol) in 200 ml of ether cooled to 0°C was added n-butyllithium (6.43 ml of a 2.1 M solution in hexanes, 13.49 mmol). The ice bath was removed, and the resulting solution was stirred at ambient temperature for 5 min before HMPA (3.81 ml, 21.88 mmol) was added. The resulting solution was stirred for approximately 2 min at room temperature then transferred via cannula to a solution of vinyl sulfone 230 (5.43 g, 7.29 mmol) in 240 ml of ether. The resulting orange solution was stirred at room temperature for 9 minutes then quenched by addition of about 300 ml of a saturated aqueous solution of ammonium chloride. The ether layer was removed, and the aqueous layer was extracted with 150 ml of ether. The combined ether layers were dried over sodium sulfate and concentrated in vacuo affording an orange oil. The crude oil was purified by chromatography on 220 g of silica gel (60-200 mesh) eluted with 1%, 3%, 5%, 8%, and 10% ethyl acetate/hexanes (500 ml portions) providing 6.59 g (97% yield) of annulated product (1S(1S),2R,3aS, 8aR)-5-(1-t-Butyldimethylsilyloxyeth-2-yl)-2t-butyldiphenylsilyloxy-1(1-cyclohexyl-1-tetrahydropyranoxylprop-2-yn-3-yl)-8a-phenylsulfonyl-1,2,3,3a,8,8a-hexahydrocyclopent[a]indene 201 as a pale yellow foam.

To a solution of bis silyl ether 201 (16.61 g, 17.86 mmol) in 800 ml of THF cooled to -10°C was added TBAF (48.2 ml of a 1.0 M solution in THF, 48.2 mmol), and the resulting solution was stirred for 2 hr. The mixture was diluted with ca. 500 ml of brine, and the organic layer was removed while the aqueous layer was extracted with 400 ml of ether. The combined organic layers were dried over sodium sulfate and concentrated in vacuo affording a crude oil. The oil was purified by chromatography on 300 g of silica gel (60-200 mesh) eluted with 15%, 25%, 30%, 35%, 40%, and 45% ethyl acetate/hexanes (500 ml portions) providing 10.89 g (75% yield) of the alcohol (1S,2R,3aS,8aR)-2-t-Butyldiphenylsilyloxy-1-(1-cyclohexyl-1-tetrahydropyranoxylprop-2-yn-3-yl)-5-(2-hydroxyeth-1-yl)-8a-phenylsulfonyl-1,2,3,3a,8,8a-hexahydrocyclo-pent[a]indene 202 as a very light tan foam.

To a solution of DMSO (2.17 ml, 30.4 mmol) in 70 ml of methylene chloride cooled to -65°C was added

TFAA (2.15 ml, 15.2 mmol), and the resulting solution was stirred at -65° to -60°C for 20 min. To the oxosulfonium salt solution was added via cannula a solution of alcohol 202 (4.03 g, 4.94 mmol) in 70 ml of methylene chloride cooled to -65°C. The resulting solution was stirred for 20 min at ca. -60°C then triethylamine (5.64 ml, 40.5 mmol) was added, and the solution was allowed to warm to ca. -45°C over 15 min then diluted with 10 ml of methanol and 5 ml of triethylamine to cleave any trifluoroacetate ester that may have formed. The solution was stirred for 5 min at ca. -15°C then was quenched by addition of 50 ml of water. The methylene chloride layer was removed, and the aqueous layer was extracted with 50 ml of methylene chloride. The combined methylene chloride layers were washed with 200 ml of a 5% aqueous hydrochloric acid solution and 200 ml of brine, dried over sodium sulfate, and concentrated in vacuo affording a thick oil. The oil was purified by chromatography on 220 g of silica gel (60-200 mesh) eluted with 10%, 15%, 20%, 25%, 35%, and 50% ethyl acetate/hexanes (500 ml portions) providing 1.715 g (43% yield) of the aldehyde [1S(1S),2R,3aS,8aR]-2-t-Butyldiphenylsilyloxy-1-(1-cyclohexyl-1-tetrahydropyranoxylprop-2-yn-3-yl)-5-(2-ethanol)-8a-phenyl sulfonyl-1,2,3,3a,8,8a-hexahydrocyclopent[a]indene 203 as a pale yellow foam.

To a solution of aldehyde 203 (1.710 g, 2.10 mmol) in 84 ml of acetonitrile, 56 ml of THF and 28 ml of water cooled to 0°C open to the atmosphere was added monobasic sodium phosphate (580 mg, 4.20 mmol) then hydrogen peroxide (0.71 ml of a 30% solution in water, 6.30 mmol). To the resulting solution was added dropwise over ca. 5 min a solution of sodium chlorite (712 mg of an 80% mixture, 6.30 mmol) in 28 ml of water. The resulting bright yellow solution was stirred at 0°C for 20 min then quenched by addition of a saturated aqueous solution of sodium bisulfite until the yellow color disappeared. The mixture was acidified by addition of a 5% aqueous hydrochloric acid solution then extracted with 3 x 50 ml of ethyl acetate. The combined ethyl acetate layers were dried over sodium sulfate (use of magnesium sulfate resulted in decomposition of the product upon concentration in vacuo) and concentrated in vacuo providing a pale yellow foam. To this acid foam was added 140 ml of t-butanol heated to 50°C and p-toluenesulfonic acid (200 mg, 1.05 mmol), and the resulting solution was stirred at 50°C for 16.5 h. The mixture was diluted with 150 ml of a concentrated aqueous solution of sodium bicarbonate and 75 ml of water then the t-butanol was removed in vacuo. The aqueous solution was extracted with 3 x 100 ml of ethyl acetate, and the combined ethyl acetate layers were dried over sodium sulfate and concentrated in vacuo to afford an oil. The oil was purified by chromatography on 80 g of silica gel (60-200 mesh) eluted with 40%, 45%, 50%, and 55% ethyl acetate/hexanes containing ca. 0.1% acetic acid (250 ml portions) providing 1037 mg (66% yield) of acid [1S(1S),2R,3aS,8aR]-2-t-Butyldiphenylsilyloxy-5-carboxymethyl-1-(1-cyclohexyl-1-hydroxyprop-2-yn-3-yl)-8a-phenylsulfonyl-1,2,3,3a,8,8a-hexahydrocyclopent[a]indene 204 as a white foam.

To a solution of 20 ml of ammonia and 5 ml of THF cooled to -78 C was added lithium metal (99mg, 14.3mmol). To the lithium solution was then added via cannula sulfone 204(1035mg, 1.39 mmol) in a solution of t-butano1(0.179 ml, 1.90mmol) and 8 ml of THF cooled to -78 C. The resulting mixture was stirred for 5 minutes at -78 C then quenched by addition of 2.5 ml of isoprene followed by addition of 2 g of ammonium chloride. The mixture was allowed to warm to room temperature, slowly letting the ammonia evaporate, then diluted with 20ml of water and extracted with 3 X 20 ml of ethyl acetate. The combined ethyl acetate layers were dried over sodium sulfate and concentrated in vacuo affording a crude oil. The crude oil was purified on ca. 70 g of silica gel (60-200 mesh) eluted with 30%, 40%, and 50% ethyl acetate/hexanes containing 0.1% acetic acid (200 ml portions) affording 700 mg of [1S(1S),2R,3aS,8aS]-2-t-Butyldiphenylsilyloxy-5-carboxymethyl-1-(1-cyclohexyl-1-hydroxyprop-2-yn-3-yl)-1,2,3,3a,8,8a-hexahydrocyclopent[a]indene 205 as a yellow foam:

To a solution of silyl ether 205 (62 mg, 0.102 mmol) in 5 ml of THF at room temperature was added ca. 50 mg of powdered 4Å molecular sieves and TBAF (1.02 ml of a 1.0 M solution in THF, 1.02 mmol). The mixture was stirred for 16 h then diluted with 10 ml of a 5% aqueous hydrochloric acid solution and extracted with 3 x 10 ml of ethyl acetate. The combined ethyl acetate layers were dried over sodium sulfate and concentrated in vacuo affording a pale yellow oil. The oil was purified by chromatography on ca. 8 g of silica gel (60-200 mesh) eluted with 70% the 90% ethyl acetate/hexanes containing ca. 0.5% acetic acid providing 26 mg (69% yield) of pseudoindynaprost 200 as a white foam. Compound 200 has the following physical characteristics: $R_f$ = 0.15 (90/10/0.5 EtOAc/Hexanes/AcOH); $[\alpha]_D$=+162°, c=0.436 (MeOH); $^1$H-NMR ($CDCl_3$) 7.15 (d, 1H, J=8.0, $H_6$ or $H_7$), 7.02 (bs, 2H, $H_6$ or $H_7$ and $H_4$), 4.17 (d, 1H, J=6.7, $H_{1'}$), 4.14 (q, 1H, J=8.6, $H_2$), 3.61 (s, 2H, $ArCH_2CO_2$), 3.57 (bq, 1H, $H_{3a}$), 3.15 (dd, 1H, J=16.7, J=8.4, $H_{8\beta}$), 2.90 (bd, 1H, J=16.7, $H_{8\alpha}$), 2.79 (bq, 1H, $H_{8a}$), 2.64 (m, 1H, $H_{3\alpha}$), 2.30 (t, 1H, J=9.5, $H_1$), 1.90-0.82 (m, 12H, $H_{3\beta}$ and c-hexyl); $^{13}$C-NMR ($CDCl_3$) 176.8 (e), 148.0 (e), 140.3 (e), 132.2 (e), 127.9 (o), 125.5 (o), 125.2 (o), 86.5 (e), 81.9 (e), 78.2 (o), 67.3 (o), 46.9 (o), 45.3 (o), 44.6 (o), 44.1 (o), 40.9 (e), 39.3 (e), 36.2 (e), 28.6 (e), 28.1 (e), 26.4 (e), 25.9 (e); exact mass (Cl, isobutane) calculated for (M+H-$H_2O$) $C_{23}H_{27}O_3$ 351.1960, found 351.1955.

EXAMPLE 8

Synthesis of [1S(1S),2R,3aS,8aS)-5-Carboxy methyl-1-(cis-1-cyclohexyl-1-hydroxyprop-2-en-3-yl)-2-hydroxy-1,2,3,3a,8,8a-hexahydrocyclopent[a]indene 210 (Trivial Name: Z-Pseudoindenaprost; n=0, p=1, q=1, covalent bond b is in the alpha configuration, K is hydroxy, Y is cis, -CH=CH-, $M_1$ is hydrogen, $M_2$ is hydroxy, L and J taken together with the carbon atom bridging those groups form a $C_6$ cycloalkyl group, $S_2$ is -AEQ, wherein A is $-CH_2-$, E is $-(CH_2)_r$ wherein r is 0, Q is -COOH, and $S_1$ and $S_3$ are hydrogen)

To a solution of pseudoindynaprost 200 (115 mg, 0.313 mmol), prepared as described in Example 7 and placed in 10 ml of ethyl acetate stirred at room temperature under 1 atm of hydrogen in a Brown hydrogenator was added a palladium catalyst supported on calcium carbonate and poisoned with lead (133 mg, 0.063 mmol). The mixture was stirred for 2.5 h then filtered through a pad of celite, washing the pad with 3 x 5 ml of a 25% methanol/ethyl acetate mixture. The filtrate was concentrated in vacuo affording a crude oil. The oil was purified by flash chromatography on 20 g of silica gel (230-100 mesh) eluted with 50% then 75% ethyl acetate/hexanes containing ca. 0.5% methanol and 0.2% acetic acid providing 78 mg of pure and 28 mg of >95% pure (by NMR analysis) (combined 92% yield) of Z-pseudoindenaprost 210 as a white powder which could be recrystallized from methylene chloride and hexanes affording a white powder having the following physical characteristics: $R_f$ = 0.3 (99:0.5:0.2 EtOAc/methanol/AcOH); mp 138-9°C; $[\alpha]_D$ = +128°, c=0.123 (MeOH); $^1$H-NMR ($CDCl_3$) 7.16 (d, 1H, J=8.1, $H_6$ or $H_7$), 7.10 (s, 1H, $H_4$), 7.09 (d, 1H, J=8.1, $H_6$ or $H_7$), 5.65 (dd, 1H, J=11.1, J=8.6, $H_{2'}$), 5.44 (bt, 1H, $H_{3'}$), 4.03 (t, 1H, J=7.7, $H_{1'}$), 3.92 (bq, 1H, $H_2$), 3.63 (s, 2H, $ArCH_2CO_2$), 3.61 (dd, 1H, J=18.0, J=7.5, $H_{3a}$), 3.05 (dd, 1H, J=16.8, J=9.4, $H_{8\beta}$), 2.77 (d, 1H, J=16.8, $H_{8\alpha}$), 2.70 (m, 1H, $H_{3\beta}$), 2.51 (m, 2H, $H_1$ and $H_{8a}$), 1.85 (bd, 1H, $H_{3\alpha}$), 1.78-1.55 (m, 6H, c-hexyl), 1.35 (m, 1H, c-hexyl), 1.28-0.84 (m, 4H, c-hexyl); $^{13}$C-NMR ($CDCl_3/CD_3CO_2D$ ca. 40:1) 177.5 (e), 148.6 (e), 140.8 (e), 133.9 (o), 133.7 (o), 131.9 (e), 127.7 (o), 125.3 (o), 77.8 (o), 72.9 (o), 52.1 (o), 46.6 (o), 45.4 (o), 43.9 (o), 40.9 (e), 40.2 (e), 35.8 (e), 28.8 (e), 28.5 (e), 26.3 (e), 26.1 (e), 25.9 (e); exact mass (EI) calculated for ($M^+$-$H_2O$) $C_{23}H_{28}O_3$ 352.2038, found 352.2042.

EXAMPLE 9

Synthesis of [1S(1R),2R,3aS,8aS)-5-Carboxy methyl-1-(1-cyclohexyl-1-hydroxyprop-3-yl)-2-hydroxy-1,2,3,3a,8,8a-hexahydrocyclopent[a]indene 220 (Trivial Name: Pseudoindanaprost; n=0, p=1, q=1, covalent bond b is in the alpha configuration, K is hydroxy, Y is $-CH_2CH_2-$, $M_1$ is hydrogen, $M_2$ is hydroxy, L and J taken together with the carbon atom bridging those groups form a $C_6$ cycloalkyl group, $S_2$ is -AEQ, wherein A is $-CH_2-$, E is $-(CH_2)_r$ wherein r is 0, Q is -COOH, and $S_1$ and $S_3$ are hydrogen).

To a solution of pseudoindynaprost 200 (81 mg, 0.220 mmol), prepared as described in Example 7 and placed in 10 ml of ethyl acetate stirred at room temperature under 1 atm of hydrogen in a Brown hydrogenator was added a 5% palladium on alumina catalyst (46.8 mg, 0.022 mmol). The mixture was stirred for 4.7 h then filtered through a pad of celite, washing the pad with 3 x 5 ml of a 25% methanol/ethyl acetate mixture. The filtrate was concentrated in vacuo affording a crude oil. The oil was purified by chromatography on 10 g of silica gel (60-200 mesh) eluted with 60%, 70%, and 85% ethyl acetate/hexanes containing 0.5% acetic acid (100 ml portions). This procedure afforded 47 mg (68% yield) of pseudoindanaprost 220 as a white powder which could be recrystallized from methylene chloride and hexanes providing a white powder having the following physical characteristics: $R_f$ = 0.3 (70% EtOAc/hexanes containing 0.2% acetic acid); mp 135-6°C, $[\alpha]_D$=+63.8°, c = -.207 (MeOH); $^1$H-NMR 7.12 (d, 1H, J=7.7, $H_6$ or $H_7$), 7.07 (s, 1H, $H_4$), 7.05 (d, 1H, J=7.7, $H_6$ or $H_7$), 3.97 (b, 3H, OH), 3.82 (q, 1H, J=7.7, $H_2$), 3.58 (s, 2H, $ArCH_2CO_2$), 3.49 (q, 1H, J=8.5, $H_{3a}$), 3.35 (m, 1H, $H_{1'}$), 3.14 (dd, 1H, J=16.6, J=9.0, $H_{8\beta}$), 2.76 (d, 1H, J=16.7, $H_{8\alpha}$), 2.52 (m, 1H, $H_{3\beta}$), 2.40 (bq, 1H, $H_{8a}$), 1.86-0.95 (m, 17H, $H_1$, $H_{3a}$, $H_{2'}$, $H_{3'}$ and c-hexyl); $^{13}$C-NMR ($CDCl_3$/MeOD ca. 40:1) 148.4 (e), 140.9 (e), 132.5 (e), 127.4 (o), 125.2 (o), 124.8 (o), 78.6 (o), 76.5 (o), 53.3 (o), 46.0 (o), 45.6 (o), 43.5 (o), 40.9 (e), 37.8 (e), 31.7 (e), 29.1 (e), 29.0 (e), 27.8 (e), 26.4 (e), 26.2 (e), 26.1 (e) (Note: carbonyl signal from acid was not seen due to long relaxation time for this signal); exact mass (CI, isobutane) calculated for (M+H)$C_{23}H_{33}O_4$ 373.2379, found 373.2372.

EXAMPLE 10

Synthesis of [1S(1S),2R,3aS,8aS)-5-[1-Carboxyeth-2-yl)-1-(1-cyclohexyl-1-hydroxyprop-2-yn-3-yl)-2-hydroxy-1,2,3,3a,8,8a-hexahydrocyclopent[a]indene 300 (Trivial Name: Homopseudoindynaprost; n=0, p=1, q=1, covalent bond b is in the alpha configuration, K is hydroxy, Y is $-CH_2CH_2-$, $M_1$ is hydrogen, $M_2$ is hydrogen, L and J taken together with the carbon atom bridging those groups form a $C_6$ cycloalkyl group, $S_2$ is -AEQ, wherein A is $-CH_2-$, E is $-(CH_2)_r$ wherein r is 1, Q is -COOH, and $S_1$ and $S_3$ are hydrogen).

To a solution of alcohol 202(4.160 g, 5.10 mmol), prepared as previously described in Example 7, and placed in 90 ml of methylene chloride stirred at room temperature in a water bath was added methanesulfonyl

chloride (1.58 ml, 20.4 mmol) followed by diisopropylethylamine (4.00 ml, 22.9 mmol). The resulting solution was stirred at ambient temperature for 15 min then washed with 2 x 100 ml of a 5% aqueous hydrochloric acid solution and 100 ml of a saturated aqueous sodium bicarbonate solution. The methylene chloride layer was dried over sodium sulfate and concentrated in vacuo affording the mesylate as a foam. To a solution of the mesylate in 110 ml of acetone was added sodium iodide (34.4 g, 229.4 mmol), and the resulting solution was stirred for 16 h at room temperature. The orange mixture was concentrated in vacuo then diluted with 200 ml of water. The aqueous solution was extracted with 2 x 120 ml of methylene chloride, and the combined methylene chloride extracts were washed with 200 ml of a saturated aqueous solution of sodium bisulfite and 200 ml of brine, dried over sodium sulfate, and concentrated in vacuo affording a mixture of free and protected propargyl alcohol containing iodides. To a solution of this mixture in 75 ml of methylene chloride and 75 ml of methanol stirred at room temperature was added a few crystals of p-toluenesulfonic acid, and the resulting solution was stirred at ambient temperature for 2.25 h. The mixture was diluted with 100 ml of water and 150 ml of a saturated aqueous sodium bicarbonate solution and extracted with 2 x 75 ml of methylene chloride. The combined methylene chloride layers were dried over sodium sulfate and concentrated in vacuo affording an orange oil which was not further purified.

To this oil (4.65 g, 5.52 mmol) in 80 ml of DMSO heated to 50°C was added sodium cyanide (1.082 g, 22.1 mmol), and the resulting orange solution was stirred for 20 min at 50°C. The mixture was diluted with 250 ml of brine and extracted with 3 x 100 ml of ethyl acetate. The combined ethyl acetate layers were dried over sodium sulfate and concentrated in vacuo affording a dark orange oil. The oil was purified by chromatography on 200 g of silica gel (60-200 mesh) eluted with 20%, 25%, 30%, 35%, and 45% ethyl acetate/hexanes (500 ml portions) providing 3.837 g (94% yield) of the nitrile [1S(1S),2R,3aS,8aR]-2-t-Butyldiphenylsilyloxy--(1-cyanoeth-2-yl)-1-(1-cyclohexyl-1-hydroxyprop-2-yn-3-yl)-8a-phenylsulfonyl-,2,3,3a,8,3a-hexahydrocyclopent[a] indene 301 as a pale yellow foam.

To a solution of sulfone 301 (356 mg, 0.480 mmol) in 25 ml of ethanol was added dibasic sodium phosphate (341 mg, 2.40mmol) then the 6% sodium amalgam (1.842 g, 4.80 mmol). The resulting heterogeneous mixture was stirred at 70°C for 1.3 h then cooled to room temperature and quenched by slow addition of about 20 ml of saturated aqueous ammonium chloride solution. The mixture was extracted with 2 X 25 ml of methylene chloride, and the combined methylene chloride layers were washed with 40 ml of a saturated aqueous solution of sodium bicarbonate, dried over sodium sulfate, and concentrated in vacuo providing a dark colored oil. The oil was purified by chromatography on 20 g of silica gel (60-200 mesh) eluted with 10%, 15%, and 20% ethyl acetate/hexanes affording 165 mg (57%) yield of the nitrile [1S(1S),2R,3aS,8aS]-2-t-Butyldiphenylsilyloxy-5-(1-cyanoeth-2-yl)-1-(1-cyclohexyl-1-hydroxyprop-2-yn-3-yl)–1,2,3,3a,8,8a-hexahydrocyclopent[a] indene 302 as a very pale yellow foam.

To a solution of silyl ether 302 (165 mg, 0.275 mmol) in 15 ml of THF stirred at room temperature with ca. 100 mg of powdered 4Å molecular sieves was added TBAF (2.75 ml of a 1.0 M solution in THF). The pale yellow colored solution was stirred at 40°C for 15 h then diluted with 20 ml of a saturated aqueous solution of sodium bisulfate and extracted with 2 x 20 ml of ethyl acetate. The combined ethyl acetate layers were dried over sodium sulfate and concentrated in vacuo affording a thick dark oil. The oil was purified by chromatography on 10 g of silica gel (60-200 mesh) eluted with 55%, 65%, and 75% ethyl acetate/hexanes (100 ml portions) providing 80 mg (80% yield) of the nitrile [1S(1S),2R,3aS,8aS]-5-(1-Cyanoeth-2yl)-1-(1-cyclohexyl-1-hydroxyprop-2-yn-3-yl)-5-hydroxy-1,2,3,3a,8,8a-hexahydrocyclopent[a]indene 303 as a light orange colored foam.

A solution of the nitrile 303 (675 mg, 1.86 mmol) in 40 ml of methanol and 40 ml of a 25% aqueous sodium hydroxide solution (w/w) was heated to 90°C with an oil bath for 5 h. The mixture was allowed to cool to room temperature then was acidified to a pH of ca. 2 by addition of a concentrated aqueous solution of hydrochloric acid. The mixture was extracted with 3 x 50 ml of ethyl acetate, and the combined ethyl acetate layers were dried over sodium sulfate and concentrated in vacuo affording an oil. The oil was partially purified by chromatography on ca. 50 g of silica gel (230-400 mesh) eluted with 75% then 85% ethyl acetate/hexane solutions both containing 1% methanol and ca. 0.2% acetic acid. The partially purified oil was then purified by chromatography on 50 g of silica gel (230-100 mesh) eluted with a 5:5:90 THF/methano/chloroform mixture containing ca. 0.2% acetic acid affording a crude solid. The solid was recrystallized from chloroform and hexanes providing 610 mg (86% yield) of compound 300, which is a very pale yellow solid. A portion of the solid was recrystallized a second time affording a white powder. Compound 300 was found to have the following physical characteristics: $R_f$ = 0.1 (EtOAc/hexanes/methanol, 67:30:3), mp 114-6°C; $[\alpha]_D$ = +143°, c=1.12 (MeOH); $^1$H-NMR ($CDCl_3$) 7.97 (b, 3H, $CO_2H$, OH), 7.11 (d, 1H, J=8.0, $H_6$ or $H_7$), 6.99 (bs, 2H, $H_6$ or $H_7$ and $H_4$), 4.15 (d, 1H, J=7.5, $H_{1'}$), 4.12 (q, 1H, J=9.0, $H_2$), 3.52 (dd, 1H, J=15.2, J=8.5, $H_{3a}$), 3.13 (dd, 1H, J=16.6, J=8.6, $H_{8\beta}$), 2.91 (t, 2H, J=7.4, $H_{2''}$), 2.85 (d, 1H, J=16.6, $H_{8\alpha}$), 2.75 (dd, 1H, J=18.0, J=9.1, $H_{8a}$), 2.64 (m, 3H, J=7.9, $H_{1''}$ and $H_{3\beta}$), 2.28 (t, 1H, J=9.5, $H_1$), 1.85-0.95 (m, 12H, $H_{3\beta}$ and c-hexyl); $^{13}$C-NMR ($CDCl_3$/$CD_3CO_2D$ ca. 15:1) 177.3 (e), 147.8 (e), 139.2 (e), 139.1 (e), 126.8 (o), 125.1 (o), 124.4 (o), 86.6 (e), 81.8 (e), 78.2 (o),

67.2 (o), 46.9 (o), 45.3 (o), 44.8 (o), 44.1 (o), 39.3 (e), 36.0 (e), 35.9 (e), 30.5 (e), 28.6 (e), 28.1 (e), 26.4 (e), 25.9 (e); exact mass (EI) calculated for ($M^+$-$H_2O$) $C_{24}H_{28}O_3$ 364.2038, found 364.2031.

EXAMPLE 11

Synthesis of[1S(1S),2R,3aS,8aS)-5-[1-Carboxyeth-2-yl)-1-(cis,-1-cyclohexyl-1-hydroxyprop-2-en-3-yl)-2-hydroxy-1,2,3,3a,8,8a-hexahydrocyclopent[a]indene 310 (Trivial Name: Z-homopseudoindenaprost; n=0, p=1, q=1, covalent bond b is in the alpha configuration, K is hydroxy, Y is cis -CH=CH-, $M_1$ is hydrogen, $M_2$ is hydroxy, L and J taken together with the carbon atom bridging those groups form a $C_6$ cycloalkyl group, $S_2$ is -AEQ, wherein A is $-CH_2-$, E is $-(CH_2)_r$ wherein r is 1, Q is -COOH, and $S_1$ and $S_3$ are hydrogen)

To a solution of homopseudoindynaprost 300 (110 mg, 0.288 mmol), prepared as described in Example 10 and placed in 10 ml of ethyl acetate stirred at room temperature under 1 atm of hydrogen in a Brown hydrogenator was added a palladium catalyst supported on calcium carbonate and poisoned with lead (133 mg, 0.063 mmol). The mixture was stirred for 1.5 h then filtered through a pad of celite, washing the pad with 3 x 5 ml of a 25% methanol/ethyl acetate mixture. The filtrate was concentrated in vacuo affording a crude oil. The oil was purified by flash chromatography on 20 g of silica gel (230-100 mesh) eluted with 50% then 75% ethyl acetate/hexanes containing ca. 0.5% methanol and 0.2% acetic acid providing 78 mg of pure and 28 mg of >95% pure (by NMR analysis) (combined 92% yield) of Z-homopseudoindenaprost 310 as a white powder which was recrystallized with a mixture of chloroform and hexanes containing a few drops of methanol to help dissolve the crude solid initially. This procedure afforded 81 mg (73% yield) of Z-homopseudoindenaprost 310 as a white powder. Compound 310 was found to have the following physical characteristics: $R_f$ = 0.1 (90:5:5 chloroform/THF/methanol); mp 173-4°C, $[\alpha]_D$ = +101°, c=0.180 (MeOH); $^1$H-NMR ($DCDl_3$) 7.12 (d, 1H, J=8.1, $H_6$ or $H_7$), 7.02 (s, 1H, $H_4$), 7.01 (d, 1H, J=8.1, $H_6$ or $H_7$), 5.65 (dd, 1H, J=11.1, J=8.5, $H_{2'}$), 5.44 (bt, 1H, $H_{3'}$), 4.04 (t, 1H, J=7.7, $H_{1'}$), 3.93 (q, 1H, J=9.1, $H_2$), 3.54 (dd, 1H, J=15.1, J=7.7, $H_{3a}$), 3.04 (dd, 1H, J=16.5, J=7.3, $H_{8\beta}$), 2.93 (t, 2H, J=7.7, $H_{1''}$ and $H_{3\beta}$), 2.75 (d, 1H, J=16.9, $H_{8\alpha}$), 2.69 (m, 3H, J=7.7, $H_{2''}$), 2.50 (m, 2H, $H_1$ and $H_{8a}$), 1.86 (bd, 1H, $H_{3\alpha}$), 1.82-1.57 (m, 6H, c-hexyl), 1.36 (m, 1H, c-hexyl), 1.25-0.81 (m, 4H, c-hexyl); $^{13}$C-NMR ($CDCl_3$/$CD_3CO_2D$ ca. 20:1) 177.4 (e), 148.4 (e), 139.7 (e), 138.9 (e), 133.7 (o), 126.6 (o), 125.1 (o), 124.3 (o), 77.8 (o), 72.9 (o), 52.1 (o), 46.6 (o), 45.4 (o), 43.9 (o), 40.2 (e), 35.8 (e), 35.7 (e), 30.4 (e), 28.8 (e), 28.5 (e), 26.3 (e), 26.0 (e), 25.9 (e); exact mass (CI, isobutane) calculated for (M+H-$H_2O$) $C_{24}H_{31}O_3$ 367.2273, found 367.2254.

EXAMPLE 12

Synthesis of [1S(1R),2R,3aS,8aS)-5-[1-Carboxyeth-2-yl)-1-(1-cyclohexyl-1-hydroxyprop-3-yl)-2-hydroxy-1,2,3,3a,8,8a-hexahydrocyclopent[a]indene 320 (Trivial Name: Homopseudoindanaprost; n=0, p=1, q=1, covalent bond b is in the alpha configuration, K is hydroxy, Y is $-CH_2CH_2-$, $M_1$ is hydrogen, $M_2$ is hydroxy, L and J taken together with the carbon atom bridging those groups form a $C_6$ cycloalkyl group, $S_2$ is -AEQ, wherein A is $-CH_2-$, E is $-(CH_2)_r$ wherein r is 1, Q is -COOH, and $S_1$ and $S_3$ are hydrogen)

To a solution of homopseudoindynaprost 300 (145 mg, 0.380 mmol), prepared as described in Example 10 and placed in 10 ml of ethyl acetate stirred at room temperature under 1 atm of hydrogen in a Brown hydrogenator was added a 5% palladium on alumina catalyst (46.8 mg, 0.022 mmol). The mixture was stirred for 4.7 h then filtered through a pad of celite, washing the pad with 3 x 5 ml of a 25% methanol/ethyl acetate mixture. The filtrate was concentrated in vacuo . affording a crude oil. The oil was purified by chromatography on 20 g of silica gel (60-200 mesh) eluted with 50% and 75% ethyl acetate/hexanes containing 0.5% methanol and 0.2% acetic acid (100 ml portions), followed by recrystallization of the crude solid from chloroform and hexanes. This procedure afforded 110 mg (75% yield) of homopseudoindanaprost 320 as a white powder: $R_f$ = 0.15 (90:5:5:0.2 chloroform/THF/methanol/AcOH); mp 171-2°C, $[\alpha]_D$ =+113°, c = 0.320 (MeOH); $^1$H-NMR ($CDCl_3$/$CD_3CO_2D$ ca. 3:1) 7.09 (d, 1H, J=7.5, $H_6$ or $H_7$), 7.01 (s, 1H, $H_4$), 6.99 (d, 1H, J=7.5, $H_6$ or $H_7$), 3.90 (q, 1H, J=8.5, $H_2$), 3.49 (q, 1H, J=8.4, $H_{3a}$), 3.42 (m, 1H, $H_{1'}$), 3.15 (dd, 1H, J=16.6, J=9.0, $H_{8\beta}$), 2.92 (t, 2H, J=7.8, $H_{2''}$), 2.80 (d, 1H, J=16.6, $H_{8\alpha}$), 2.66 (t, 2H, J=7.8, $H_{1''}$), 2.60 (m, 1H, $H_{3\beta}$), 2.44 (bq, 1H, $H_{8a}$), 1.90-0.97 (m, 17H, $H_1$, $H_{3\alpha}$, $H_{2'}$, $H_{3'}$, and c-hexyl); $^{13}$C-NMR ($CDCl_3$/$CD_3CO_2D$ ca. 3:1) 178.7 (e), 148.0 (e), 139.9 (e), 138.6 (e), 126.3 (o), 124.7 (o), 124.0 (o), 78.5 (o), 76.7 (o), 52.8 (o), 45.7 (o), 45.4 (o), 43.0 (o), 40.5 (e), 37.6 (e), 35.6 (e), 31.1 (e), 30.2 (e), 28.9 (e), 28.4 (e), 27.5 (e), 26.2 (e), 26.04 (e), 25.97 (e), 25.89 (e); exact mass (EI) calculated for ($M^+$) $C_{24}H_{34}O_4$ 386.2457, found 386.2456.

EXAMPLE 13

Synthesis of [2R, 3R(1R), 3aS]-7-Carboxymethoxy-3-(1-cyclohexyl-1-hydroxyprop-3-yl)-2-hydroxy-2,3,3a,4,5, 9b-hexahydro-1H-benz[e]indene 520 (Trivial Name: Tetralanaprost; n=1, p=1, q=2, covalent bond b is in the alpha configuration, K is hydroxy, Y is cis -CH=CH-, $M_1$ is hydrogen, $M_2$ is hydrogen, L and J taken together with the carbon atom bridging those groups form a $C_6$ cycloalkyl group, $S_3$ is -AEQ, wherein A is -O-, E is $-(CH_2)_r$ wherein r is 1, Q is -COOH, and $S_1$ and $S_2$ are hydrogen).

A solution of commercially available phenethyl alcohol 501 (59.0 g, 2552.2 mmol) in 750 ml of methylene chloride was cooled to 0 C. Diisopropylethylamine(66.7 ml, 383.1mmol) was added all at once followed by dropwise addition over 15 minutes of the chloromethyl methyl ether (29.1 ml, 383.1 mmol) resulting in some fuming. The reaction was stirred for 21 hours during which time it was allowed to warm to room temperature. 10 ml of water was added to the reaction to quench any excess chloromethyl methyl ether, and the reaction was diluted with 250 ml of methylene chloride. The solution was brominated, washed with 2 X 500 ml of 5% aqueous HCl solution and 500 ml of brine, dried over sodium sulfate, and concentrated in vacuo providing a crude oil. The oil was chromatographed on about 1200 g silica gel(60-200 mesh), eluted with 12%, 20%, and 25% ethyl acetate/hexanes affording 54.54 g (78% yield) of the arene 2-(2-Bromo-5-methoxyphenyl)-1-methoxymethoxyethane 503 as a clear, almost colorless oil.

To a solution of arene 503 (6.77 g, 24.6mmol) in 80 ml of THF cooled to -78 C was added t-butyllithium (29.4 ml of a 1.6 M solution in pentane, 47.1 mmol) resulting in an orange solution. This was stirred for 40 minutes at -78 C then transferred via cannula to an acetylenic copper solution formed from the mixture of 0.6 M (23.5 mmol) THF/diisopropyl sulfide solution of copper bromide [I] dimethyl sulfoxide complex and an acetylenic anion formed from the mixture of 3-methoxy-3-methylbutlyne in THF to which n-Butyllithium had been added. To this solution was added the ammonium salt 16 (13.00 g, 21.4 mmol) previously described in Example 2. The solution was stirred at -78° C for 50 minutes, and quenched by addition of 300 ml of saturated aqueous ammonium chloride solution. The mixture was warmed to room temperature, and extracted with 2 X 250 ml of methylene chloride, and the combined organic layers were washed with 400 ml of a 1:1 saturated aqueous ammonium chloride/concentrated ammonia hydroxide solution, and 400 ml of saturated aqueous ammonium chloride solution. The organic layer was dried over sodium sulfate and concentrated in vacuo to provide an orange oil. The oil was chromatographed upon about 500 g of 60-200 mesh silica gel, and eluted with 20%, 25%, 30%, and 35% ethyl acetate/hexanes providing 11.05 g (79% yield) of the product (1R,4R)-4-t-Butyldiphenylsilyloxy-1-[4-methoxy-2-(2-methoxymethoxyethyl)-1-phenyl]-2-phenylsulfonylcyclopent-2-ene 504 as a dark oil.

A solution of the MOM acetal 504 (8.15 g, 12.4 mmol) in 500 ml of isopropyl alcohol with a catalytic amount of p-toluenesulfonic acid (0.284 g, 1.5 mmol) was heated to reflux for 18 h. The solution was then allowed to cool to room temperature over 4 h. The mixture was concentrated in vacuo affording an orange oil which was dissolved in 500 ml of methylene chloride, washed with 400 ml of a saturated aqueous solution of sodium bicarbonate, dried over sodium sulfate and concentrated in vacuo providing 7.10 g (95% yield) of the alcohol (1R,4R)-4-t-Butyldiphenylsilyloxy-1-[2-(2-Hydroxyethyl)-4-methoxy-1-phenyl]-2-phenylsulfonyl cyclopent-2-ene 505 as a yellow/orange foam.

To a solution of triphenylphosphine (0.979 g, 3.73 mmol) in 25 ml of methylene chloride stirred at room temperature was added NCS (0.517 g, 3.88 mmol), and the solution was cooled to 0°C for 10 min. The mixture was monitored by TLC to insure that no unreacted triphenylphosphine was present in the solution (in some cases more NCS was added until all the triphenylphosphine had reacted). A solution of alcohol 505 (1.757 g, 2.87 mmol) in 50 ml of methylene chloride cooled to 0°C was then added via cannula to the chlorinating reagent providing a purple solution which was stirred for 90 min at 0°C then quenched with 75 ml of water. The methylene chloride layer was removed and the aqueous layer was extracted with 75 ml of methylene chloride. The combined methylene chloride layers were washed with 150 ml of brine, dried over sodium sulfate, and concentrated in vacuo providing a purple oil. The oil was chromatographed on 80 g silica gel (60-200 mesh) eluted with 10%, 15% and 20% ethyl acetate/hexanes affording 1.600 g (87% yield) of the chloride (IR, 4R)-4-t-Butyldiphenylsilyloxy-1-[2-(2-chloroethyl)-4-methoxy-1-phenyl]-2-phenysulfonylcyclopent-2-ene 506 as a very pale yellow solid powder.

A solution of acetylene 19 (3.680 g, 16.5 mmol) in 130 ml of ether was cooled to 14°C. n-Butyllithium (9.16 ml of a 1.8 M solution in hexanes, 16.5 mmol) was added dropwise to the acetylene solution resulting in a yellow colored mixture which was stirred for 10 min before 3.90 ml of HMPA was added. The anion solution was stirred for 10 min more at 14°C then transferred via cannula in 2 min to a solution of chloride 506 (5.274 g, 8.24 mmol) in 130 ml of ether cooled to 14°C. The dark orange reaction mixture was stirred for 4 min more in a 14°C water bath. The reaction was then quenched with the addition of 250 ml of a saturated aqueous ammonium chloride solution. The ether layer was removed, and the aqueous layer was extracted with 100 ml of ether. The combined ether layers were dried over sodium sulfate and concentrated in vacuo to give a dark oil. The oil was chromatographed on ca. 300 g silica gel (60-200 mesh) eluted with 10%, 15%, 25%, and 35% ethyl acetate-/hexanes providing 6.46 g (95%) of annulated product 507 as a yellow foam.

A solution of sulfone 507 (6.46 g, 7.82 mmol) in 1000 ml of absolute ethanol was stirred with dibasic sodium phosphate (5.55 g, 39.1 mmol). 6% sodium amalgam (30.0 g, 78.2 mmol) was added to the ethanol solution, and the resulting yellow colored mixture was heated to reflux for 5 h then cooled to room temperature for 1 h. The mixture was cooled to 0°C and 500 ml of a saturated aqueous solution of ammonium chloride was added SLOWLY, and the mixture was allowed to warm to room temperature over 12 h. The biphasic mixture was extracted with 1 x 800 ml and 1 x 350 ml of methylene chloride, and the combined extracts were concentrated in vacuo then diluted with 100 ml of water, 300 ml of a saturated aqueous ammonium chloride solution, and 500 ml of methylene chloride. The methylene chloride layer was removed and the aqueous layer was extracted with 100 ml of methylene chloride. The combined methylene chloride layers were dried over sodium sulfate and concentrated in vacuo providing an orange oil; the orange oil was dissolved in 80 ml of chloroform, 550 ml of methanol, and 2 ml of water. Toluenesulfonic acid (0.297 g, 1.56 mmol) was added, and the solution was heated to reflux for 108 h during which time it became dark brown in color. The mixture was cooled to ambient temperature over 12 h then diluted with 750 ml of methylene chloride and washed with 750 ml of a saturated aqueous sodium bicarbonate solution. The aqueous bicarbonate layer was extracted with 250 ml of methylene chloride and the combined methylene chloride layers were dried over sodium sulfate and concentrated in vacuo affording a dark oil. The oil was chromatographed on ca. 300 g of silica gel (60-200 mesh) eluted with 15%, 22%, 30%, 40%, 50%, and 60% (500 ml portions) of ethyl acetate/hexanes providing 1.80 g (65% yield for the desulfonylation/deprotection steps) of the diol [2R, 3S(1S), 3aS, 9bS]-3-(1-Cyclohexyl-1-hydroxyprop-2-yn-3-yl)-2-hydroxy-7-methoxy-2,3,3a,4,5,9b-hexahydro-1H-benz[e]indene 508 as a white solid. The diol 508 was normally used without further purification but could be recrystallized from ether/hexanes affording white needles.

To a solution of aryl methyl ether 508 (1.31 g, 3.73 mmol) in 95 ml of THF was added lithium diphenylphosphide (95.2 ml of a 0.47 M solution in THF, 44.8 mmol) via syringe resulting in a red solution which was heated to reflux and stirred for 28 h. (The lithium diphenylphosphide was prepared by adding chlorodiphenylphosphine (1 equivalent) to a solution of excess lithium wire (4 equivalents) in THF at room temperature and letting the resulting deep red solution stir for 36 h at room temperature. The solution was then stored in the freezer (-20°C) under argon for up to 6 months with little if any loss of activity.) The mixture was allowed to cool to room temperature then quenched with slow addition of ea. 200 ml of 5% aqueous hydrochloric acid. After stirring for 12 h with the acid, the mixture was diluted with Ca. 100 ml of brine and extracted with 3 x 200 ml of ethyl acetate. The ethyl acetate layers were combined, dried over sodium sulfate, and concentrated in vacuo providing a heavy yellow oil. The oil was chromatographed upon 300 g silica gel (60-200 mesh) eluted with 35%, 40%, 45%, 50%, 55%, and 60% ethyl acetate/hexanes (500 ml portions) affording 1.21 g (95% yield) of the phenolic diol [2R, 3S(1S), 3aS, 9bS]-3-(1-Cyclohexyl-1-hydroxyprop-2-yn-3-yl)-2,7-dihydroxy-2,3,3a,4,5,9b-hexahydro-1H-benz[e]indene 509 as a white foam.

To a solution of propargyl alcohol 509 (241 mg, 0.709 mmol) in 50 ml of absolute ethanol was added sodium bicarbonate (376 mg, 3.54 mmol) and 5% platinum on carbon catalyst (1.38 g, 0.354 mmol). The mixture was stirred under 1 atm of hydrogen in a Brown hydrogenator for 4.5 h at ambient temperature. The mixture was filtered (gravity), and the cake washed with 2 x 30 ml of absolute ethanol. The combined filtrate was concentrated in vacuo providing 510 as a thick oil. It should be noted that diol phenol 510 was not a very stable compound, and it was best to proceed with the next step immediately. The oil was used without further purification.

To a solution of phenol 510, obtained from the aforementioned procedure, in 15 ml of chloroacetonitrile was added cesium carbonate (508 mg, 1.56 mmol), and the mixture was stirred for 1.75 h at room temperature. The mixture was diluted with 20 ml of ether and filtered through a 2 cm pad of celite. The pad was washed with 3 x 25 ml of ethyl acetate, and the combined filtrates were concentrated in vacuo affording a dark orange oil. The oil was chromatographed on ca. 35 g of silica gel (60-200 mesh) eluted with 15%, 30%, and 50% THF-/methylene chloride (200 ml portions) providing 171 mg (63% yield for the two steps) of nitrile [2R, 3R(1R), 9bS]-7-Carboxymethoxy-3-(1-cyclohexyl-1-hydroxyprop-3-yl)-2-hydroxy-2,3,3a,4,5,9b-hexahydro-1H-benz[e]indene 511 as a very pale yellow solid.

A solution of nitrile 511 (245 mg, 0.640 mmol) in 15 ml of methanol and 15 ml of a 25% (w/w) aqueous sodium hydroxide solution was heated to reflux for 12 h. The solution was cooled 0°C and slowly acidified to a pH of approximately 2 (monitored by universal pH paper) by addition of a 50% concentrated aqueous hydrochloric acid solution/water mixture. The mixture was then extracted with 3 x 60 ml of ether. Use of ethyl acetate instead of ether resulted in complete destruction of the product when the ethyl acetate was removed in vacuo due presumably to trace amounts of acetic acid present. The combined ether layers were concentrated in vacuo to provide a thick oil. This oil was relatively unstable and had to be purified immediately. purification was accomplished by placing the oil on a 4 cm diameter by 10 cm long column of silica gel (60-200 mesh) quickly eluted with ethyl acetate until an upper $R_f$ impurity had been removed. The column was then eluted with 15%, 25% and 50% methanol/ethyl acetate to obtain the acid. In this manner 220 mg (86% yield) of tetralanaprost was

obtained as a white powder. Tetralanaprost 520 was determined to have the following physical characteristics: $R_f$ = 0.15 (94:5:1 EtOAc/MeOH/AcOH); mp 110-2°C; $[\alpha]_D$=+30.2°, c=0.450 (MeOH); $^1$H-NMR ($CDCl_3$) 7.04 (d, 1H, J=8.3, $H_9$), 6.73 (dd, 1H, J=8.3, J=2.4, $H_8$), 6.67 (d, 1H, J=2.4, $H_6$), 4.64 (s, 2H, $OCH_2CO_2H$), 4.20 (m, 2H, $H_2$ and $H_{1'}$), 3.26 (q, 1H, $H_{9b}$), 2.75 - 2.28 (m, 4H), 1.92 - 0.92 (m, 22H); $^{13}$C-NMR (MeOD/$CDCl_3$ ca. 1:10) 157.1, 139.5, 133.5, 130.4, 115.1, 113.g, 78.7, 77.0, 68.1, 53.9, 44.7, 44.5, 43.1, 38.2, 33.0, 31.3, 30.4, 30.3, 29.5, 29.0, 27.6, 27.4, 27.3 (Note: acid carbonyl carbon signal not observed due to long relaxation time); exact mass (Cl, isobutane) calculated for (M+H-$HOCO_2H$) $C_{22}H_{30}O_2$ 327.2324, found 327.2318.

EXAMPLE 14

Synthesis of [2R,3S(1S),3aS,9bS]-7-Carboxy methoxy-3-(1-cyclohexyl-1-hydroxyprop-2-yn-3-yl)-2-hydroxy-2,3,3a,4,5,9b-hexahydro-1H-benz[e]indene 530 (Trivial Name: Tetralynaprost; n=0, p=1, q=2, covalent bond b is in the alpha configuration, K is hydroxy, Y is -C≡C-, $M_1$ is hydrogen, $M_2$ is hydroxy, L and J taken together with the carbon atom bridging those groups form a $C_6$ cycloalkyl group, $S_3$ is -AEQ, wherein A is -0-, E is $-(CH_2)_r$ wherein r is 1, Q is -COOH, and $S_1$ and $S_2$ are hydrogen).

The phenol 509 (1090 mg, 3.21 mmol), prepared as described in Example 13, was stirred at room temperature in a heterogeneous mixture of 35 ml of chloroacetonitrile and cesium carbonate (2.00 g, 6.16 mmol) for 6 h. The dark colored mixture was then filtered through a 2 cm pad of celite which was washed with 3 x 40 ml of ether and 3 x 40 ml of methanol. The filtrate was diluted with 250 ml of brine and extracted with 3 x 150 ml ethyl acetate. The combined ethyl acetate layers were dried over sodium sulfate and concentrated in vacuo providing a dark thick oil. The oil was chromatographed on ca. 150 g silica gel (60-200 mesh) eluted with 10%, 14%, 18%, and 21% THF/methylene chloride affording 930 mg (75% yield) of [2R, 3S(1S), 3aS, 9bS]-7-Cyanomethoxy--(1-cyclohexyl-1-hydroxyprop-2-yn-3-yl)-2-hydroxy-2,3,3a,4,5,9b-hexahydro-1H-benz[e]indene 521, a very pale yellow solid. The nitrile was normally used without further purification but could be recrystallized from 10% ethyl acetate/THF and hexanes affording a pale yellow powder.

A solution of nitrile 521 (1030 mg, 2.72 mmol) in 50 ml of methanol and 25 ml of a 25% (w/w) aqueous sodium hydroxide solution was heated to reflux for 18 h then allowed to cool to room temperature over 2 h. The solution was cooled 0°C and slowly acidified by addition of ca. 150 ml of a 10% aqueous hydrochloric acid solution. The product "fluffed-out" of solution and was collected by filtration. The almost white solid was washed with 3 x 50 ml of water and 5 x 50 ml hexanes and dried providing 813 mg (75% yield) of tetralynaprost 530 as a white solid having the following physical characteristics: $R_f$ = 0.15 (94:5:1 EtOAc/MeOH/AcOH); mp 87-9°C (partially decomposed); $[\alpha]_D$=+91.5°, c=0.180, (MeOH); $^1$H-NMR ($CD_3CO_2D$/$CDCl_3$ ca. 1:20) 7.05 (d, 1H, J = 8.4 $H_9$), 6.73 (dd, 1H, J = 8.4, J = 2.3, $H_8$), 6.68 (d, 1H, J = 2.3, $H_6$), 4.65 (s, 2H, $OCH_2CO_2H$) 4.28 - 4.17 (m, 2H, $H_2$ and $H_{1'}$), 3.24 (q, 1H, J = 8.3, $H_{9b}$), 2.94 (b, 3H, OH and $Co_2H$), 2.75 - 2.22 (m, 4H, $H_{1\beta}$, $H_5$ and $H_3$), 2.00 - 1.61 (m, 8H, $H_{3a}$, $H_{1\alpha}$, $H_4$ and c-hexyl), 1.47 (m, 1H, c-hexyl), 1.43 - 1.03 (m, 6H, c-hexyl); $^{13}$C-NMR ($CD_2CO_2D$) 171.4, 157.8, 140.0, 134.9, 131.2, 115.8, 114.7, 88.5, 84.1, 79.1, 68.2, 66.4, 46.6, 46.3, 45.3, 45.0, 38.6, 29.6, 29.5, 28.4, 28.2, 27.8 (Note: any signals from ca. 36 to 30 ppm were obscured by the solvent signal); exact mass (EI) calculated for (M+) $C_{24}H_{30}O_5$ 398.2093, found 398.2089.

EXAMPLE 15

Synthesis of (1R, I'S, 2R, 3aS, 9aS)-2, 3, 3a, 4, 9, 9a-Hexahydro-5-carboxymethoxy-1-(1′ cyclohexyl-1′-hydroxyprop-2′-yn-3′-yl)-2-hydroxy- 1H-benz[f]indene 600 (Trivial Name: Isotetralynaprost; n=1, p=1, q=1, covalent bond b is in the alpha configuration, K is hydroxy, Y is -C≡C-, $M_1$ is hydrogen, $M_2$ is hydroxy, L and J taken together with the carbon atom bridging those groups form a $C_6$ cycloalkyl group, $S_1$ is -AEQ, wherein A is -0-, E is $-(CH_2)_r$ wherein r is 1, Q is -COOH, and $S_2$ and $S_3$ are hydrogen).

A mixture of commercially available 2,3-dimethoxybenzoic acid 601 (50.0 g, 0.274 mol) and $SOCl_2$ (100 mL, 1.37 mmol) was stirred at room temperature (Drierite tube) for 17 hr. The excess $SOCl_2$ was evaporated using a water aspirator with a hot water bath; the last traces of $SOCl_2$ were removed by azeotropic distillation with $CCl_4$ (2x50 mL), affording the crude acid chloride as easily-sublimed needles.

A solution of the crude acid chloride in $CH_2Cl_2$ (75 mL) was added dropwise to 2-methyl-2-amino-1-propanol (57.0 mL, 0.598 mmol) in $CH_2Cl_2$ (140 mL) at 0°C (Drierite tube) over 45 min. A white precipitate of 2-methyl-2-amino-1-propanol hydrochloride formed after a few minutes. The mixture was stirred an additional 1 hr at room temperature, filtered, and the filter cake washed with $CH_2Cl_2$ (2x50 mL). Evaporation of the combined filtrates afforded 76.9 g of crude amide 2,3-Dimethyl-N-(1-hydroxy-2-methyl-1-prop-2-yl)benzamide 602 which was recrystallized from EtOH/$H_2O$ to afford 62.23 g of white needles and 14.8 g of crude amide from the mother liquor. The latter was subjected to chromatography (250 g 60-200 mesh silica gel, 50% to 100% EtOAc/hexane gradient) to afford an additional 7.39 g of pure amide 602, for a total of 69.62 g, 0.274 mol, 99% yield, mp 86-88°C.

To a mechanically-stirred solution of $SOCl_2$ (56 mL, 0.768 mol) in $CH_2Cl_2$ (120 mL) at 5°C (Drierite tube) was added dropwise a solution of amide 602 (62.23 g, 0.246 mol) in $CH_2Cl_2$ (300 mL) at a rate to maintain the reaction temperature below 10°C over 70 min. Stirring was continued for an additional 10 min at this temperature at which time the mixture was cautiously basified by slow addition of 10% NaOH (1 L), maintaining the reaction temperature below 25°C. After separation of the layers, the aqueous layer was extracted with $CH_2Cl_2$ (200 mL). The combined organic layers were washed with saturated NaCl (200 mL), dried ($Na_2SO_4$) and evaporated to afford 56.0 g of white solid. Recrystallization ($CCl_4$/hexane, 3 crops) afforded pure 2-(2′,3′-Dimethoxyphenyl)-4,4-dimethyl-2-oxazoline 603 as white prisms (55.06 g, 0.234 mol, 95% yield).

To a mechanically-stirred solution of bis-methyl ether 603 (46.23 g, 0.196 mol) in THF (240 mL) at 0°C was added MeMgBr (136 mL of a 2.89 M solution in ether, 0.393 mol) dropwise at a rate to maintain the reaction temperature <10°C. The cooling bath was removed and stirring continued for 48 hr. The reaction was quenched by drop-wise addition of saturated $NH_4Cl$ (100 mL), then diluted with deionized water (1 L). The layers were separated and the aqueous phase extracted with ether (3x200 mL). The combined organics were washed with saturated NaCl (200 mL), dried ($Na_2S0_4$) and the solvent evaporated to afford the crude oxazoline 604 (48.8 g) as an oil. Short-path distillation (100-102°C, 0.4 mm) gave pure 4,4-Dimethyl-2-[(3′-methoxy-2′-methyl)phenyl]-2-oxazoline 604 as a colorless oil (41.17 g, 0.188 mol, 96% yield).

To a solution of the oxazoline 604 (3.96 g, 18.1 mmol) in THF (70 mL) at -78°C was added nBuLi (7.3 mL of a 2.58 M solution in hexane, 18.8 mmol) slowly, followed by warming to 0°C for 20 min. The resultant deep-red anion solution was cooled to -78°C and transferred via cannula over 8 min into a vigorously-stirred slurry of CuI (1.71 g, 8.98 mmol) in THF (70 mL) at -78°C. This mixture was stirred 60 min, during which time it became almost homogeneous and the color darkened somewhat. The resultant cuprate solution was transferred via cannula over 15 min into a vigorously-stirred solution of ammonium salt 16 (5.21 g, 8.58 mmol) in THF (70 mL) at -78°C. The red cuprate color was converted to a tan-green (almost black) color as quickly as the cuprate was transferred. After stirring 2 hr at -78°C, the nearly homogeneous reaction mixture was quenched with saturated $NH_4Cl$ (100 mL) and the cooling bath removed. When the mixture had warmed to room temperature, it was diluted with 1:1 concentrated $NH_4OH$/saturated $NH_4Cl$ (200 mL) and extracted with $CH_2Cl_2$ (3x200 mL). The combined organic layers were washed with 1:1 concentrated $NH_4OH$/ saturated $NH_4Cl$ (2x200 mL) and deionized water (200 mL). Drying ($Na_2SO_4$) and evaporation afforded 8.30 g of dark, foamy glass. Dissolution of this material in toluene (50 mL), slow addition of hexane to the cloud point, and cooling in the freezer afforded 3.61 g of pure (1R,4R)-4-(t-Butyldiphenyl silyloxy-1-[1′-(4″, 4″-dimethyl-2″-oxazolinyl)-3′-methoxy-2′-tolyl)-2-phenylsulfonyl-2-cyclopentene 605 as small needles. Evaporation of the mother liquor and chromatography of the residue (200 g 60-200 mesh silica gel, 10% to 30% EtOAc/hexane gradient) afforded an additional 1.08 g of 605 for a total of 4.69 g, 6.90 mmol, 80% yield.

To a solution of oxazoline 605 (9.28 g, 13.6 mmol) in $CH_2Cl_2$ (100 mL) at 0°C was added MeOTf (1.65 mL, 14.6 mmol). After 100 min, glacial HOAc (1.75 mL, 30.6 mmol) then $NaBH_3CN$ (3.20 g, 50.9 mmol) in THF (50 ml) were added sequentially and the cooling bath removed. The solution was stirred 15 hr the diluted with deionized water (500 mL) and extracted with $CH_2Cl_2$ (3x100 mL). The combined organic layers were washed with 10% $Na_2CO_3$ (100 mL), saturated NaCl (100 mL), dried ($Na_2CO_3$ and evaporated to afford pure (1R,4R)-4-(t-Butyldiphenylsilyloxy)-1-{3′-[N-(1-hydroxy-2″-methylpropyl)-N-methylamino)methyl}-1′-methoxy-2′-tolyl) -2-phenylsulfonylcyclopent-2-ene 606 as a white foam, 8.98 g, 12.9 mmol, 95% yield.

To a vigorously-stirred solution of amino alcohol 606 (8.98 g, 12.9 mmol) in $ClCO_2Et$ (100 mL) at -78°C was added $Et_3N$ (17.9 mL, 128 mmol) dropwise and the cooling bath allowed to warm. On some runs, the mixture solidified. **CAUTION** as the mixture warms to ca. 0°C, vigorous $CO_2$ evolution occurs, accompanied by rapid exotherm; the reaction vessel must be adequately vented: (An explosion occurred on one 13 mmol run, apparently due to inadequate ventilation). When the mixture had reached room temperature and $CO_2$ evolution ceased, it was diluted with iced deionized water (500 mL) and extracted with $CH_2Cl_2$ ((3x100 mL). The combined organics were washed with 5% HCl (100 mL), saturated NaCl (100 mL), dried ($Na_2SO_4$) and evaporated to afford 14.13 g of brown oil. Chromatography (200 g of 60-200 mesh silica gel, 30% EtOAc/hexane) afforded pure (1R,4R)-4-(t-Butyldiphenyl silyloxy)-1-(3′-chloromethyl-1′-methoxy-2′-tolyl)-2-phenylsulfonylcyclopent-2-ene 607 as a clear, very heavy oil, 12.9 mmol, 99% yield. Crystallization from toluene/heptane gave a low recovery of large prisms, mp 45.5-47°C.

To a solution of acetylene 19 (5.77 g, 26.0 mmol) in THF (100 mL) at 0°C was added nBuLi (10.6 mL of a 2.58 M solution in hexane, 27.3 mmol) over 4 min. The solution was stirred 10 min, at which time the ice bath was replaced by a water bath, and stirring continued for 15 min to effect thermal equilibration. HMPA (1.0 mL) and a solution of vinyl sulfone 607 (8.17 g, 12.9 mmol) in THF (100 mL) were added sequentially, the latter over 5 minutes via cannula. The resultant golden yellow solution was stirred 90 min, then quenched with saturated $NH_4Cl$ (100 mL). The mixture was diluted with deionized water (1 L), and extracted with hexane (3x200 mL). The combined organics were washed with deionized water (200 mL) dried ($Na_2SO_4$) and evaporated to

afford a heavy oil. Chromatography (350 g 60-200 mesh silica gel, 10% to 20% EtOAc/hexane gradient) afforded recovered compound 604 (3.08 g, 13.9 mmol, 53%). Continued elution afforded tricycle (1R,1′S.2R,3aS,9aR)-2,3,3a,4,9,9a-Hexahydro-2-(t-Butyldiphenylsilyloxy)-1-(1′-cyclohexyl-1′-tetrahydropyr anoxyprop-2′-yn-3′-yl)-5-methoxy-9a-phenylsulfonyl-1H-benz[f]-indene 608 as a foam, 7.67 g, 9.39 mmol, 73% yield.

A solution of sulfone 608 (7.46 g, 9.13 mmol), pTsOH-$H_2O$ (0.3552 g, 1.87 mmol) and 2:1 MeOH/$CHCl_3$ (150 mL) was heated at reflux for 72 hr then cooled to room temperature and washed with 10% $Na_2CO_3$ (300 mL). The aqueous layer was extracted with $CH_2Cl_2$ (2x100 mL). The combined organics were washed with saturated NaCl (100 mL) dried ($Na_2SO_4$) and evaporated to afford 6.66 of solid (1R,1S,2R,3aR,9aS) -2,3,3a,4,9-pentahydro-1-(1′-cyclohexyl-1′-hydroxyprop-2′-yn-3′ -yl)-2-hydroxy-5-methoxy-9a-phenyl-sulfonyl-H-benz[f] indene 609. Recrystallization from $CHCl_3$/hexane afforded pure 609 as tiny needles.

A mixture of sulfone diol 609 (3.67 g, 7.67 mmol), $Na_2HPO_4$ (r.42 g, 38.2 mmol), sodium amalgam (29.39 g, 6% sodium, 76.7 mmol) and EtOH (100 mL) was heated at reflux for 1 hr, cooled on an ice-water bath, quenched with saturated $NH_4Cl$ (100 mL) and the cooling bath removed. When the amalgam had been destroyed (generally overnight), the mixture was diluted with deionized water (500m) and extracted with $CH_2Cl_1$ (3x100 mL). The combined organics were washed with saturated NaCl (100 mL), dried ($Na_2SO_4$) and evaporated to afford a brown foam, 2.46 g. This mixture was subjected to chromatography (Merck C column, flow rate 9 mL/min, W detection at 254 nm, 10% to 50% EtOAc/hexane gradient, employing 1L at each 10% step), to afford two fractions.

The first fraction contained (1R, 1′S, 2R, 3aS, 9aR)-2,3,3a,4,9,9a-Hexahydro-1-(1′-cyclohexyl-1′-hydroxyprop-2′-yn-3′-yl)-2-hydroxy-5-methoxy-1H-benz[f]indene 701 as a foam, 0.65 g, 1.83 mmol, 24% yield. The physical characteristics of compound 701 include: Ir: 2.92 (broad, OH), 4.55 (weak, acetylene). $^1$H NMR: 7.11 (t, 1H, $H_7$), 6.77 (d, 1H, H8), 6.68 (d, 1H, H6), 4.49 (m, 1H, $J_{21}$=1.6 Hz, $J_{23\alpha}$=obscured, $J_{23\beta}$=8.3 Hz, H2), 4.15 (d of d, 1H, $J_{1'1}$=1.9 Hz, H1′), 3.82 (s, 3H, $OCH_3$), 3.18 (d of d, 1H, $J_{4\beta 3a}$=5.3 Hz, $J_{4\beta 4\alpha}$=16.9 Hz, H4β), 2.95 (d of d, 1H, all coupling constants obscured, H9β), 2.93 (d of d, 1H, $J_{12}$=1.6 Hz, $J_{19a}$=6.4 Hz, $J_{11'}$=1.9 Hz, H1), 2.91 (d of d, 1H, all coupling constants obscured, H9α), 2.60 (d of t, 1H, $J_{3\beta 2}$=8.3 Hz, $J_{3\beta 3\alpha}$=14.1 Hz, $J_{3\beta 3a}$=10.4 Hz, H3β), 2.27 (d of d, 1H, $J_{4\alpha 3a}$=11.6 Hz, $J_{4\alpha 4\beta}$=16.9 Hz, H4α), 2.02 (m, 1H, $J_{9al}$=6.4 Hz, $J_{9a3a}$=12.3 Hz, $J_{9a9\alpha}$ and $J_{9a9\beta}$ obscured, H9a), 1.90 (m, 1H, $J_{3a3\alpha}$ obscured, $j_{3a3}$=10.4 Hz, $j_{3a4\alpha}$=11.6 Hz, $J_{3a4\beta}$=5.3 Hz, $J_{3\alpha 9a}$= 12.3 Hz, H3a), 1.35 (m, 1H, $j_{3\alpha 2}$ and $j_{3\alpha 3a}$ obscured, $J_{3\alpha 3\beta}$=14.1 Hz, H3α), 1.85-1.00 (m, 11H, cyclohexyl). $^{13}$C NMR: 157.25 (e), 148.10 (e), 126.01 (e), 125.27 (o), 121.75 (o), 106.83 (o), 84.71 (e), 84.42 (e), 79.22 (o), 67.09 (o), 55.15 (o), 44.13 (o), 43.81 (o), 42.03 (o), 41.23 (e), 38.52 (o), 32.38 (e), 29.88 (e), 28.62 (e), 28.00 (e), 26.17 (e), 25.78 (e).

The second fraction contained (1R, 1′S, 2R, 3aS,9aS)-2,3,3a,4,9,9a-Hexahydro-1-(1′-cyclohexyl-1′-hydroxyprop-2′-yn-3′-yl)-2-hydroxy-5-methoxy-1H-benz[f]indene 610 as a foam, 1.25 g, 3.53 mmol, 46% yield. The physical characteristics of compound 610, an intermediate used in the synthesis of isotetralynaprost include: IR: 2.92 (broad, OH), 4.48 (weak, acetylene). $^1$H NMR: 7.10 (t, 1H, H7), 6.79 (d, 1H, H8), 6.74 (d, 1H, H6), 4.13 (d of d, 1H, $J_{a'a}$=1.0 Hz, H1′), 3.92 (m, 1H, $J_{21}$=9.6 Hz, $J_{23\alpha}$=6.0 Hz, $J_{23\beta}$=<1 Hz, H2), 3.79 (s, 3H, $OCH_3$), 2.76 (d of d, 1H, $J_{9\beta 9\alpha}$ =14.4 Hz, $J_{9\beta 9a}$=5.7 Hz, H9β), 2.60 (m, 2H, coupling constants obscured, H4α and H4β), 2.54 (d of d, 1H, $J_{9\alpha 9\beta}$=14.4 Hz, $J_{9\alpha 9a}$=5.7 Hz, H9α), 2.32 (m, 2H, J9a1=9.6 Hz, J9a9α=5.7 Hz, J9a9β=5.7 Hz, J3α2=6.0 Hz, other coupling constants obscured, H9a and H3α), 2.14 (m, 1H, $J_{3\beta 3\alpha}$ obscured, $J_{3\beta 3a}$<1 Hz, H3β), 2.03 (t, 1H, $J_{12}$=9.6 Hz, $J_{19a}$=9.6 Hz, $J_{11'}$=1.0 Hz, H1), 1.90-1.10 (m, 11H, cyclohexyl), 1.00 (m, 1H, J3a3β<1 Hz, other coupling constants obscured). $^{13}$C NMR: 156.48 (e), 139.37 (e), 126.24 (e and o), 120.69 (o), 108.34 (o), 86.94 (e), 81.78 (e), 76.13 (o), 66.91 (o), 55.26 (o), 44.46 (o), 44.13 (o), 41.65 (o), 39.61 (e), 32.43 (e), 28.70 (e), 28.15 (e), 26.46 (e), 25.90 (e), 25.20 (e).

Larger scale runs were more conveniently purified via preparative HPLC on a Waters Prep 500 instrument, employing 2 silica columns, eluting with 25% EtOAc/hexanes, flow rate 0.2 L/min.

A solution of methyl ether 610 (1.01 g, 2.85 mmol), $Lipph_2$ (61 mL of a 0.47 M solution in THF, 28.7 mmol) and THF (20 mL) was heated at reflux 14 hr then quenched with saturated $NH_4Cl$ (100 mL). The organics were extracted with EtOAc (3x100 mL), washed with saturated NaCl (100 mL), dried ($Na_2SO_4$) and evaporated. Chromatography of the residue (200 g 60-200 mesh silica gel, 50% to 60% EtOAc/hexane gradient) gave crude 611. This material was dissolved in $CH_2Cl_2$ (50 mL) and acetic anhydride (1.6 M1, 17 mmol), pyridine (2.8 mL, 34 mmol) and DMAP (17.0 mg, 0.139 mmol) and sequentially. After 19 hr stirring, the mixture was diluted with $CH_2Cl_2$ (100 mL), washed with 5% HCl (2x50 mL), saturated NaCl (50 mL), dried ($Na_2SO_4$) and evaporated to afford the crude triacetate. Chromatography (40 g 60-200 mesh silica gel, 10% to 20% EtOAc/hexane gradient) afforded the pure triacetate. This material was hydrolyzed by stirring a solution in MeOH (10 mL) with $K_2CO_3$ (4.0 g, 28.9 mmol) for 4 hr, then cautiously acidifying with 5% HCl (75mL). The organics were extracted with EtOAc (3x30 mL), dried ($Na_2SO_4$) and evaporated to afford pure (1R, 1′S, 2R, 3aS, 9aS)-2,3,3a,4,9,9a-Hexahydro-1-(1′-cyclohexyl-1′-hydroxyprop-2′-yn-3′-yl)-2, 5-dihydroxy-1H-benz[f]indene 611 as a foam, 0.70

g, 2.06 mmol, 72% yield.

A mixture of compound 611, chloroacetonitrile (12.0 mL, 190 mmol) and $Cs_2CO_3$ (1.91 g, 5.86 mmol) was stirred for 24 hr, then filtered through cellite. The filter cake was washed with $CH_2Cl_2$ (3x20 mL) and the filtrates combined. The $CH_2Cl_2$ was removed on a rotary evaporator. The excess chloroacetonitrile was removed by Kugelrohr distillation (water aspirator pressure, 50°C water bath) to afford 2.05 of dark glass. Chromatography (80 g 60-200 mesh silica gel, 80% to 100% $CH_2Cl_2$/hexane gradient) gave pure (1R, 1′S, 2R, 3aS, 9aS)-2, 3, 3a, 4, 9, 9a-Hexahydro-5-cyanomethyoxy-1-(1′-cyclohexyl-1′-hydroxyprop-2′-yn-3′-yl)-2-hydroxy-1H-benz[f] indene 612 as a white foam, 85% yield.

A mixture of nitrile 612 (0.2985 g, 0.787 mmol), methanol (71 mL) and NaOH (21 mL of a 25% aqueous solution) was heated at reflux for 24 hr, then cooled to room temperature. The solution was acidified with 5% HCl (150 mL) and extracted with $CH_2Cl_2$ (3x100 mL). The combined organics were washed with saturated NaCl (100 mL) and the layers quickly separated, as a fluffy precipitate began to form. After standing overnight, filtration afforded pure compound 600 (0.2873 g., 0.721 mmol, 92% yield). The filtrate was dried ($Na_2SO_4$), evaporated and the residue subjected to chromatography (60 g 60-200 mesh silica gel, eluting sequentially with hexane, 50% EtOAc/hexane, EtOAc, 1% HOAc/EtOAc and finally 5% MeOH/1% HOAc/ EtOAc) to afford 0.69 g of an off-white foam. Recrystallization from EtOAc/hexane provided small prisms, mp 173-174°C. The physical characteristics of compound 600 include:

$[a]_D$=+121.9° (c 1.00, MeOH). Ir (KBr): 2.90 (very broad, $CO_2H$, 4.56 (weak, acetylene), 5.80 (c=O). $^1H$ NMR ($d_6$-acetone): 7.08 (t, 1H, H7), 6.80 (d, 1H, H8), 6.76 (d, 1H, H6), 4.69 (s, 2H, H2″), 4.09 (d of d, 1H, H1′), 3.93 (m, 1H, H2), 2.76 (d of d, 1H, H9β), 2.69 (d, 2H, H4α and H4β, 2.60 (d of d, 1H, H9α), 2.36 (m, 2H, H3β and H9a), (m, 1H, H3α), 2.00 (t, 1H, H1), 1.95-1.20 (m, 12H, H3β and cyclohexyl). $^{13}C$ NMR ($d_6$-acetone): 170.06 (e), 155.74 (e), 140.29 (e), 127.32 (e), 126.63 (o), 121.66 (o), 110.14 (o), 86.68 (e), 82.49 (e), 76.48 (o), 66.78 (o), 65.47 (e), 49.34 (e), 44.98 (o), 42.89 (o), 40.91 (e), 32.94 (o), 32.69 (e), 26.89 (e), 26.35 (e), 25.66 (e). Note: two carbon resonances were apparently obscured by the acetone heptet. Exact mass: calculated for $C_{24}H_{28}O_4$ (M-$H_2O$): 380.1988. Found 380.1985. Elemental analysis: calculated for $C_{24}H_{30}O_5$: C, 72.33; H, 7.59. Found: 71.86, H 8.09.

## EXAMPLE 16

Synthesis of [1S(1S),2R,3aS,9aS]-5-Carborn ethyoxy-7-(Z-1-cyclohexyl--hydroxyprop-2-en-3-yl)-2-hydroxy-2,3,3a,4,9,9a-hexahydro-1H-benz[f]indene 620 (Trivial Name: Z-Isotetralenaprost; n=1, p=1, q=1, covalent bond b is in the alpha configuration, K is hydroxy, Y is cis, -CH=CH-, $M_1$ is hydrogen, $M_2$ is hydrogen, L and J taken together with the carbon atom bridging those groups form a $C_6$ cycloalkyl group, $S_1$ is -AEQ, wherein A is -O-, E is $-(CH_2)_r$ wherein r is 1, Q is -COOH, and $S_2$ and $S_3$ are hydrogen).

The procedure used to prepare 620 was the same as the one previously described for the synthesis of Z-pseudoindenaprost 210 (See Example 8) except as follows: isotetralynaprost 600 (117 mg, 0.295 mmol) was used as the substrate and the reaction was only allowed to stir for 1.25 h. This procedure afforded 118 mg (100% yield) of Z-isotetralenaprost 620 as a white foam having the following physical characteristics: $R_f$ = 0.35 (98:2:0.5 EtOAc/methanol/AcOH); $^1H$-NMR ($CDCl_3$) 7.05 (t, 1H, J=7.5, $H_7$), 6.81 (d, 1H, J=7.3, $H_8$), 6.65 (d, 1H, J=7.9, $H_6$), 5.61 (dd, 1H, J=9.7, J=8.5, $H_{2'}$), 5.38 (dd, 1H, J=9.7, J=10.3, $H_{3'}$), 4.59 (s, 2H, $OCH_2CO_2$), 4.01 (bt, 1H, $H_{1'}$), 3.69 (m, 1H, $H_2$), 2.80-2.51 (m, 4H, $H_{9\beta}$, $H_{4\alpha}$, $H_{4\beta}$, and $H_{9\alpha}$), 2.39 (m, 2H, $H_{3\beta}$ and $H_{9a}$), 2.15 (m, 1H, $H_{3\alpha}$), 2.02 (m, 1H, $H_1$), 1.91-0.82 (m, 12H, $H_{3\alpha}$ and c-hexyl); $^{13}C$-NMR ($CDCl_3$) 154.9 (e), 140.5 (e), 134.1 (o), 133.7 (o), 127.4 (e), 126.2 (o), 121.8 (o), 109.8 (o), 76.0 (o), 73.2 (o), 65.5 (e), 51.6 (o), 43.9 (o), 41.5 (o), 40.3 (e), 32.5 (o), 31.8 (e), 28.8 (e), 28.5 (e), 26.4 (e), 26.0 (e), 25.9 (e), 25.4 (e) (Note: Carbonyl signal of acid not observed due to long relaxation time of this signal); exact mass (EI) calculated for ($M^+$-$H_2O$) $C_{24}H_{30}O_4$ 382.2144, found 382.2149.

## EXAMPLE 17

Synthesis of Epiisotetralynaprost 700 (Trivial Name: Epiisotetralynaprost; n=1, p=1, q=1, covalent bond b is in the beta configuration, K is hydroxy, Y is -C≡C-, $M_1$ is hydrogen, $M_2$ is hydroxy, L and J taken together with the carbon atom bridging those groups form a $C_6$ cycloalkyl group, $S_1$ is -AEQ, wherein A is -0-, E is $-(CH_2)_r$ wherein r is 1, Q is -COOH, and $S_2$ and $S_3$ are hydrogen).

Methyl ether 701 (1.01 g, 2.84 mmol, prepared as described in Example 15) is converted to (1R, 1′S, 2R, 3aS, 9aR)-2, 3, 3a, 4, 9, 9a-Hexahydro-1-(1′-cyclohexyl-1′-hydroxyprop-2′-yn-3′-yl)-2, 5-dihydroxy-H-benz[f]indene 702 employing the same procedure as used for the conversion of compound 610 to 611 in Example 15. Compound 702 was produced as an off-white solid, 0.62 g, 1.82 mmol, 64% yield.

Triol 702 was converted to (1, 1′S, 2R, 3aS, 9aR)-2, 3, 3a, 4, 9, 9a-Hexahydro-5-cyanomethoxy-1-(1′-cyclohexyl-1′-hydroxyprop-2′-yn-3′-yl)-2-hydroxy-1H-benz[f]indene 703 in 85% yield by the procedure employed for the synthesis of compound 612 in Example 15.

Compound 703 (0.24 g, 0.63 mmol) was then converted by hydrolysis to epiisotetralynaprost 700 using the procedure set forth in Example 15 for conversion of compound 612 to that of compound 600, giving an off-white foam (0.21 g, 0.53 mmol, 84% yield). Recrystallization from EtOAc/hexane afforded 700 as small prisms, mp. 169-170°C having the following physical characteristics: [α] $^D$=-18.6° (c 0.67, MeOH). IR (KBr): 2.85 (very broad, $CO_2H$), 4.44 (weak, acetylene), 5.67 (C=O). $^1H$ NMR ($d_6$-acetone): 7.05 (t, 1H, H7), 6.76 (d, 1H, H8), 6.66 (d, 1H, H6), 4.71 (s, 2H, H2″), 4.40 (m 1H, H2), 4.11 (d of d, 1H, H1′), 3.25 (d of d, 1H, H9β), 2.86 (m, 2H, H9a and H9α), 2.53 (d of 6, 1H, H3β), 2.27 (d of d, 1H, H4α), 2.10-1.60 (m, 5H, H3a and cyclohexyl), 1.47 (m, 1H, cyclohexyl methine), 1.10-1.00 (m, 4H, cyclohexyl). $^{13}C$ NMR ($d_6$-acetone): 170.09 (e), 156.67 (e), 139.11 (e), 126.58 (o), 126.22 (e), 122.99 (oO, 108.63 (o), 85.90 (e), 84.09 (e), 78.99 (o), 66.78 (o), 65.15 (e), 45.18 (o), 44.84 (o), 42.63 (o), 41.84 (e), 38.97 (o), 32.80 (e), 27.02 (e), 26.42 (e). Note: three carbon resonances were apparently obscured by the acetone heptet. Exact mass: calculated for $C_{24}H_{28}O_4$ (M-$h_2O$): 380.1988. Found: 380.1986. Elemental analysis: calculated for $C_{24}H_{30}O_5$: C, 72.33, H 7.59. Found: C 72.49, H 8.20.

## EXAMPLE 18

Synthesis of [1S(1S),2R,3aS,9aR]-5-Carbomethyoxy-7-(Z-1-cyclohexyl-1-hydroxyprop-2-en-3-yl)-2-hydroxy-2,3,3a,4,9,9a-hexahydro-1H-benz[f]indene 720 (Trivial Name: Z-epiisotetralenaprost; n=1, p=1, q=1, covalent bond b is in the beta configuration, K is hydroxy, Y is cis -CH=CH-, $M_1$ is hydrogen, $M_2$ is hydrogen, L and J taken together with the carbon atom bridging those groups form a $C_6$ cycloalkyl group, $S_1$ is -AEQ, wherein A is -O-, E is -$(CH_2)_r$ wherein r is 1, Q is -COOH, and $S_2$ and $S_3$ are hydrogen).

The procedure used to prepare 720 was the same as the one previously described for the synthesis of 210 (See Example 8) except as follows: epiisotetralynaprost 700 (61 mg, 0.153 mmol) was used as the substrate, the reaction was only allowed to stir for 1 h, and the crude solid obtained was recrystallized from ethyl acetate and hexanes using general drops of methanol to help dissolve the solid. This procedure afforded 47 mg (77% yield) of Z-epiisotetralenaprost 720 as a white powder having the following physical characteristics: $R_f$ = 0.3 (95:5:0.5 EtOAc/methanol/AcOH); mp 170-1°C; $^1H$-NMR ($CDCl_3$) 7.08 (t, 1H, J=7.5, $H_7$), 6.81 (d, 1H, J=7.4, $H_8$), 6.68 (d, 1H, J=7.8, $H_6$), 5.55 (dd, 1H, J=11.8, J=9.8, $H_{2'}$), 5.38 (dd, 1H, $H_{1'}$), 4.68 (s, 2H, $OCH_2CO_2$), 4.22 (dd, 1H, $H_{1'}$), 4.16 (m, 1H, $H_2$), 3.26 (dd, 1H, $H_{4\beta}$), 3.03 (m, 1H, $H_{9\beta}$), 2.74 (m, 2H, $H_{9a}$ and $H_{9\alpha}$), 2.56 (dt, 1H, $H_{3\beta}$), 2.38 (dd, 1H, $H_{4\alpha}$), 2.13 (m, 1H, $H_1$), 1.97 (dt, 1H, $H_{3\alpha}$), 1.85-0.95 (m, 11H, c-hexyl); exact mass (EI) calculated for ($M^+$-$H_2O$) $C_{24}H_{30}O_4$ 382.2144, found 382.2144.

## EXAMPLE 19

Synthesis of [1S(1R),2R,3aS,9aR]-5-Carbomethyoxy-7-(1-cyclohexyl-1-hydroxyprop-3-yl)-2-hydroxy-2,3,3a,4,9,9a-hexahydro-1H-benz[f]indene 730 (Trivial Name: Epiisotetralanaprost; n=1, p=1, q=1, covalent bond b is in the beta configuration, K is hydroxy, Y is -$CH_2CH_2$-, $M_1$ is hydrogen, $M_2$ is hydroxy, L and J taken together with the carbon atom bridging those groups form a $C_6$ cycloalkyl group, $S_1$ is -AEQ, wherein A is -O-, E is -$(CH_2)_r$ wherein r is 1, Q is -COOH, and $S_2$ and $S_3$ are hydrogen).

The procedure used to prepare 7300 was the same as the one previously described for the synthesis of 620 (See Example 16) except as follows:
epiisotetralynaprost 700 (166 mg, 0.417 mmol) was used as the substrate, the hydrogenation reaction was only allowed to stir for 4.5 h. The crude oil obtained from the hydrogenation reaction was esterified as described for the synthesis of 333 and purified by chromatography on ca. 25 g of silica gel (60-200 mesh) eluted with 50% then 65% ethyl acetate/hexanes providing 105 mg of 339 (61% yield for the two steps) and 38 mg of 340. A crude solid was formed which was recrystallized from ethyl acetate and hexanes containing a few drops of methanol to help dissolve the solid. This procedure afforded 102 mg (100% yield, 61% yield for the three steps) of epiisotetralanaprost 730 as a white powder: $R_f$=0.3 (95:5:0.5 EtOAc/methanol/AcOH); mp 197-8°C; $^1H$-NMR ($CDCl_3/CD_3CO_2D$ ca. 1:40) 7.03 (t, 1H, J=7.8, $H_7$), 6.75 (d, 1H, J=7.5, $H_8$), 6.60 (d, 1H, J=8.0, $H_6$), 4.71 (s, 2H, $OCH_2CO_2$), 4.18 (bt, 1H, $H_2$), 3.49 (bs, 1H, $H_{1'}$), 3.23 (dd, 1H, J=17.0, J=5.0, $H_{4\beta}$), 2.73 (m, 3H, $H_{9a}$, $H_{9\alpha}$ and $H_{9\beta}$), 2.48 (dt, 1H, $H_{3\beta}$), 2.30 (dd, 1H, J=17.0, J=11.7, $H_{4\alpha}$), 2.10-0.98 (m, 17H, $H_1$, $H_{3a}$, $H_{2'}$, $H_3$, and c-hexyl); exact mass (EI) calculated for ($M^+$) $C_{24}H_{34}O_5$ 402.2406, found 402.2410.

## EXAMPLE 20

Synthesis of[1R-]1α(3S), 2β, 3aα, 5E, 6aα]] Octahydro-1-(3-cyclohexyl-3-hydroxyprop-1-ynyl)-5-(5-hydroxy-3,3-dimethylpentylidene)pentalen-2-ol 821(Trivial name: Carbynatriol; n=1, p=0, q=1, covalent bond b is

in the alpha configuration, K is hydroxy, Y is -C=C-, $M_1$ is hydrogen, $M_2$ is hydroxy, L and J taken together with the carbon atom bridging those groups form a $C_6$ cycloalkyl group, G is $=CHCH_2C(CH_3)_2CH_2Q$, wherein Q is $-CH_2OH$)

A solution of 50.0 g (0.312 mol) of commercially available 3,3-dimethylglutaric acid 805 in 1L THF was cooled to 0°C and with mechanical stirring 700 mL (0.70 mol, 1M in THF) of diborane solution was added dropwise over a period of 3h. After the addition was complete, the cooling bath was removed and stirring was continued at ambient temperature for three days. The reaction was carefully quenched by slow addition of 350 mL saturated $K_2CO_3$ and the organic phase removed. The aqueous phase was extracted with 200 mL ether and the combined organic extracts were washed with 300 mL water and dried ($MgSO_4$). Solvent removal on a rotary evaporator afforded 41 g (100%) of the diol 3,3-Dimethyl-1,5-pentanediol 806 as a heavy colorless oil phys data omitted

To a solution of 44 g (0.333 mol) of the diol 806, 0.88 g (3.30 mol) of 18-crown-6, and 56.9 g (0.333 mol) of benzyl bromide in 530 mL THF was added a suspension of 12.6 g (0.316 mol) of KH in 175 mL THF via cannula over a period of 90 min. It was necessary to stir the KH suspension vigorously to insure efficient cannulation. After the addition was complete, the mixture was stirred at ambient temperature for 1h, quenched by slow addition of 150 mL saturated $NH_4Cl$, and the organic phase removed. The aqueous phase was extracted with 200 mL ether and the combined organic extracts were washed with 200 mL saturated NaCl and dried ($MgSO_4$). Solvent removal afforded a yellow oil which was chromatographed on 1 Kg of silica gel (100-200 mesh) using 20% ethyl acetate in hexane. Collection of the product fraction and solvent removal afforded 51.3 g (70%) of the benzyl ether 5-Benzyloxy-3,3-dimethyl pentanol 807 as a colorless oil.

A solution of 51.30 g (0.231 mol) of alcohol 807 in 660 mL methylene chloride was cooled to 0°C and with mechanical stirring 28 g powdered molecular sieves and 59.7 g (0.277 mol) pyridinium chlorochromate were added. After 2h the cooling bath was removed and stirring at ambient temperature continued for 2 hours. The reaction mixture was poured into a suspension of 20 g powdered molecular sieves in 1.2 L ether and the resulting mixture was stirred vigorously for 20 min before being filtered through Celite. The filtrate was concentrated to 300 mL, diluted with 1L ether, washed with saturated NaCl (2x400 mL), and dried $MgSo_4$). Solvent removal afforded a dark green liquid which was chromatographed on a 20″x3″ silica gel (100-200 mesh) column using 10% ethyl acetate in hexane. Collection of the product fraction and solvent removal on a rotary evaporator provided 33.5 g (66%) of the aldehyde 5-Benzyloxy-3,3-dimethylpentanal 808 as a colorless oil.

To a stirred suspension of 1.45 g (0.0603 mol) of sodium hydride in 37 mL THF was added one drop of water followed by dropwise addition of a solution of 19.64 g (0.0431 mol) of Wadsworth-Emmons reagent 809 in 37 mL THF. The mixture was stirred 1h at ambient temperature before being cooled to -10°C. A solution of 9.48 g (0.0431 mol) of aldehyde 808 in 27 mL THF was added to the anionic mixture dropwise with stirring over a period of 20 min. The mixture was allowed to warm slowly to ambient temperature, a drop of water was added, and stirring was continued for an additional 14h. The mixture was quenched by slow addition of 60 mL saturated $NH_4Cl$ and the organic phase removed. The aqueous phase was diluted with 200 mL water and extracted with methylene chloride (2x100 mL). The combined organic extracts were washed with 150 mL of saturated NaCl and dried ($MgSO_4$). Solvent removal afforded 21.10 g (99%) of an inseparable 5:1 mixture of the desired isomer (Z)-t-Butyl-7-Benzyloxy-5,5-dimethyl-2-(trimethylstannyl)methylhept-2-enoate 810Z to an undesired E isomer 810E (not shown).

A solution of 21.10 g (0.0426 mol) of crude t-butylester mixture 810Z and 810E in 165 mL methylene chloride was cooled to 0°C and 171 mL (0.171 mol, 1M in hexane) of diisobutylaluminum hydride solution was added slowly over a period of 45 min. The mixture was warmed to ambient temperature and stirred 17h. The reaction mixture was cooled to 0°C and 83 mL 10% NaOH was added slowly and carefully with vigorous stirring. The resulting emulsion was filtered through Celite and the filtrate concentrated to approximately 100 mL on a rotary evaporator. The resulting liquid was taken up in 225 mL methylene chloride and washed with 60 mL 10% NaOH. The aqueous phase was extracted with methylene chloride (2x40 mL) and the combined organic phases were washed with 125 mL saturated NaCl and dried ($MgSO_4$). Solvent removal afforded a light pale yellow oil which was chromatographed on a 20″x3″ silica gel (230-400 mesh) column using gradient elution (5 to 15% ethyl acetate in hexane). The undesired E isomer (E)-7-Benzyloxy-5,5-dimethyl-2-(trimethylstannyl)methyl-hept-2-en-1-ol 811E (2.61 g) eluted first ($R_f$ = 0.30, 15% ethyl acetate in hexane) followed by 12.0 g of the desired Z isomer (Z)-7-Benzyloxy-5,5-dimethyl-2-(trimethylstannyl)methyl-hept-2-en-1-ol 811Z ($R_f$ = 0.26, 15% ethyl acetate in hexane). Total combined yield of both isomers was 77% phys.

To a solution of 4.12 g (9.75 mmol) of alcohol 811Z in 65 mL methylene chloride at 0°C was added 6.79 mL (39.0 mmol) of diisopropylethylamine followed by 1.85 mL (24.4 mmol) of methoxymethyl chloride. The cooling bath was removed and stirring at ambient temperature continued for 2h before the mixture was diluted with 75 mL methylene chloride, washed with ice cold 5% HCl (2x75 mL), 75 mL saturated $NaHCO_3$, 75 mL saturated NaCl, and dried ($MgSO_4$). Solvent removal on a rotary evaporator afforded an orange oil which was

chromatographed on a 18″x3″ silica gel (100-200 mesh) column using gradient elution (2% to 10% ethyl acetate in hexane). Collection of product fraction and solvent removal provided 4.22 g (92.7%) of the methoxymethyl ether (Z)-7-Benzyloxy-1-methoxymethoxy-5,5-dimethyl-2-(trimethylstannyl)-methyl hept-2-ene 812Z as a colorless oil.

A mixture of 0.736 g (3.58 mmol) of copper bromide-dimethylsulfide complex, 1.00 g (11.5 mmol) of lithium bromide, and 7.8 mg (0.123 mmol) of copper powder in 8 mL THF was stirred several minutes at room temperature until all traces of yellow color (copper [II]) disappeared then the reaction mixture was cooled to -100°C. In a separate flask a solution of 1.61 g (3.46 mmol) of stannane 812Z in 14 mL THF was cooled to -100°C and treated with 1.64 mL (3.46 mmol, 2.11M in hexane)n-butyl lithium to produce lithiun reagent 813. After stirring 1 min the cold copper mixture was added quickly to the alkyl lithium solution via cannula. The resulting bromocuprate reagent 814 was stirred 1 min at -100°C before a solution of 1.50 g (2.47 mmol) of ammonium salt 16 in 7 mL methylene chloride was added dropwise over a period of 2 min. After the addition was complete, the reaction mixture was stirred 1 h at -100°C before being quenched by slow addition of 15 mL saturated $NH_4Cl$. The mixture was diluted with 150 mL saturated $NH_4Cl$ and extracted with ethyl acetate (3x100 mL). The combined organic extracts were washed with 150 mL saturated NaCl and dried ($MgSO_4$). Solvent removal afforded a brown oil which was chromatographed on a 14″x2″ silica gel (100-200 mesh) column using gradient elution (5 to 15% ethyl acetate in hexane). Collection of the product fraction and solvent removal afforded 1.50 g (79.2%) of the addition product (1R,4R)-1-[(E)-7-Benzyloxy-2- (methoxymethoxy)methyl-5 ,5-dimethylhept-2-enyl]-4-t-butyldiphenylsilyloxy-2-phenylsulfonylcyclopent-2-ene 815 as a thick colorless oil.

To a solution of 6.60 g (8.61 mmol) of methoxymethyl ether 815 in 420 mL isopropanol was added 0.33 g (1.72 mmol) of p-toluenesulfonic acid and the resulting mixture was heated at reflux for 25 h. The isopropanol was removed on a rotary evaporator and the oily residue was taken up in 350 mL ethyl acetate. The organic phase was washed with saturated NaCl (2x150 mL), dried ($MgSO_4$), and solvent removal afforded 6.21 g (100%) of alcohol 816 as a tan oil. The crude alcohol was used directly in the preparation of chloride 817, but further purification of the allylic alcohol could be accomplished by chromatography on a silica gel (100-200 mesh) column using 20% ethyl acetate in hexane as eluant. This provides the pure alcohol (1R,4R)-1-[(E)-7-Benzyloxy-2-hydroxymethyl-5,5-dimethylhept-2-enyl]-4-t-butyldiphenylsilyloxy-2-phenyl-sulfonylcyclopent-2 -ene 816 as a colorless oil.

To a solution of 2.94 g (11.2 mmol) of triphenylphosphine and 1.55 g (11.6 mmol) of N-chlorosuccinimide in 73 mL methylene chloride at 0°C was added a solution of 6.21 g (8.61 mmol) of alcohol 816 in 70 mL methylene chloride all at once with vigorous stirring. The resulting light purple mixture was stirred 30 min at 0°C and washed with 150 mL water. The aqueous wash was extracted with methylene chloride (2x75 mL) and the combined organic phases were washed with 150 mL saturated NaCl and dried ($MgSO_4$). Solvent removal afforded a purple oil which was chromatographed on a 25″x3″ silica gel (100-200 mesh) column using 10% ethyl acetate in hexane. Collection of the product fraction and solvent removal afforded 5.80 g (90.9%) of the chloride (1R,4R)-1-[(E)-7-Benzyloxy-2-chloromethyl-5, 5-dimethylhept-2-enyl]-4-t-butyldiphenylsilyloxy-2-phenylsulfonylcyclopent-2-ene 817 as a thick nearly colorless oil.

To a solution of 1.75 g (7.86 mmol) of chiral acetylene 19 in 37 mL THF at 0°C was added 3.58 mL (8.25 mmol, 2.30 M in hexane) n-butyl lithium. After stirring 10 min at 0°C, 0.068 mL (0.393 mmol) hexamethylphosphoramide (HMPA) was added followed immediately by dropwise addition of a solution of 2.91 g (3.93 mmol) of chloride 817 in 26 mL THF. After the addition was complete (10 min), the cooling bath was removed and stirring continued at ambient temperature for 1h. The reaction was quenched by the addition of 35 mL saturated $NH_4Cl$, diluted with 150 mL water, and extracted with ethyl acetate (3x125 mL). The combined organic extracts were washed with 150 mL saturated NaCl and dried ($MgSO_4$). Solvent removal afforded a golden brown oil which was chromatographed on a 20″x4″ silica gel (100-200 mesh) column using 10% ethyl acetate in hexane. Collection of the product fraction and solvent removal afforded 3.31 g (90.9%) of the desired bicyclic adduct [1-S-[1α(3S), 2β, 3aα, 5E, 6aα]] Octahydro-5-(5-benzyloxy-,3-dimethyl-pentylidene)-2-t- butyldiphenylsilyloxy-(3-cyclohexyl-3-tetrahydropyranyloxypropynyl)-6a-phenylsulfonylpentalene 819 as a tan oily foam.

To a solution of 2.61 g (2.82 mmol) of adduct 819 in 52 mL methanol and 12 mL chloroform was added 0.134 g (0.70 mmol) of p-toluenesulfonic acid monohydrate. The mixture was refluxed for 96h, diluted with 300 mL saturated $NaHCO_3$, and extracted with ethyl acetate (3x100 mL). The combined organic extracts were washed with 150 mL saturated NaCl and dried ($MgSO_4$). Solvent removal afforded a brown oil which was chromatographed on a 16″x2″ silica gel (100-200 mesh) column using gradient elution (20 to 40% ethyl acetate in hexane). Collection of the product fraction and solvent removal afforded 1.63 g (95%) of the diol [1S-[1α(3S), 2β, 3aα, 5E, 6aα]] Octahydro-5-(5-benzyloxy-,3-dimethyl-pentylidene)-1-(3-cyclohexyl-3-hydroxyprop-1-ynyl)-6a-phenylsulfonyl-pentalen-2-ol 820 as a tan foam.

To 266 mL liquid ammonia at -78°C was added 80 mL THF with vigorous stirring and after 15 min, 0.53 g (76.9 mmol) of lithium wire was added. After the blue mixture was stirred 15 min, a solution of 1.55 g (2.56 mmol)

of diol 820 and 0.96 mL (10.2 mmol) of t-butylalcohol in 50 mL THF was added slowly over 10 min. The reaction mixture was stirred at -78°C for 15 min before 4.5 mL (45 mmol) isoprene and excess solid $NH_4Cl$ were added. The cooling bath was removed and the ammonia was allowed to evaporate with stirring under a steam of air. The residue was diluted with 400 mL water and extracted with ethyl acetate (4x150 mL). The combined organic extracts were washed with 250 mL saturated NaCl and dried ($MgSO_4$). Solvent removal afforded a tan oil which was chromatographed on a 16″x1″ silica gel (100-200 mesh) column using 1:1 ethyl acetate in hexane. Collection of the product fraction and solvent removal afforded 0.815 g (85%) of the triol [1R-1[1a(3S), 2β, 3aα, 5E, 6aα]] Octahydro-1-(3-cyclohexyl-3-hydroxyprop-1-yryl)-5-(5-hydroxy-3,3 dimethylpentylidene) pentalen-2-01 821 as a white foam. Compound 821 was determined to have the following physical characteristics: $[\alpha]D^{20}$ +97.7(c < 1.00, $CHCl_3$); TLC $R_f$ = 0.28 (4:1, ethyl acetate:hexane); $^1H$ NMR ($CDCl_3$) δ 5.31 (bt, 1H, $J_{1',2'}$ = 7.1 Hz, $J_{allylic}$<2 Hz, pentylidene CH), 4.08 (dd, 1H, $J_{3',cyclohexyl}$ = 6.1 Hz, $J_{1,3'}$<2 Hz, H3′), 3.87 (ddd, 1H, $J_{2,4\beta}$ = 7.1 Hz, $J_{2,4\alpha}$ = 9.4 Hz, $J_{1,2}$ = 8.5 Hz, H2), 3.57 (dt, 2H, $J_{4',5'}$ = 7.1 Hz, $J_{H,OH}$ = 3.3 Hz, $CH_2OH$), 2.50-1.65 (m, 16H, H1, H3, H3a, H4, H6, H6a, $CH_2CMe_2$, cyclohexyl), 1.48 (t, 2H, $J_{4',5'}$ = 7.1 Hz, $CH_2CH_2OH$), 1.30-0.95 (m, 6H, cyclohexyl), 0.87 (s, 3H, $CH_3$), 0.85 (s, 3H, $CH_3$); $^{13}C$ NMR ($CDCl_3$) δ 142.26 (e, C5), 119.21 (o, $CHCH_2$), 87.31 (e, C2′), 81.55 (e, C1′), 77.67 (o, C2), 67.17 (o, C3′), 59.49 (e, $CH_2OH$), 45.98 (o, Cl), 44.90 (o, C6a), 44.64 (o, C3a), 44.61 (e, C6), 44.25 (o, cyclohexyl CH), 43.47 (e, C3), 42.42 (e, C4), 40.99 (e, $CH_2CMe_2$), 39.08 (e, $CH_2CH_2OH$), 35.48 (e, $CMe_2$), 32.99 (e, cyclohexyl), 28.73 (e, cyclohexyl), 28.23 (e, cyclohexyl), 28.04 (o, $CH_3$), 27.07 (o, $CH_3$), 26.48 (e, cyclohexyl), 25.96 (e, cyclohexyl); IR (neat) $cm^{-1}$ (μm) 3340 (3.00), 2935 (3.42), 2860 (3.50), 2240 (4.47), 1450 (6.90), 1365 (7.33), 1080 (9.28), 1015 (9.85), 730 (13.7); mass spectrum, Cl, m/z (rel. intensity) 375 (M+H, 3), 357 (M+H-$H_2O$, 77), 339 (M+H-2$H_2O$, 100), 218 (7), 161 (5). Exact mass (Cl); calcd for $C_{24}H_{38}O_3$ (M+H); 375.2899, found 375.2892.

EXAMPLE 21

Synthesis of [3aS-[2E, 3aα, 4α(3S), 5β, 6aα]] 3,3-Dimethyl-5-[hexahydro-4-(3-cyclohexyl-3-hydroxyprop-1-ynyl)-5-hydroxy-2(1H)-pentalenylidene]pentanoic acid 822 (Trivial name: Carbynaprost; n=1, p=0, q=1, covalent bond b is in the alpha configuration, K is hydroxy, Y is -C≡C-, $M_1$ is hydrogen, $M_2$ is hydroxy, L and J taken together with the carbon atom bridging those groups form a $C_6$ cycloalkyl group, G is =$CHCH_2C(CH_3)_2CH_2Q$, wherein Q is -COOH)

A solution of 0.49 g (2.14 mmol) of platinum [IV] oxide (Engelhard lot no. 2510) in 90 mL water was hydrogenated at 45 psi on a Parr hydrogenator for 4h. The suspension of finely divided platinum metal was alternately evacuated (2 mm Hg) and flushed with argon (6x) to remove any remaining hydrogen. An additional 66 mL water was added along with 3.51 g (41.7 mmol) solid $NaHCO_3$, 1.37 g sand, and 70 mg sodium lauryl sulfate. Oxygen was bubbled through the resulting mixture for 5 min as it was heated to 60°C using an oil bath. A solution of 0.40 g (1.07 mmol) of triol 821 in 80 mL diglyme was added all at once and vigorous stirring, heating, and oxygen bubbling continued for 20h. The mixture was cooled to ambient temperature and filtered through a pad of Celite. The filtrate was acidified by dropwise addition of 30% HCl and the resulting solution extracted with ethyl acetate (3x150 mL). The combined organic extracts were washed with 150 mL saturated NaCl and dried ($MgSO_4$). The resulting solution was concentrated to approximately 50 mL on a rotary evaporator, 0.30 g solid $Na_1CO_3$ was added, and the remaining diglyme removed by distillation on high vacuum (0.30 mm) using minimal heating. The pot residue was taken up in 100 mL water and made just acidic by dropwise addition of 30% HCl. The acidic solution was extracted with ethyl acetate (3x75 mL), the combined organic extracts were washed with 100 mL saturated NaCl, and the organic phase was dried ($MgSO_4$). Solvent removal provided a tan oil which was chromatographed on a 12″x3/4″ silica gel (230-400 mesh) column using hexane:ethyl acetate:acetic acid (1:0.8:0.01). Collection of the product fraction and solvent removal afforded 0.32 g (76.9%) of the acid 822 as a nearly colorless oil having the following physical characteristics: $[\alpha]D^{20}$ + 90.1 (c = 1.01, $CH_3OH$); TLC $R_f$ = 0.35 (4:1:0.1, ethyl acetate:hexane:acetic acid); $^1H$ NMR ($CDCl_3$) δ 5.32 (bt, 1H, $J_{4,5}$ = 7.0 Hz, $J_{allylic}$ <2 Hz, H5), 4.09 (dd, 1H, $J_{3'', cyclohexyl}$ = 6.0 Hz, $J_{3'',4}$<2 Hz, H3″), 3.88 (ddd, 1H $J_{5',6\beta'}$ = 7.0 Hz, $J_{5',6\alpha'}$ = 9.5 Hz $J_{4',5'}$ = 8.8 Hz, H5′), 2-41 (m, 1H, $J_{1\beta',6a'}$ = 8.5 Hz, $J_{1\alpha,6a'}$= 2 Hz, $J_{6\beta',6a'}$ = 8.3 Hz, $J_{6\alpha',6a'}$ = 7.3 Hz, H6a′), 2.37-2.00 (m, 10H, H2, H4, H1′, H3a′, H3β′, H6β′, cyclohexyl CH), 1.90 (m, 1H, H4′), 1.78 (m, 2H, cyclohexyl), 1.70 (m, 2H, cyclohexyl), 1.60 (m, 1H, H3α′), 1.48 (m, 1H, H6α′), 1.30-0.95 (m, 6H, cyclohexyl), 0.98 (s, 3H, $CH_3$), 0.95 (s, 3H, $CH_3$);

$^{13}C$ NMR ($CDCl_3$) δ 177.72 (e, C1), 143.42 (e, C2′), 118.45 (o, C5), 87.27 (e, C2″), 81.54 (e, C1″), 77.92 (o, C5′), 67.28 (o, C3″), 46.31 (o, C4′), 45.29 (e, C2), 44.79 (o, C3a′), 44.17 (o, C6a′), 41.68 (e, C3′), 40.68 (e, C6′), 39.91 (e, C1′), 38.87 (e, C4), 37.53 (o, cyclohexyl CH), 35.63 (e, $CMe_2$), 34.03 (e, cyclohexyl), 28.69 (e, cyclohexyl), 28.20 (e, cyclohexyl), 27.32 (o, 2$CH_3$), 26.45 (e, cyclohexyl), 25.94 (e, cyclohexyl); IR (neat) $cm^{-1}$ (μm) 3350 (2.97), 3100 (3.21), 2220 (4.46), 1710 (5.83), 1450 (6.90), 1375 (7.28), 1240 (8.07); mass spectrum, Cl, m/z (rel. intensity) 389 (M+H, 1), 371 (M+H-$H_2O$, 48), 353 (M+H-2$H_2O$, 100); exact mass (EI): calcd

for $C_{24}H_{36}O_4$(M+): 388.2613, found: 388.2613.

EXAMPLE 22

Synthesis of [3aS-[2E, 3aα, 4α(1Z, 3R), 5β, 6aα]] 3,3-Dimethyl-5-[hexahydro-4-(3-cyclohexyl-3-hydroxyprop-1-enyl)-5-hydroxy-2(1H)-pentalenylidenelpentanoic acid 823 (Trivial name: Z-Carbenaprost; n=1, p=0, q=1, covalent bond b is in the alpha configuration, K is hydroxy, Y is cis -C=C-, $M_1$ is hydrogen, $M_2$ is hydroxy, L and J taken together with the carbon atom bridging those groups form a $C_6$ cycloalkyl group, G is $=CHCH_2C(CH_3)_2CH_2Q$, wherein Q is -COOH)

A mixture of 0.100 g (0.257 mmol) of acid 822, 8.2 mg of 5% palladium on alumina (0.0039 mmol Pd), 6.5 mL ethyl acetate, and 4.9 mL hexane was cooled to 0°C and hydrogenated at atmospheric pressure on a Brown hydrogenation apparatus for 1h. The mixture was concentrated to 1-2 mL on a rotary evaporator and the concentrate applied to a 8″x1/2″ silica gel (230-400 mesh) column and eluted with hexane: ethyl acetate: acetic acid (1:0.50:0.01). Collection of the product fraction and solvent removal provided 69 mg (69%) of Z-acid 823 as a white solid which was recrystallized using chloroform/hexane to provide fine white needles. The compound 823 has the following set of physical characteristics: $M_p$ 125-128°C; [α]D[20] +41.4 (c=0.33, $CHCl_3$); TLC $R_f$ = 0.33 (7:3, ethyl acetate:hexane); $^1$HNMR ($CDCl_3$) δ 5.89 (bs, 2H, OH), 5.60 (dd, 1H, $J_{1'',2''}$ = 11.2 Hz, $J_{1'',4'}$ = 8.1 Hz, H1″), 5.38 (dd, 1H, $J_{1'',2''}$ = 11.2 Hz, $J_{2'',3''}$ = 8.0 Hz, H2″), 5.37 (bt, 1H, $J_{4,5}$ = 6.9 Hz, $J_{allylic}$ < 2 Hz, H5), 4.11 (dd, 1H, $J_{2'',3''}$ = 8.0 Hz, $J_{3'',cyclohexyl}$ = 7.7 Hz, H3″), 3.73 (ddd, 1H, $J_{5',6\alpha'}$ = 8.6 Hz, $J_{5'6\beta'}$ = 6.7 Hz, $J_{4',5'}$ = 8.2 Hz, H5′), 2.50-1.60 (m, 18H, H2, H4, H1′, H3′, H3a′, H4′, H6′, H6a′, cyclohexyl), 1.45-0.90 (m, 6H, cyclohexyl), 1.05 (bs, 6H, $CH_3$); $^{13}$C NMR ($CDCl_3$ δ 177.25 (e, C1), 143.94 (e, C2′), 134.27 (o, C2″), 133.47 (o, C1″), 188.05 (o, C5), 77.78 (o, C5′), 73.35 (o, C3″), 51.67 (o, C4′), 46.52 (o, C3a′), 45.59 (e, C2), 44.01 (o, C6a′), 41.84 (e, C3′), 41.79 (e, C6′), 38.53 (e, C1′), 37.73 (o, cyclohexyl CH), 36.22 (e, C4), 34.08 (e, C3), 28.87 (e, cyclohexyl), 28.66 (e, cyclohexyl), 27.99 (o, $CH_3$), 27.16 (o, $CH_3$), 26.44 (e, cyclohexyl), 26.16 (e, cyclohexyl), 26.04 (e, cyclohexyl); IR (neat) $cm^{-1}$ (μm); 3400 (2.94), 2920 (3.42), 1700 (5.88), 1440 (6.95), 1230 (8.12), 1080 (9.26), 750 (13.3); mass spectrum, Cl, m/z (rel. intensity) 373 (M+H-$H_2O$, 12), 355 (M+H-2$H_2O$, 100), 337 (5), 207 (1); exact mass (Cl): calcd for $C_{24}H_{35}O_2$ (M+H-2$H_2O$); 355.2637, found 355.2637.

EXAMPLE 23

Synthesis of [3aS-[2E, 3aα, 4α(3R), 5β, 6aα]] 3,3-Dimethyl-5-[hexahydro-4-(3-cyclohexyl-3-hydroxypropyl)-5-hydroxy-2(1H)-pentalenylidene]pentanoic Acid 824 (Trivial Name: Carbanaprost; n=1, p=0, q=1, covalent bond b is in the alpha configuration, K is hydroxy, Y is $-Ch_2=C_2-$, $M_1$ is hydrogen, $M_2$ is hydroxy, L and J taken together with the carbon atom bridging those groups form a $C_6$ cycloalkyl group, G is $=CHCH_2C(CH_3)_2CH_2Q$, wherein Q is -COOH).

A mixture of 0.122 g (0.314 mmol) of acid 822 13.4 mg of 5% palladium on alumina (0.0063 mmol Pd), 8.5 mL ethyl acetate, and 6 mL hexane were cooled to 0°C and hydrogenated at atmospheric pressure on a Brown hydrogenation apparatus for 6h. The reaction mixture was concentrated to 1-2 mL on a rotary evaporator and the concentrate applied to a 10″x1/2″ $AgNO_3$ pretreated silica gel (230-400 mesh, 19 g silica gel slurried with 120 mL of 11% $AgNO_3$ in $CH_3CN$ and dried) column and eluted with hexane: ethyl acetate: acetic acid (1:0.50:0.01). The initial product fraction contained 7 mg of Z-acid 823 ($R_f$ = 0.30, $AgNO_3$ impregnated plate, 4:1:0.10, ether: hexane: acetic acid). The second compound to elute was the desired tetrahydroacid 824, and solvent removal from the collected product fraction afforded 77 mg (63%) of acid 824 as a white foam having the following set of physical characteristics: [α]D[20]+27.9 (c=0.30, $CHCl_3$); TLC $R_f$ = 0.25 ($AgNO_3$ impregnated plate, 4:1:0.10, ether:hexane: acetic acid); $^1$H NMR ($CDCl_3$) δ 6.00 (bs, 3H, OH), 5.31 (bt, 1H, $J_{4,5}$ = 7.0 Hz, $J_{allylic}$< 2 Hz H5), 3.68 (m, 1H, coupling obscured, H5′), 3.35 (m, 1H, couplings obscured, H3″), 2.60-1.90 (m, 10H, H2, H4, H1′, H3′, H3a′, H6β′), 1.90-0.95 (m, 18H, H1″, H2″, H4′, H6a′, H6α′, cyclohexyl), 1.03 (s, 6H, $CH_3$); $^{13}$C NMR ($CDCl_3$) δ 177.37 (e, Cl), 144.85 (e, C2′), 117.40 (o, C5), 78.72 (o, C5′), 76.94 (o, C3″), 53.27 (o, C4′), 46.32 (o, C3a′), 45.79 (o, C6a′), 45.37 (e, C2), 41.79 (e, C3′), 41.69 (e, C6′), 40.44 (e, C1′), 39.54 (e, C4), 38.04 (o, cyclohexyl CH), 34.09 (e, C3), 33.07 (e, C2″), 29.50 (e, C1″), 27.90 (e, cyclohexyl), 27.74 (e, cyclohexyl), 27.25 (o, 2$CH_3$), 26.35 (e, cyclohexyl), 26.20 (e, cyclohexyl); IR (neat) $cm^{-1}$ (μm) 3370 (2.96), 2940 (3.40), 2860 (3.50), 1710 (5.85), 1450 (6.90), 1375 (7.27), 1240 (8.07), 1045 (9.55), 760 (13.2); mass spectrum, C1, m/z (rel. intensity) 393 (M+H, 5), 375 (M+H-$H_2O$, 21), 357 (M+H-2$H_2O$, 100), 339 (5); exact mass (C1): calcd for $C_{24}H_{39}O_3$ (M+H-$H_20$); 375.2899, found 375.2899.

EXAMPLE 24

Synthesis of [3aS-[aα, 4(3R), 5β, 6aα]] 3,3-Dimethyl-5-[octahydro-4-(3-cyclohexyl-3-hydroxypropyl)-5-hydroxy-2-pentalenyl]pentanoic Acid 825 (Trivial name: Dihydrocarbanaprost; n=1, p=0, q=1, covalent bond b is in the alpha configuration, K is hydroxy, Y is $-Ch_2=C_2-$, $M_1$ is hydrogen, $M_2$ is hydroxy, L and J taken together with the carbon atom bridging those groups form a $C_6$ cycloalkyl group, G is $-CH_2CH_2C(CH_3)_2CH_2Q$, wherein Q is -COOH).

A mixture of 80 mg (0.206 mmol) of acid 822, 30.7 mg of 5% palladium on alumina (0.015 mmol Pd), and 7.5 mL ethanol was hydrogenated at atmospheric pressure and ambient temperature on a Brown hydrogenation apparatus for 2h. The mixture was concentrated to 1-2 mL and the concentrate applied to a 8″x3/4″ silica gel (230-400 mesh) column and eluted using hexane: ethyl acetate: acetic acid (1:0.80:0.01). Collection of the product fraction and solvent removal afforded 59.3 mg (73%) of the reduced acid 825 as a white solid which was recrystallized with chloroform/hexane to afford white needles having the following physical characteristics: Mp 125-131°C; $[\alpha]D^{20}$+13.9 (c=0.39, $CHCl_3$); TLC $R_f$ = 0.32 (4:1:0.10, ethyl acetate:hexane:acetic acid); $^1H$ NMR ($CDCl_3$) δ 3.88 (bs, 2H, OH), 3.78 (ddd, 1H, $J_{5',6\alpha'}$ = 8.3 Hz, $J_{5',6\beta}$ = 7.0 Hz, $J_{4',5'}$ = 8.5 Hz, H5′), 3.39 (m, 1H, H3″), 2.22 (s, 2H, H2), 2.20-0.90 (m, 29H, H4, H5, H1′, H2′, H3′, H3a′, H4′, H6′, H6a′, H1″, H2″, cyclohexyl), 1.01 (s, 6H, $CH_3$); $^{13}C$ NMR ($CDCl_3$) δ 174.78 (e, C1), 80.49 (o, C5′), 76.38 (o, C3″), 54.92 (o, C4′), 47.68 (o, C2′), 45.98 (e, C2), 44.88 (o, C3a′), 43.29 (o, C6a′), 42.03 (e, C3′), 41.84 (e, C6′), 41.59 (e, C1′), 41.29 (e, C2″), 39.02 (o, cyclohexyl CH), 32.83 (e, C1″), 32.38 (e, C3), 29.59 (e, C5), 29.57 (e, C4), 29.37 (e, cyclohexyl), 27.70 (e, cyclohexyl), 27.25 (o, $2CH_3$), 26.57 (e, cyclohexyl), 26.39 (e, cyclohexyl), 26.23 (e, cyclohexyl); IR ($CHCl_3$) $cm^{-1}$ (μm) 3370 (2.96), 2960 (3.42), 2840 (3.52), 1690 (5.91), 1240 (8.07), 1215 (8.24), 1120 (8.92), 1090 (9.17), 1040 (9.60); mass spectrum, CI, m/z (rel. intensity) 395 (M+H, 6), 377 (M+H-$H_2O$, 12), 359 (M+H-$2H_2O$, 100), 341 (6), 263 (4); exact mass (EI): calcd for $C_{24}H_{39}O_2$ (M-2HO): 358.2871, found 358.2868.

EXAMPLE 25

Synthesis of [1S-[1α(1E,3R), 2β, 3aα, 5E, 6aα]] Octahydro-1-(3-cyclohexyl-3-hydroxypropenyl)-5-(5-hydroxy-3,3-dimethylpentylidene)pentalen-2-ol 828 (Trivial name: E-Carbenatriol; n=1, p=0, q=1, covalent bond b is in the alpha configuration, K is hydroxy, Y is trans $-CH_2=C_2-$, $M_1$ is hydrogen, $M_2$ is hydroxy, L and J taken together with the carbon atom bridging those groups form a $C_6$ cycloalkyl group, G is $=CHCH_2C(CH_3)_2CH_2Q$, wherein Q is $-CH_2OH$).

A solution of 0.775 g (2.04 mmol) of E-((1S)-1-t-butyldimethylsilyloxy-3-iodo-prop-2-enyl) cyclohexane in 10 mL THF was cooled to -95°C and treated with 3.51 mL (4.08 mmol, 1.16 M in pentane) of t-butyllithium. Stirring was continued 10 min at low temperature before a solution of 0.377 g (0.51 mmol) of chloride 817 in 5 mL THF was added dropwise via a syringe. After the addition was complete, the cooling bath was warmed to -50°C over a period of 5 min and held at this temperature for 15 min. The reaction was quenched by addition of 15 mL saturated $NH_4Cl$, diluted with 70 mL water, and extracted with ethyl acetate (3x35 mL). The combined organic extracts were dried ($MgSO_4$) and the solvent removed on a rotary evaporator to afford an orange oil which was chromatographed on a 10″x1″ silica gel (230-400 mesh) column using 10% ethyl acetate in hexane. Collection of the product fraction and solvent removal afforded 0.423 g (87%) of [1S-[1α(1E, 3R), 2β, 3aα, 5E 6aα]] Octahydro-5-(5-benzyloxy-, 3-dimethylpentylidene)-1-(3-t-butyldimethylsilyloxy-3-cyclohexylprop-1-enyl)-2-t-butyldiphenylsilyloxy-6a-phenyl sulfonylpentalene 826 as a white foam.

A solution of 0.39 g (0.406 mmol) of disilylether 826 in 4.9 mL THF containing 700 mg powdered molecular sieves was treated with 4.06 mL (4.06 mmol, 1M in THF) tetrabutylammonium fluoride solution and stirring at ambient temperature continued for 18h. The reaction mixture was treated with an additional 2.00 mL (2.00 mmol) of tetrabutylammonium fluoride solution and stirring continued 6h longer. The brown mixture was filtered, diluted with 40 mL water, and extracted with methylene chloride (3x25 mL). The combined organic extracts were dried ($MgSO_4$) and solvent removal afforded a brown oil which was chromatographed on a 10″x1/2″ silica gel (100-200 mesh) column using ethyl acetate:hexane (1:1). Collection of the product fraction and solvent removal provided 0.204 g (83%) of diol [1S-[1α(1E, 3R), 2β, 3aα, 5E, 6aα]]Octahydro-5-(5-benzyloxy-,3-dimethylpentylidene)-1-(3-cyclohexyl-3-hydroxyprop-1-enyl)-6a-phenyl-sulfonyl pentalen-2-ol 827 as a white foam.

To 84 mL liquid ammonia at -78°C was added 26 mL THF with vigorous stirring and after 15 min 0.165 g (23.7 mmol) of lithium wire was added. After the resulting blue solution was stirred for 15 min, a solution of 0.48 g (0.791 mmol) of diol 827 and 0.30 mL (3.16 mmol) of t-butylalcohol in 15 mL THF was added slowly over a period of 5 min. The reaction was stirred at -78°C for 15 min before 1.39 mL (13.9 mmol) isoprene and excess solid $NH_4Cl$ were added. The cooling bath was removed and the ammonia was allowed to evaporate with stirring under a stream of air. The residue was diluted with 175 mL water and extracted with ethyl acetate (3x75 mL). The combined organic extracts were washed with 75 mL saturated NaCl and dried ($MgSO_4$). Solvent removal afforded a tan oil which was chromatographed on a 10″x1″ silica gel (100-200 mesh) column using ethyl acetate:hexane (3:2). Collection of the product fraction and solvent removal afforded 0.249 g (83.5%) of triol 828 as a white foam having the following physical characteristics: $[\alpha]D^{20}$=78.7 (c=0.91 $CHCl_3$); TLC $R_f$ =

0.12 (4:1, ethyl acetate:hexane); $^1H$ NMR ($CDCl_3$) δ 5.43 (m, 2H, $J_{trans}$ = 16.8 Hz, $J_{1,1'}$ = 8.1 Hz, $J_{2',3'}$= 9.2 Hz, H1′, H2′), 5.34 (bt, 1H, $J_{1',2'}$ = 7.0 Hz, $J_{allylic}$ < 2Hz, pentylidene CH), 3.80-3.55 (bs, 3H, OH), 3.70 (dd, 1H, $j_{2'3'}$ = 9.2 Hz, $j_{3'cyclohexyl}$ = 7.9 Hz, H3′), 3.62 (m, 3H, $j_{4'5'}$= 7.1 Hz, $j_{2,3\beta}$ = 6.6 Hz, $j_{2,3\alpha}$ = 9.0 Hz, $j_{1,2}$ = 8.9 Hz, $CH_2OH$, H2), 2.45-1.60 (m, 16H, H1, H3, H3a, H4, H6, H6a, $CH_2CMe_2$, cyclohexyl), 1.50 (t, 2H, $J_{4'5'}$ = 7.1 Hz, $CH_2CH_2OH$), 1.40-1.00 (m, 6H, cyclohexyl), 0.95 (s, 3H, $CH_3$), 0.92 (s, 3H $CH_3$); $^{13}$CNMR ($CDCl_3$) δ 142.89 (e, C5), 134.76 (o, C2′), 133.83 (o, C1′), 118.68 (o, $CHCH_2$), 77.91 (o, C2), 76.85 (o, C3′), 59.29 (e, $CH_2OH$), 57.11 (o, C1), 44.79 (o, C6a), 43.40 (e, C6), 43.07 (o, C3a), 42.25 (e, C3), 41.46 (e, C4), 38.50 ($CH_2CMe_2$), 37.16 (o, cyclohexyl CH), 35.90 (e, $CH_2CH_2OH$), 32.97 (e, $CMe_2$), 29.14 (e, cyclohexyl), 28.79 (e, cyclohexyl), 28.28 (o, $CH_3$), 27.21 (o, $CH_3$), 26.61 (e, cyclohexyl), 26.14 (e, cyclohexyl), 26.06 (e, cyclohexyl); IR (neat) $cm^{-1}$ (μm) 3340 (2.99), 2940 (3.40), 1450 (6.90), 1365 (7.32), 1085 (9.21), 1020 (9.80), 975 (10.3), 910 (11.0), 730 (13.7); mass spectrum, CI, m/z (rel. intensity) 375 (M+H-$H_2$, 5), 359 (M+H-$H_2O$, 79), 341 (M+H-2$H_2O$, 100), 323 (7); exact mass (CI): calcd for $C_{24}H_{37}O$ (M+H-2$H_2O$); 341.2844; found 341.2835.

EXAMPLE 26

Synthesis of[3aS-[2E, 3aα, 4α(1E, 3R), 5β, 6aα]]

3,3-Dimethyl-5-[hexahydro-4-(3-cyclohexyl-3-hydroxyprop-1-enyl)-5-hydroxy-2(1H)-pentalenylidene] pentanoic Acid 829 (Trivial Name: E-Carbenaprost; n=1, p=0, q=1, covalent bond b is in the alpha configuration, K is hydroxy, Y is trans -$CH_2$=$C_2$-, $M_1$ is hydrogen, $M_2$ is hydroxy, L and J taken together with the carbon atom bridging those groups form a $C_6$ cycloalkyl group, G is =$CHCH_2C(CH_3)_2CH_2Q$, wherein Q is -COOH).

A solution of 1.01 g (0.446 mmol) of platinum [IV] oxide (Engelhard lot no. 2510) in 33 mL water was hydrogenated at 45 psi on a Parr hydrogenator for 4h. The suspension of finely divided platinum metal was alternately evacuated (2 mm Hg) and flushed with argon (6x) to remove any remaining hydrogen. The aqueous suspension was treated with 0.73 g (8.70 mmol) of solid $NaHCO_3$ and and oxygen was bubbled through the mixture for 5 min as it was heated to 60°C using an oil bath. A solution of 84 mg (0.223 mmol) of triol 828 in 20 mL diglyme was added all at once and vigorous stirring, heating, and oxygen bubbling continued for 2.5h. Another 100 mg (0.440 mmol) of platinum [IV] oxide in a solution of 3 mL diglyme and 3 mL water was hydrogenated at 45 psi on a Parr hydrogenator during this time and the resulting mixture of platinum metal was added to the reaction mixture after it had been evacuated and flushed with argon (6x). Stirring, heating at 60°C, and oxygen bubbling continued an additional 12h before the reaction mixture was cooled and filtered through a pad of Celite. The filtrate was acidified by dropwise addition of 30% HCl and the resulting solution extracted with ethyl acetate (3x50 mL). The combined organic extracts were washed with 50 mL saturated NaCl and dried ($MgSO_4$). The resulting solution was concentrated to approximately 10 mL on a rotary evaporator, 50 mg solid $Na_2CO_3$ was added and the remaining diglyme removed by distillation on high vacuum (0.30 mm) using minimal heating. The pot residue was taken up in 30 mL water, made just acidic by dropwise addition of 30% HCl, and extracted with ethyl acetate (3x20 mL). The combined organic extracts were washed with 20 mL saturated NaCl and dried ($MgSO_4$). Solvent removal afforded a tan oil which was chromatographed on a 10″x1/2″ silica gel (230-400 mesh) column using hexane: ethyl acetate: acetic acid (1:1:0.005). Collection of the product fraction and solvent removal afforded 50.5 mg (58%) of E-acid 829 as thick colorless oil having the following physical characteristics: [α]$D^{20}$+103.5 (c=0.40, $CHCl_3$); TLC $R_f$ = 0.33 (9:1:0.10, ethyl acetate:hexane:acetic acid); $^1H$ NMR ($CDCl_3$) δ 5.47 (m, 2H, $J_{trans}$ = 15.1 Hz, $J_{1'',4'}$ = 8.3 Hz, $J_{2'',3''}$ = 7.3 Hz, H1″ and H2″), 5.32 (bt, 1H, $J_{4,5}$ = 6.6 Hz, $J_{allylic}$ < 2Hz, H5), 4.80 (bs, 2H, OH), 3.76 (dd, 1H, $J_{2'',3''}$ = 7.3 Hz, $J_{3'',cyclohexyl}$ = 7.1 Hz, H3″), 3.67 (ddd, 1H, $J_{5',6\beta'}$ = 7.1 Hz, $J_{5'\,6\alpha}$, = 9.8 Hz, $J_{4'5'}$=9.6 Hz, H5′), 2.45-1.65 (m, 16H, H1′, H2, H4′, H3a′, H3β′, H4, H6a′, H6β′, cyclohexyl), 1.40-0.85 (m, 8H, H6α′, H3α′, cyclohexyl), 1.08 (s, 3H, $CH_3$), 1.03 (s, 3H, $CH_3$); $^{13}$C NMR ($CDCl_3$) δ 177.52 (e, Cl), 144.00 (e, C2′), 134.74 (o, C2″), 133.44 (o, C1″), 117.78 (o, C5), 77.96 (o, C5′), 76.95 (o, C3″), 56.97 (o, C4′), 45.09 (e, C2), 44.92 (o, C3a′), 42.97 (o, C6a′), 41.49 (e, C3′), 40.99 (e, C6′), 38.18 (e, C1′), 37.33 (o, cyclohexyl CH), 35.87 (e, C4), 33.89 (e, C3), 28.89 (e, cyclohexyl), 28.67 (e, cyclohexyl), 27.32 (o, $CH_3$), 27.29 (o, $CH_3$), 26.45 (e, cyclohexyl), 25.99 (e, cyclohexyl), 25.91 (e, cyclohexyl); IR (neat) $cm^{-1}$ (μm) 3340 (2.29), 2940 (3.40), 1710 (5.85), 1450 (6.90), 1410 (7.09), 1290 (7.75), 1085 (9.21), 1000 (10.0), 975 (10.3); mass spectrum, CI, m/z (rel. intensity) 373 (M+H-$H_2O$, 30), 355 (M+H-2$H_2O$, 100), 337 (4); exact mass (CI): calcd for $C_{24}H_{37}O_3$ (M+H-$H_2O$): 373.2742, found 373.2738.

Scheme (1)

## REACTION SCHEMES FOR ANALOGS AND ANALOG INTERMEDIATES

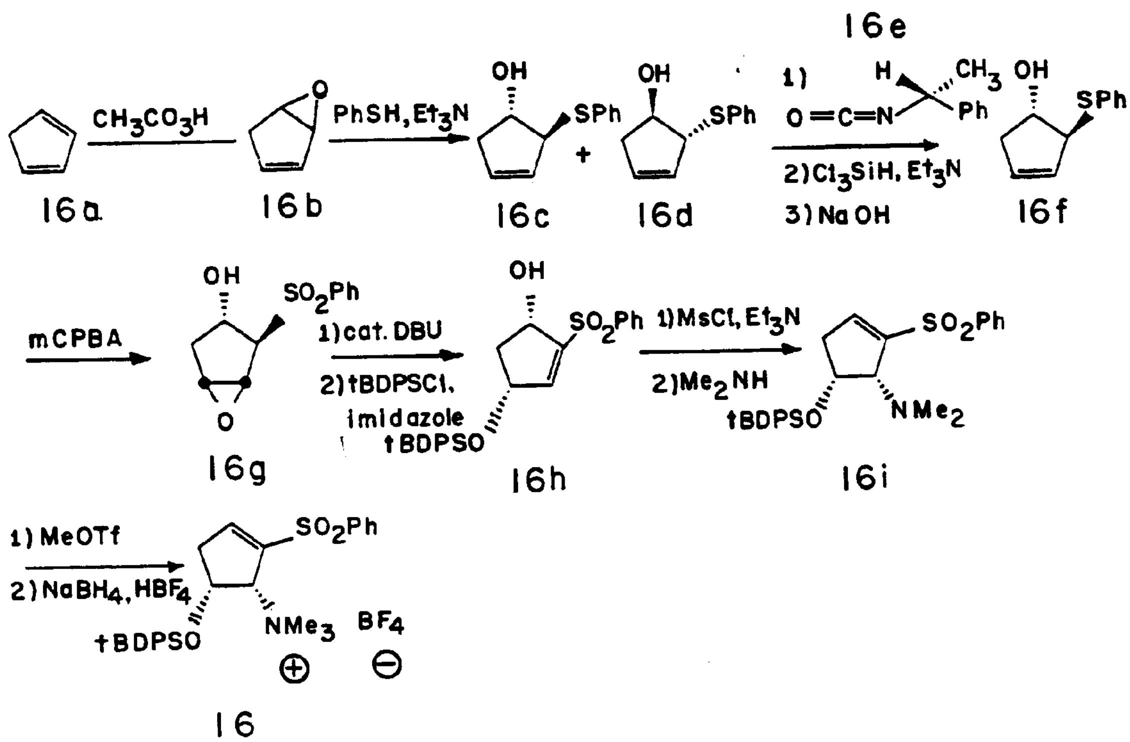

**Scheme (2)**

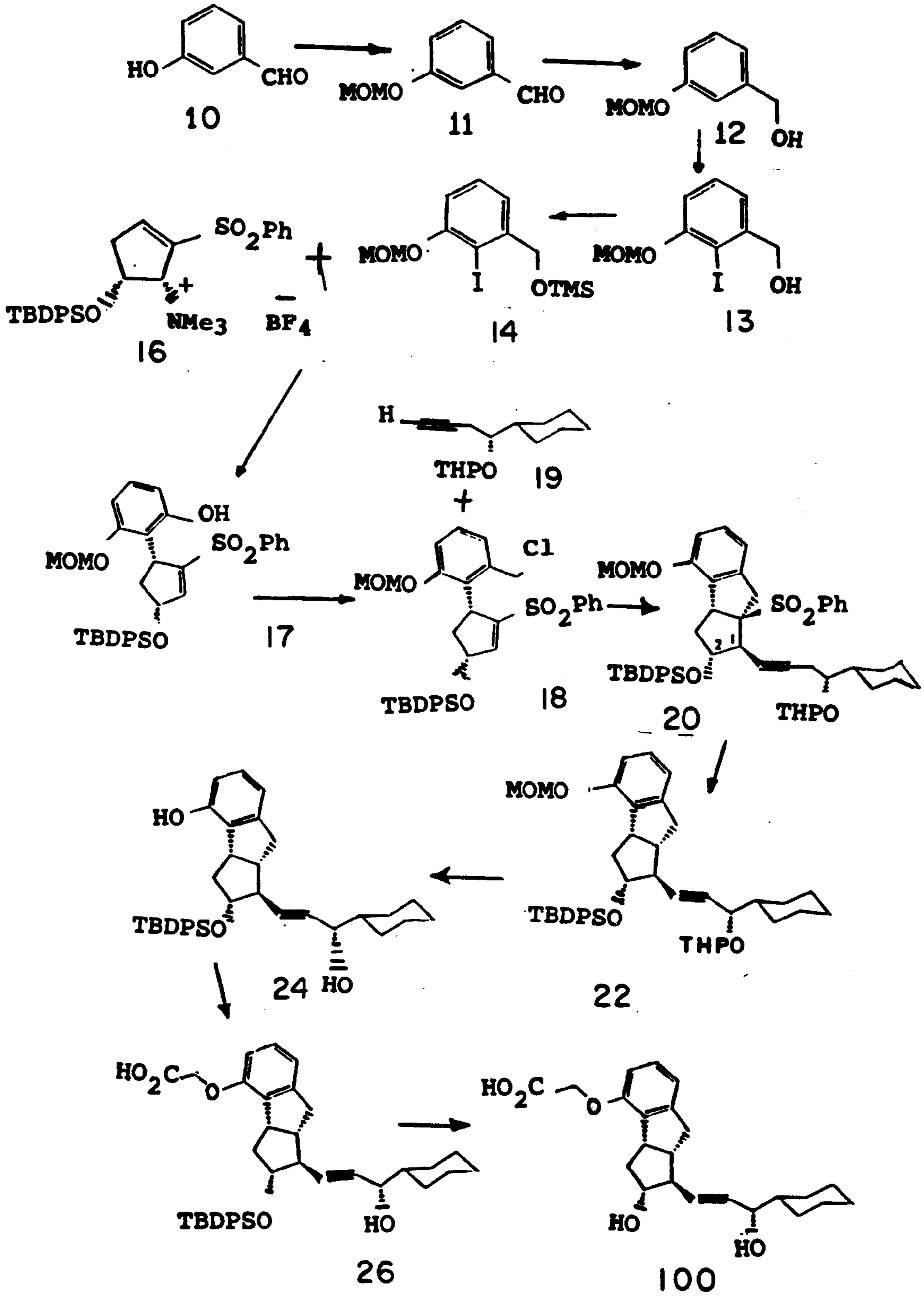

10, 11, 12, 13, 14, 16, 17, 18, 19, 20, 22, 24, 26, 100

**Scheme (3)**

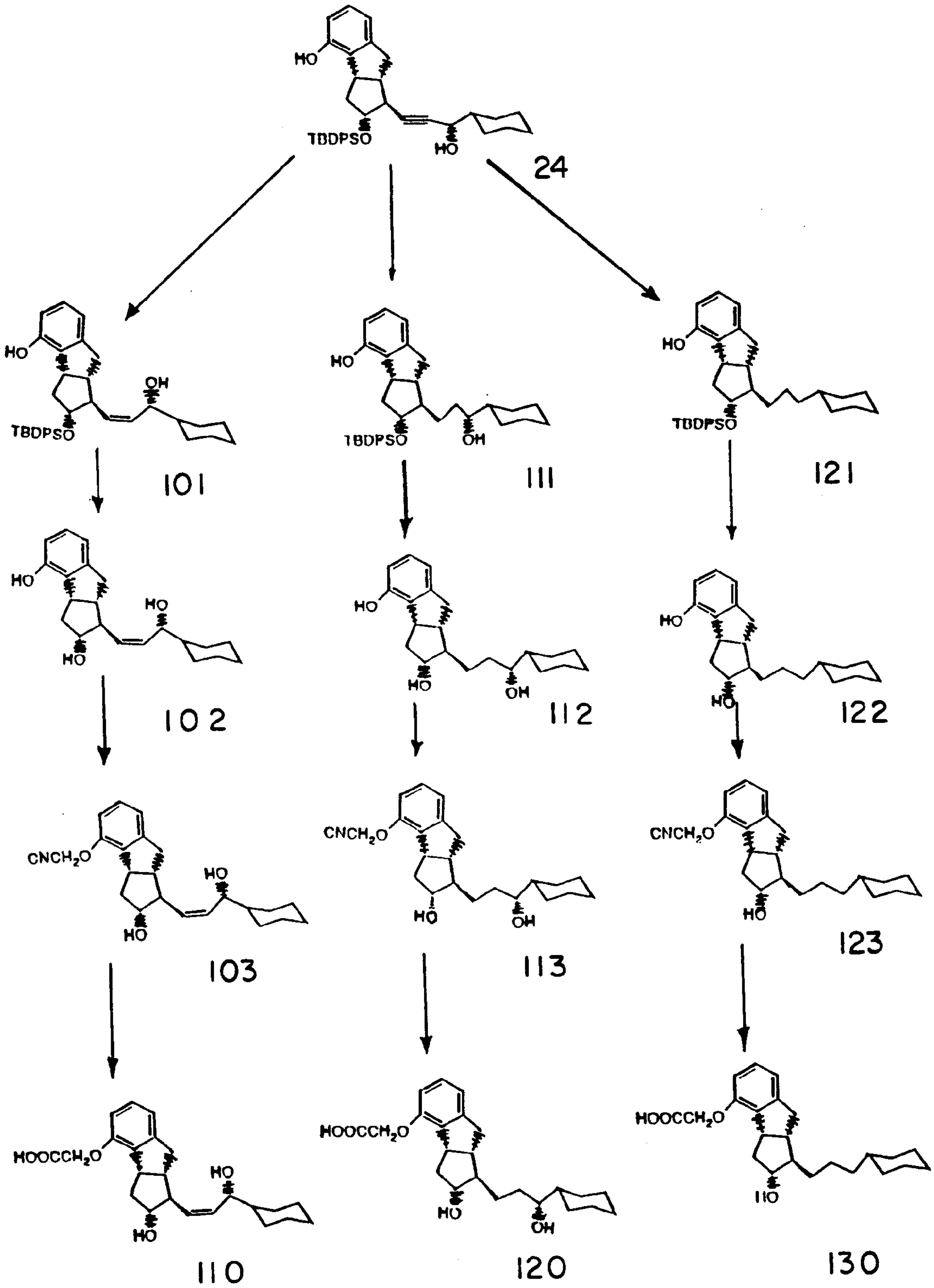

24

101 111 121

102 112 122

103 113 123

110 120 130

**Scheme (4a)**

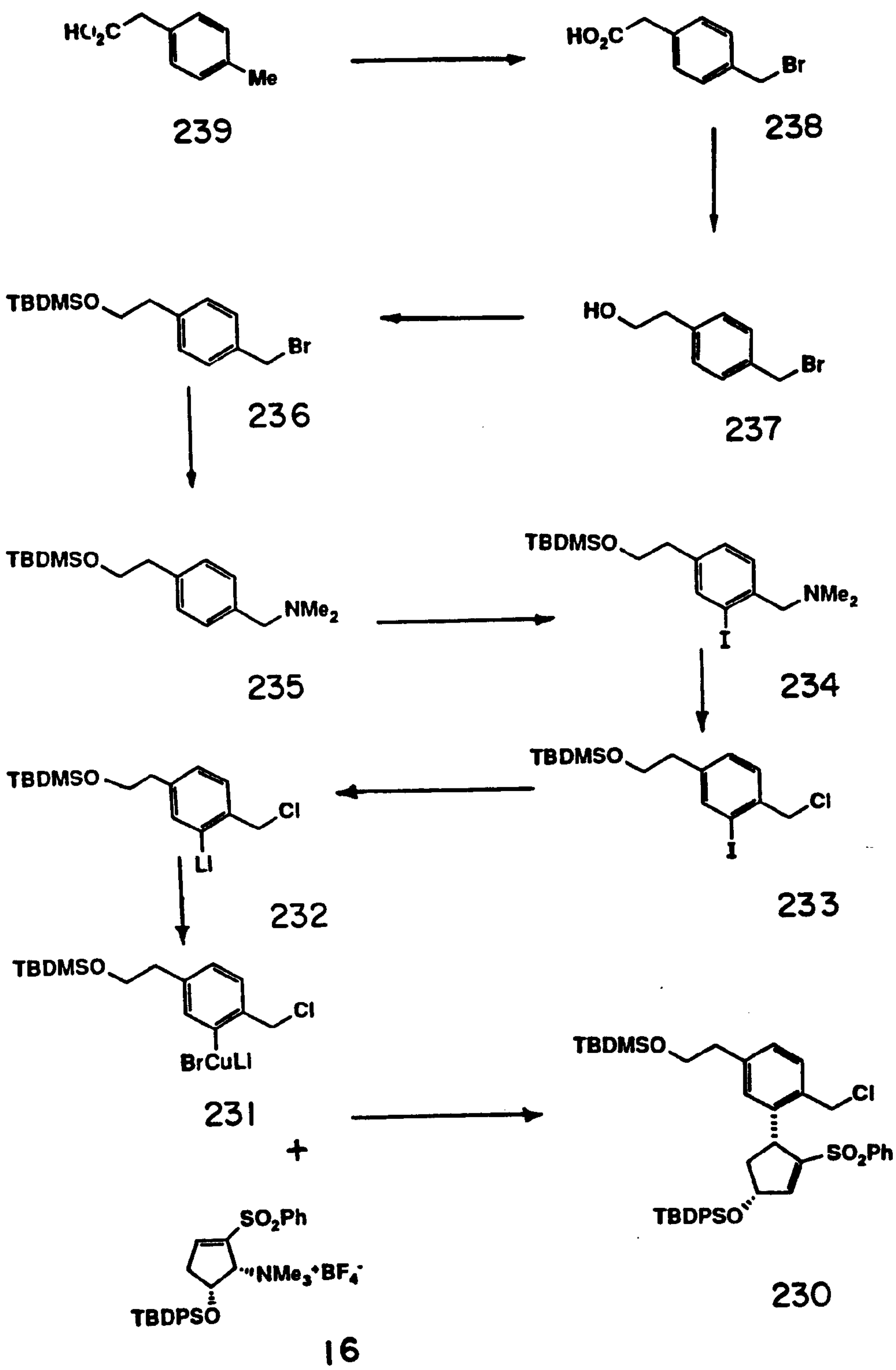

239

238

236

237

235

234

232

233

231

+

16

230

Scheme (4b)

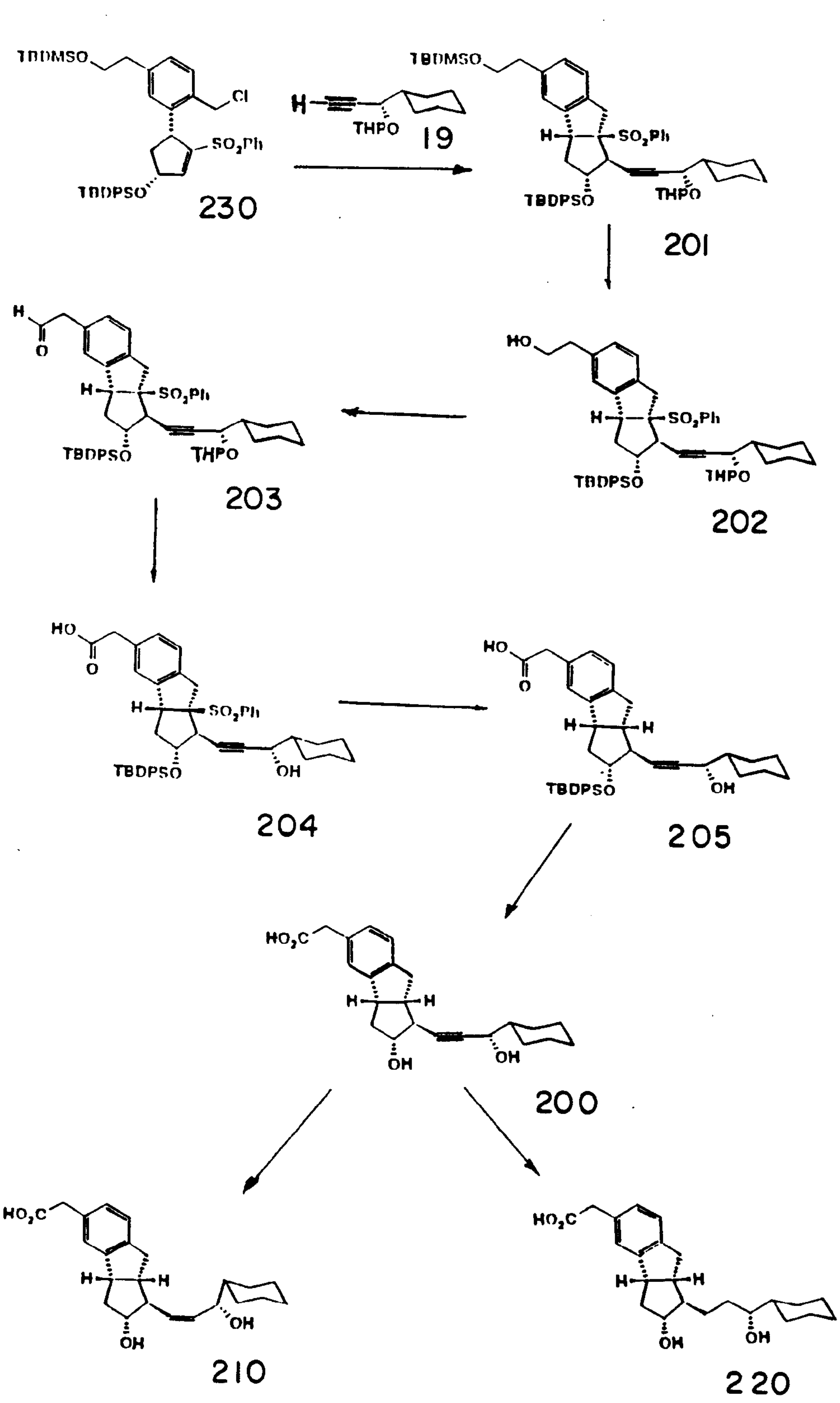

230 19 201 203 202 204 205 200 210 220

Scheme (5)

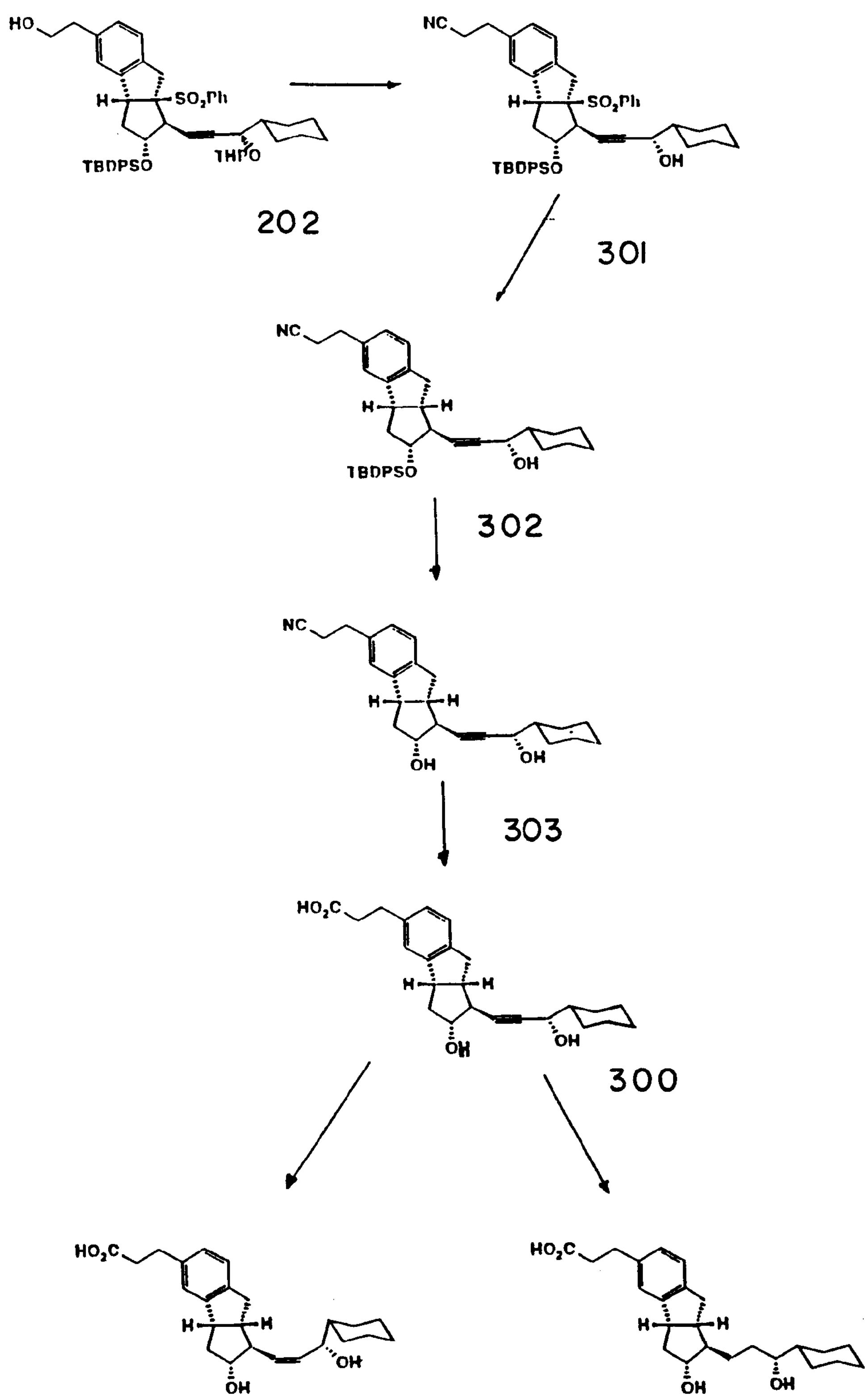

202

301

302

303

300

310

320

Scheme 6

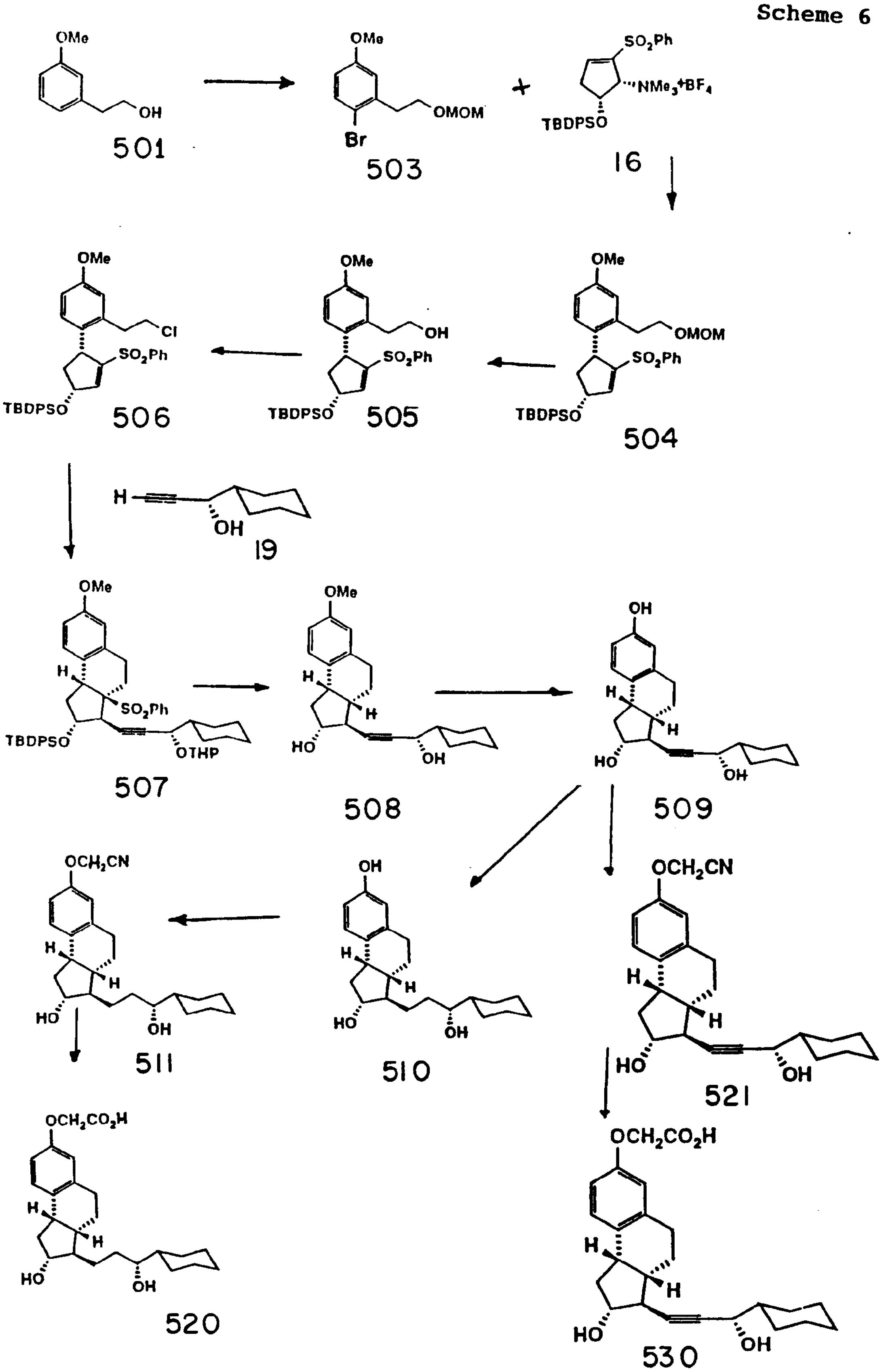

501

503

16

504

505

506

19

507

508

509

510

511

521

520

530

Scheme (7a)

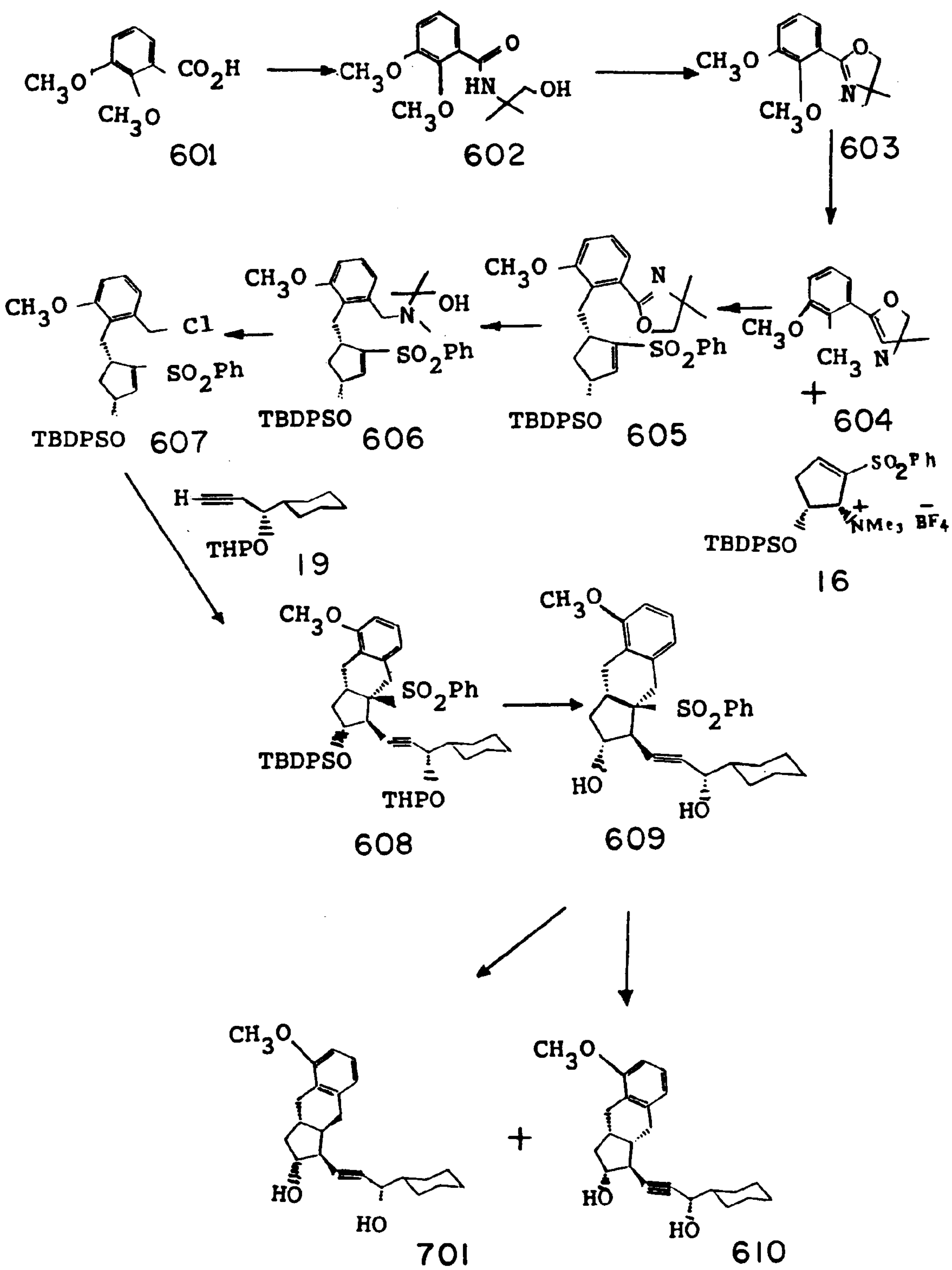

601 602 603 604 16 605 606 607 19 608 609 701 610

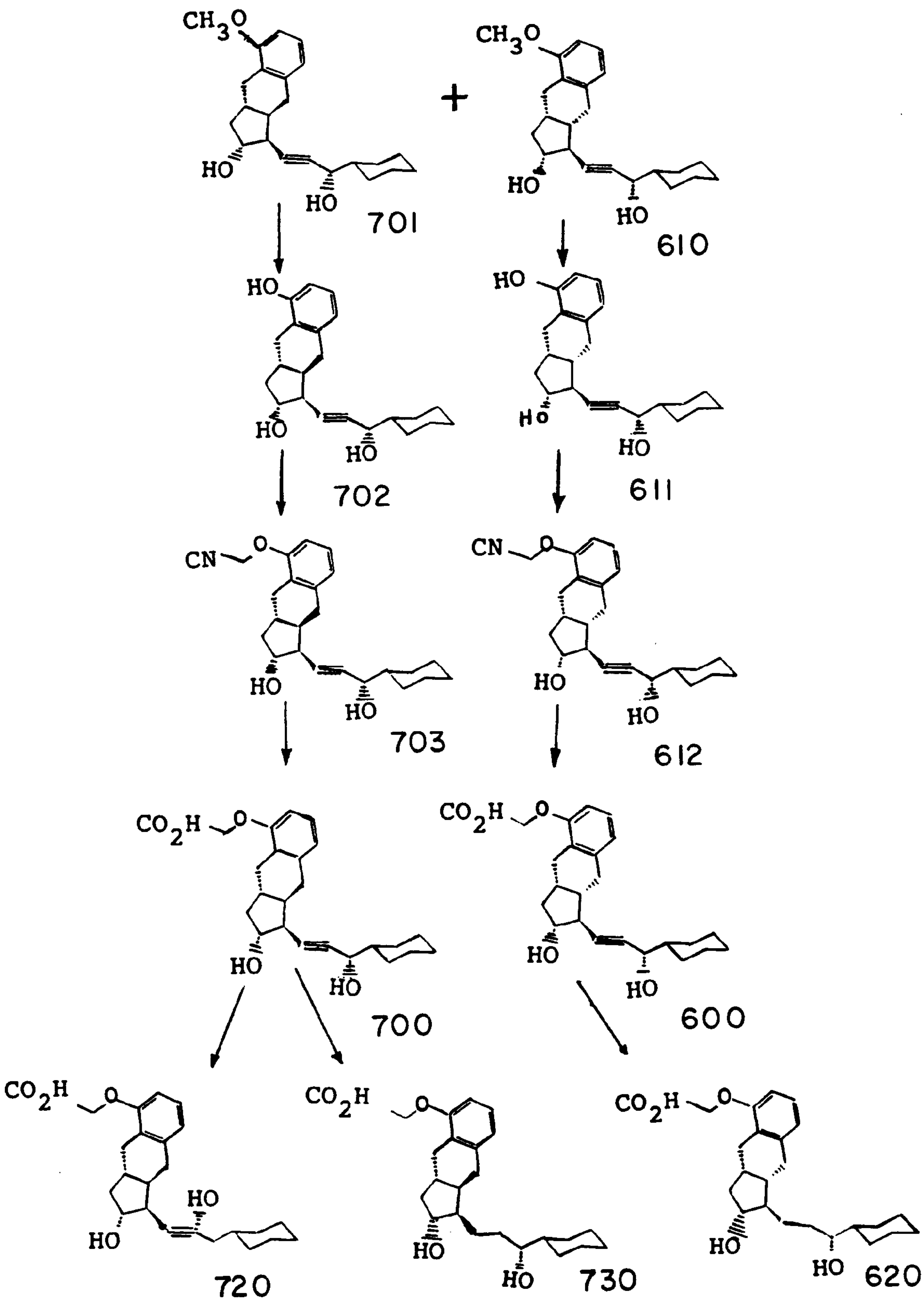

701 610 702 611 703 612 700 600 720 730 620

Scheme (8a)

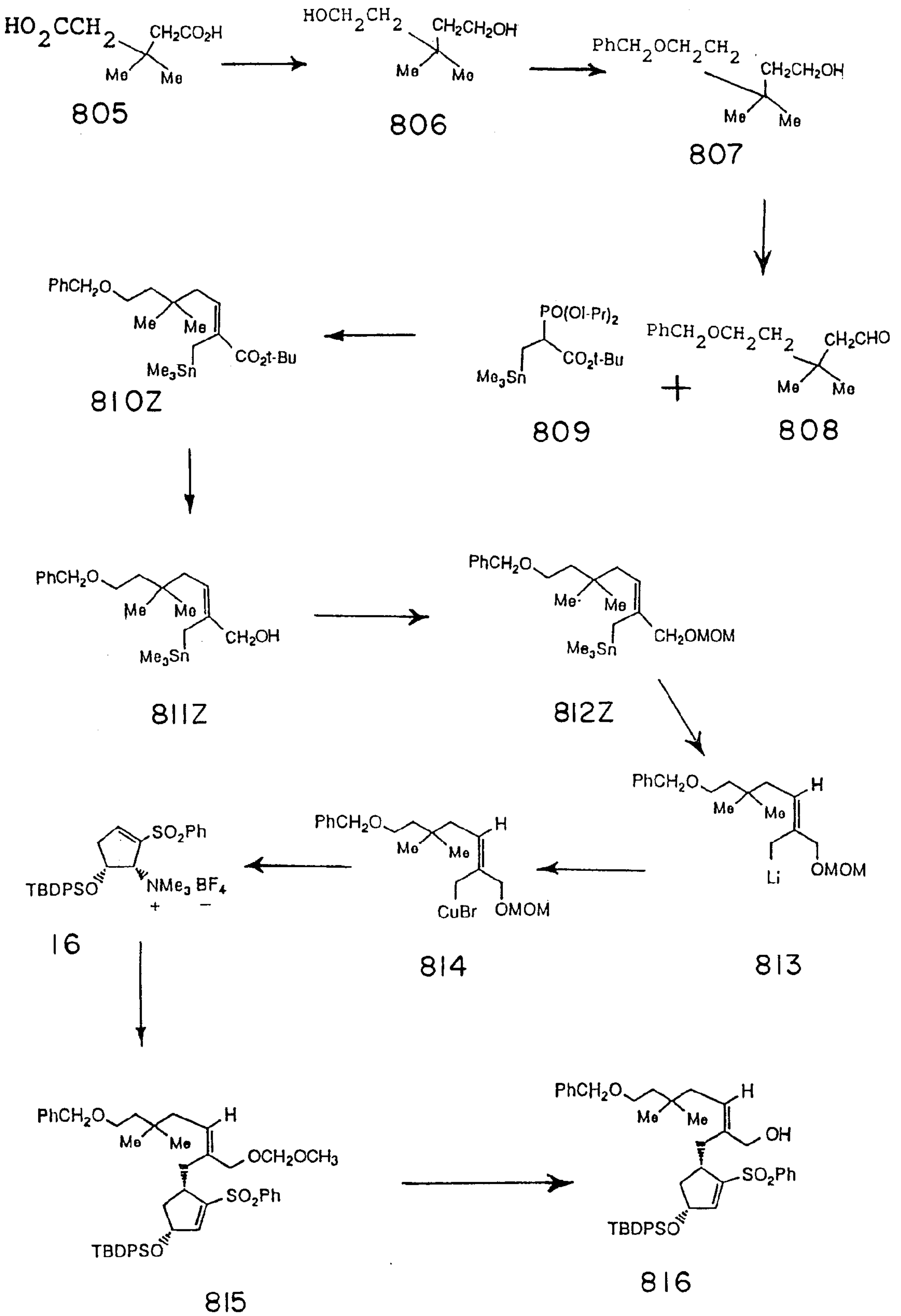

805 806 807 809 808 810Z 811Z 812Z 813 814 16 815 816

**Scheme (8b)**

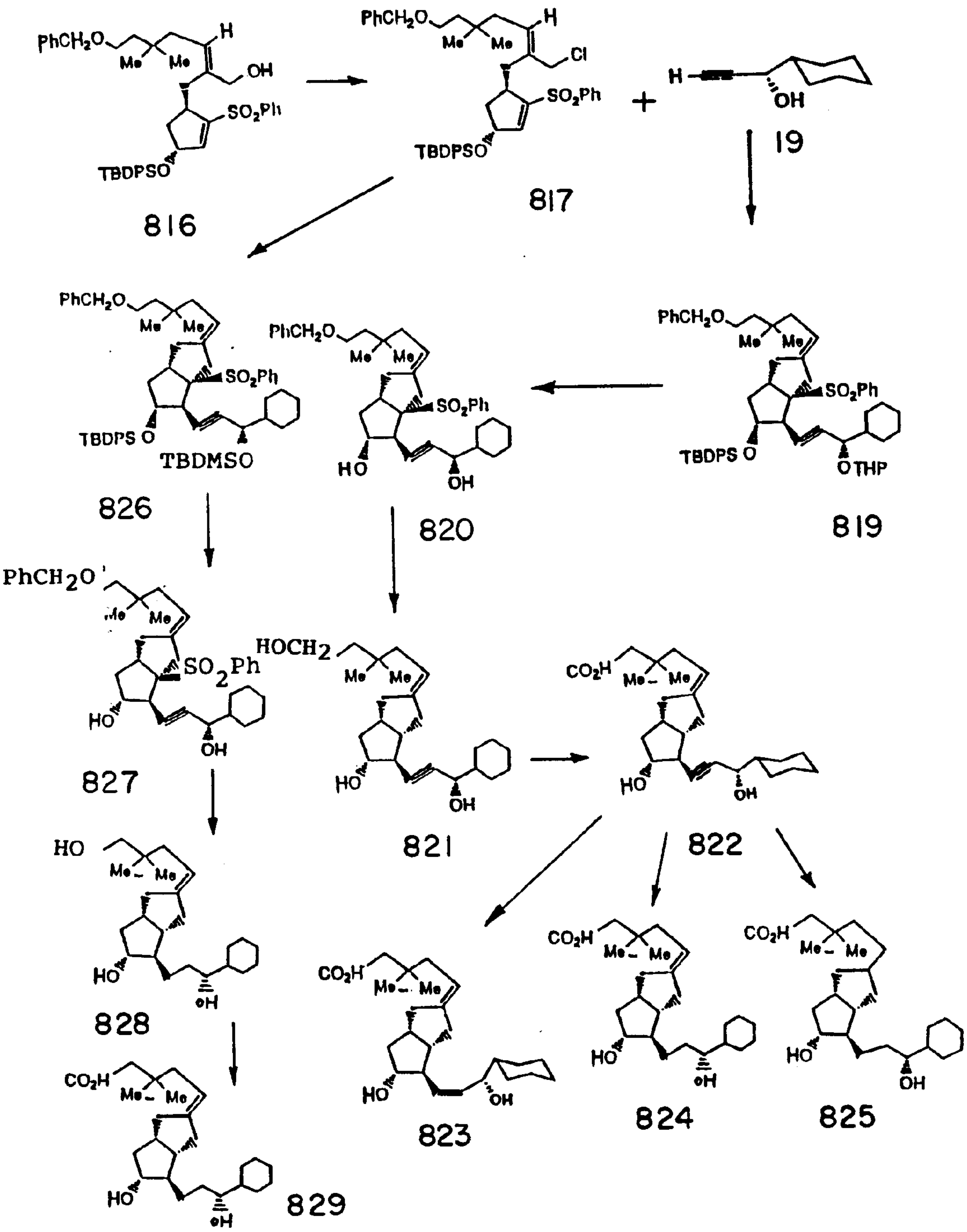

816 817 19 826 820 819 827 821 822 828 823 824 825 829

The present carbacyclin analogs exhibit prostacyclin-like pharmacological activities. They are useful as agents in the study, prevention, control, and treatment of prostacyclin responsive diseases, and other undesirable physiological conditions in mammals, particularly including humans. In particular, the present compounds have useful application as antithrombotic agents, anti-ulcer agents, anti-shock agents, anti-metastatic agents,

and anti-asthma agents, as described more particularly below.

(a) Platelet Aggregation Inhibition

The carbacyclin analogs of this invention inhibit platelet aggregation and thereby help to prevent the formation of thrombi in mammals, including man. For example, the present compounds are useful in the treatment and prevention of myocardial infarctions, to treat and prevent post-operative thrombosis, to promote patency of vascular grafts following surgery, to treat peripheral vascular diseases, and to treat conditions such as atheriosclerosis, arteriosclerosis, blood clotting defects due to lipemia, and other clinical conditions in which the underlying etiology is associated with lipid imbalance or hyperlipidemia. Other applications include use in geriatric patients to reduce potential for renal or cerebral ischemia, and for long term prophylaxis following myocardial infarctions and strokes. Further, because of their vasodilating properties, the present carbacyclin analogs are useful in the treatment of shock, where kidney infarction can occur due to lack of blood flow.

The present compounds can be administered orally or parenterally, e.g. intravenously, subcutaneously, intramuscularly, or in the form of sterile implants for prolonged release. For rapid physiological response, especially in emergency situations, the intravenous route of administration is preferred. Parenteral doses range from about 0.01 to about 10 mg, more preferably about 0.1 to about 10 mg per kg of body weight per day, the exact dose depending on the age, weight, and condition of the patient or animal, and on the frequency and route of administration.

The preferred route of administration for the present compounds is oral administration, although other non-parenteral routes (e.g., buccal, rectal, sublingual) are employed in preference to parenteral routes. Oral dosage forms can be conventionally formulated (tablets, capsules, suspensions etc.) and administered 2-4 times daily. Oral dose levels range from about 0.05 to 100 mg, more preferably about .1 to about 10 mg, per kg of body weight per day are effective.

The present compounds also find application in vitro. For example, the can be added to whole blood prior to storage. Additionally, whole blood or buffers containing these compounds can be circulated through donor organs, e.g., heart and kidneys, prior to transplant. They are also useful for preparation of platelet rich concentrates for use in treating thrombocytopenia or for use in conjunction with chemotherapy and radiation therapy. As a whole blood additive and for other in vitro applications, the present compounds can be used at a concentration of about .001 to about 1.0 mg per ml.

(b) Gastric Secretion Reduction

The carbacyclin analogs of this invention can be used to reduce and control gastric secretion and thereby reduce or avoid gastrointestinal ulcer formation and accelerate the healing of existings ulcers. For such indications, the present carbacyclin analogs can be administered intravenously, subcutaneously, or intramuscularly in a parenteral dose of about 0.01 to about 10 mg per kg of body weight per day, the exact dose depending on the age, weight, and condition of the patient or animal, and on the frequency and route of administration. Preferably, however, the present compounds are administered orally or by other non-parenteral routes. A daily oral dose of about 1.0 to about 100 mg per kg of body weight is most preferred, typically administered in 1-6 divided doses per day. Once healing of the ulcers has been accomplished, a lower maintenance dosage can be employed to prevent recurrence of the condition.

(c) NOSAC-Induced Lesion Inhibition

Coadministration of carbacyclin analogs of this invention is effective to reduce the undesirable gastrointestinal effects often associated with administration of anti-inflammatory prostaglandin synthetase inhibitors. U.S. Patent No. 3,781,429 describes prostaylandins having a similar effect. Accordingly the present carbacyclin analogs are useful, for example, in reducing the known ulcerogenic effects of, for example, the non-steroidal antiinflammatory agents.

(d) Bronchodilation (Anti-asthma)

The present compounds also find use in the treatment of asthma. For example, these compounds are useful as bronchodilators or as inhibitors of mediator-induced bronchoconstriction, such as that produced by histamine released from mast cells activated by an antigen-antibody complex. Thus, these compounds are effective to facilitate breathing in conditions such as bronchial bronchitis, bronchiectasis, pneumonia, and emphysema. Such purposes, the present compounds can be aministered in a variety of dosage forms, e.g., orally in the form

of tablets, capsules, or liquids; rectally in the form of suppositories; parenterally (intravenous administration being preferred in emergency situations); by inhalation in the form of aerosols or solutions for nebulizers; or by insufflation in the form of powder. Doses in the range of about 0.01 to 5 mg per kg of body weight are used 1 to 4 times a day, the exact dose depending on the age, weight, and condition of the , patient and on the frequency and route of administration. For the above use these carbacyclin analogs can be combined advantageously with other anti-asthmatic agents, such as sympathomimetics (isoproterenol, phenylephrine, ephedrine, etc.); xanthine derivatives (theophylline and aminophylline); and corticosteroids (ACTH and prenisolone).

These compounds are most conveniently administered to human asthma patients by inhalation. For administration by the oral inhalation route with conventional nebulizers or by oxygen aerosolization it is convenient to formulate the present compound in dilute solution, preferably at concentrations of about one part active compound to about 100 to about 200 parts by weight of total solution. Conventional excipients may be employed to stabilize such solutions or to provide isotonic media; for example, sodium chloride, sodium citrate, citric acid, sodium bisulfite, and the like can be employed. For administration as a self-propelled dosage unit for administering the active ingredient in aerosol form suitable for inhalation therapy the composition can comprise a compound of this invention suspended in an inert propellant (such as a mixture of dichlorodifluoromethane and dichlorotetrafluoroethane) together with a co-solvent, such as ethanol, flavoring materials and stabilizers. Suitable means for employing aerosol inhalation therapy techniques are described, for example, in U.S. Pat. No. 3,868,691.

The carboxylic acids represented by Formula 50-55 when Q is -COOH can be used in the free acid form as their alkyl esters or as their pharmacologically acceptable salt. Preferred esters are $C_1$ to $C_{12}$ esters. Methyl and ethyl are especially preferred for optimum absorption of the compound by the body or experimental animal system; and straight-chain octyl, nonyl, decyl, undecyl, and dodecyl are especially preferred for prolonged activity.

Pharmacologically acceptable salts of this invention can be formed with pharmacologically acceptable metal cations, ammonia, or amines. Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amines for forming salts of the present invention are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, dietylenetriamine, adamantylamine, and the like; aliphatic, cycloaliphatic, araliphatic amines containing up to and including about 18 carbon atoms, as well as heterocyclic amines, e.g., piperidine, morpholine, pyrrolidine, pipetazine, and lower-alkyl derivatives thereof, e.g. 1-methylpipetidine, 4-ethylmorpholine, 1-isoptopylpyrrolidine, 2-methylpyrrolidine, 1,4-dimethylpiperazine, 2-methylpiperidine, and the like as well as amines containing water-solubilizing or hydrophilic groups, e.g., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydtoxymethyl) aminomethane, N-phenylethanolamine, N-(1-tertamylphenyl)-diethanolamine, galactamine, N-methylglucosamine, ephedrine, phenylephrine, epinephrine, procaine, and the like. Further useful amine salts are the basic amino acid salts, e.g., lysine and arginine.

## Claims

1. A compound of the formula

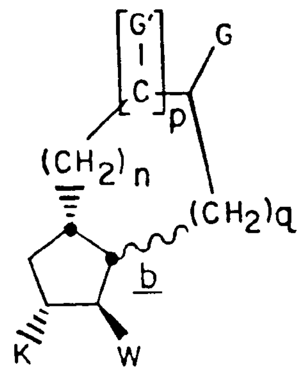

wherein K is hydrogen, hydroxymethyl, or hydroxy;
W is a group of the formula

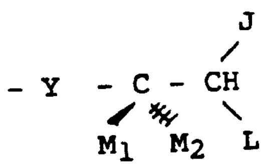

wherein Y is $-CH_2CH_2-$, <u>cis</u> -CH=CH-, <u>trans</u> -CH=CH-, or -C≡C-,
$M_1$ and $M_2$ are independently hydrogen or hydroxy, or $M_1$ and $M_2$ taken together form =O; and
L and J are independently hydrogen or $C_1$-$C_6$ alkyl, or L and J taken together with the carbon atom bridging those groups form a $C_3$-$C_7$ cycloalkyl group; covalent bond <u>b</u> is in the alpha (α) or beta (β) configuration;
n is 0 or 1, p is 0 or 1, and q is 1 or 2; provided that
when n = 0, covalent bond <u>b</u> is in the alpha configuration;
when n = p = q = 1, bond <u>b</u> is in the beta configuration;
when p = 0, n = q = 1 and G is $-CH_2CH_2C(CH_3)_2CH_2Q$, or $=CHCH_2C(CH_3)_2CH_2Q$ wherein Q = -COOH or $-CH_2OH$;
when p = 1, G and G′ taken together with the carbon atoms to which they are bonded form a group of the formula

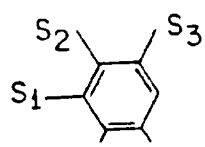

wherein $S_1$, $S_2$, and $S_3$ are hydrogen or a group -AEQ wherein A is $-CH_2-$ or -O-; E is $-(CH_2)_r-$ wherein r is 0-4, and Q is as defined above provided that at least two of $S_1$, $S_2$ and $S_3$ are hydrogen; and
when Q is -COOH, the pharmaceutically acceptable non-toxic salts and $C_1$-$C_{12}$ alkyl esters of the carboxylic acids represented thereby.

**2.** The compound of claim 1 wherein K is hydroxy.

**3.** The compound of claim 1 wherein n=0 and P=q=1.

**4.** The compound of claim 3 wherein K is hydroxy.

**5.** The compound of claim 4 wherein $S_1$ is -AEQ and A is -O-.

**6.** The compound of claim 5 wherein $S_1$ is $-OCH_2COOH$ and wherein J and L taken together with the carbon atom bridging those groups form a $C_3$-$C_7$ cycloalkyl group.

7. The compound of claim 6 wherein the cycloalkyl group is cyclohexyl.

8. The compound of claim 7 wherein $M_1$ is hydrogen and $M_2$ is hydroxy.

9. The compound of claim 7 wherein $M_1$ and $M_2$ are hydrogen.

10. The compound of claim 4 wherein $S_2$ is -AEQ.

11. The compound of claim 10 wherein A is $-CH_2-$ and r is 0.

12. The compound of claim 11 wherein Q is -COOH.

13. The compound of claim 11 wherein Q is $-CH_2OH$.

14. The compound of claim 10 wherein A is $-CH_2-$ and r is 1.

15. The compound of claim 14 wherein Q is -COOH.

16. The compound of claim 14 wherein Q is

17. The compound of claim 11 or claim 14 wherein J and L taken together with the carbon atom bridging those groups form a $C_3$-$C_7$ cycloalkyl group.

18. The compound of claim 17 wherein the cycloalkyl group is cyclohexyl, $M_1$ is hydrogen, and $M_2$ is hydroxy.

19. The compound of claim 17 wherein $M_2$ is hydroxy.

20. The compound of claim 17 wherein the cycloalkyl group of cyclohexyl.

21. The compound of claim 1 wherein n=0, p=1 and q=2.

22. The compound of claim 21 wherein $S_3$ is -AEQ.

23. The compound of claim 21 wherein $S_3$ is $-O(CH_2)_rQ$.

24. The compound of claim 23 wherein r is 1 and Q is -COOH.

25. The compound of claim 21, claim 23 or claim 24 wherein $M_1$ is hydrogen and $M_2$ is hydroxy.

26. The compound of claim 25 wherein J and L taken together with the carbon atom bridging those groups form a cyclohexyl group.

27. The compound of claim 2 wherein n=p=q=1.

28. The compound of claim 27 wherein AEQ.

29. The compound of claim 28 wherein A is -0-.

30. The compound of claim 28 wherein $S_1$ is $-OCH_2Q$.

31. The compound of claim 30 wherein Q is -COOH.

32. The compound of claim 29 or claim 30 wherein $M_1$ is hydrogen and $M_2$ is hydroxy.

33. The compound of claim 32 wherein J and L taken together with the carbon atom bridging those groups from a $C_3$-$C_7$ cycloalkyl group.

34. The compound of claim 33 wherein the cycloalkyl group is cyclohexyl.

**35.** The compound of claim 34 wherein Y is -C≡C-, cis-CH=CH=CH- or $-CH_2CH_2-$.

**36.** The compound of claim 2 wherein n=q=1 and p=0.

**37.** The compound of claim 36 wherein G is $-CH_2CH_2C(CH_3)_2CH_2Q$.

**38.** The compound of claim 36 wherein Q is -COOH.

**39.** The compound of claim 36 wherein G is $=CHCH_2(CH_3)_2CH_2Q$.

**40.** The compound of claim 39 wherein Q is -COOH.

**41.** The compound of claim 36 wherein Q is $-CH_2OH$.

**42.** The compound of claim 36, claim 38, claim 40 or claim 41 wherein $M_1$ is hydrogen and $M_2$ is hydroxy.

**43.** The compound of claim 42 wherein J and L taken together with the carbon atom bridging those groups form a $C_3$-$C_7$ cycloalkyl group.

**44.** The compound of claim 43 wherein the cycloalkyl group is cyclohexyl.

**45.** A compound of the formula

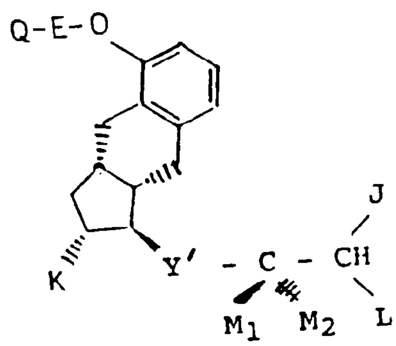

wherein

K is hydrogen, hydroxy or hydroxymethyl;

Y′ is cis-CH=CH-, trans -CH=CH-, or -C≡C=;

$M_1$ and $M_2$ are independently hydrogen or hydroxy or $M_1$ and $M_2$ taken together form =O;

L and J are independently hydrogen or $C_1$-$C_7$ alkyl or L and J taken together with the carbon atom bridging those groups form a $C_3$-$C_7$ cycloalkyl group;

E is $-CH_2$ - or $-CH_2CH_2-$; and

Q is -COOH or $-CH_2OH$; and when Q is -COOH the pharmaceutically acceptable salts of the carboxylic acids represented thereby.

**46.** The compound of claim 45 wherein K is hydroxy.

**47.** The compound of claim 45 wherein the group -OEQ is $-OCH_2COOH$.

**48.** The compound of claim 46 wherein the group -OEQ is $-OCH_2COOH$.

**49.** The compound of claim 48 wherein Y′ is -C≡C-.

**50.** The compound of claim 49 wherein $M_1$ is hydrogen and $M_2$ is hydroxy.

**51.** The compound of claim 50 wherein the groups J and L taken together with the carbon atom bridging those groups from a $C_3$-$C_7$ cycloalkyl group.

**52.** The compound of claim 51 wherein the cycloalkyl group is cyclohexyl.

**53.** The compound of claim 48 wherein Y′ is cis-CH=CH-, $M_1$ is hydrogen, $M_2$ is hydroxy and J and L taken together with the carbon atom bridging those groups from a cyclohexyl group.

**54.** A pharmaceutical composition comprising an effective amount of a compound of claim 1 and a pharmaceutically acceptable excipient therefor.

**55.** A pharmaceutical composition comprising an effective amount of a compound of claim 3 and a pharmaceutically acceptable excipient therefor.

**56.** A pharmaceutical composition comprising an effective amount of a compound of claim 10 and a pharmaceutically acceptable excipient therefor.

**57.** A pharmaceutical composition comprising an effective amount of a compound of claim 20 and a pharmaceutically acceptable excipient therefor.

**58.** A pharmaceutical composition comprising an effective amount of a compound of claim 26 and a pharmaceutically acceptable excipient therefor.

**59.** A pharmaceutical composition comprising an effective amount of a compound of claim 34 and a pharmaceutically acceptable excipient therefor.

**60.** A pharmaceutical composition comprising an effective amount of a compound of claim 35 and a pharmaceutically acceptable excipient therefor.

**61.** A pharmaceutical composition comprising an effective amount of a compound of claim 45 and a pharmaceutically acceptable excipient therefor.

**62.** A pharmaceutical composition comprising an effective amount of a compound of claim 52 and a pharmaceutically acceptable excipient therefor.

European Patent Office

# EUROPEAN SEARCH REPORT

Application Number

EP 92300451.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB - A - 2 121 802 (UPJOHN) * Claims * | 1,45, 54 | C 07 C 59/72<br>C 07 C 43/23<br>A 61 K 31/557 |
| A | US - A - 4 668 814 (P.A. ARISTOFF) * Claims 1-10 * | 1,45 | |
| A | US - A - 4 544 764 (P.A. ARISTOFF) * Totality * | 1,45 | |
| A | US - A - 4 306 075 (P.A. ARISTOFF) * Claims * | 1,45 | |
| A | EP - A - 0 347 243 (WELLCOME) * Claims * | 1,45, 54-62 | |

TECHNICAL FIELDS SEARCHED (Int. Cl.5)

C 07 C 59/00
C 07 C 43/00
A 61 K 31/00

The present search report has been drawn up for all claims

| Place of search | Date of completion of the search | Examiner |
|---|---|---|
| VIENNA | 13-04-1992 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)